# EUROPEAN PATENT SPECIFICATION

(11) **EP 1 268 826 B1**
(45) Date of publication and mention of the grant of the patent: **30.04.2008**
(21) Application number: 01915416.0
(22) Date of filing: 26.03.2001
(51) Int. Cl.: C12N 15/82

(54) **CESTRUM YELLOW LEAF CURLING VIRUS PROMOTERS**
PROMOTOREN DES CESTRUM YELLOW LEAF CURLING VIRUS
PROMOTEURS DU VIRUS DES FEUILLES JAUNES EN CUILLERE DU CESTRUM

(30) Priority: 27.03.2000 GB 0007427; 28.04.2000 GB 0010486; 26.01.2001 EP 01101802; 28.02.2001 US 272076 P
(43) Date of publication of application: 02.01.2003
(73) Proprietor: Syngenta Participations AG, 4058 Basel (CH)
(72) Inventor: HOHN, Thomas, CH-4144 Arlesheim (CH); STAVOLONE, Livia, CH-8057 Zürich (CH); DE HAAN, Petrus, Theodorus, NL-2343 RR Oegstgeest (NL); LIGON, Hope, Thompson, Apex, NC 27502 (US); KONONOVA, Maria, Raleigh, NC 27613 (US)
(74) Representative: Kock, Michael Andreas
(86) International application number: PCT/EP2001/003408
(87) International publication number: WO 2001/073087

(56) References cited:
- EP-A- 0 426 641
- WO-A-84/02913
- US-A- 5 824 857

## Description

The present invention relates to novel DNA sequences that function as promoters of transcription of associated nucleotide sequences in plants. More specifically, this invention relates to novel promoters that confer constitutive expression to an associated nucleotide sequence of interest.

In the field of agriculture there exists a continuous desire to modify plants according to one's needs. One way to accomplish this is by using modern genetic engineering techniques. For example, by introducing a gene of interest into a plant, the plant can be specifically modified to express a desirable phenotypic trait. For this, plants are transformed most commonly with a heterologous gene comprising a promoter region, a coding region and a termination region. When genetically engineering a heterologous gene for expression in plants, the selection of a promoter is a critical factor. While it may be desirable to express certain genes only in response to particular stimuli or confined to specific cells or tissues, other genes are more desirably expressed constitutively, i.e. throughout the plant at all times and in most tissues and organs. In the past, the 35S promoter from Cauliflower Mosaic Virus (CaMV 35S promoter) has been widely used for constitutive expression of heterologous genes in plants. There are, however, occasions where it is desirable to use alternative promoters. Therefore, it is a major objective of the present invention to provide such alternative promoters for expression of a nucleotide sequence of interest in plants. The invention also provides recombinant DNA molecules, expression vectors and transgenic plants comprising the promoters of the present invention.

The present invention thus provides:
a DNA sequence capable of driving expression of an associated nucleotide sequence, wherein said DNA sequence is obtainable from the genome of Cestrum yellow leaf curling virus (CmYLCV). Preferred is a DNA sequence which is obtainable from the CmYLCV full-length transcript promoter and comprises the nucleotide sequence depicted in SEQ ID NO:1.

In particular, DNA sequences are provided, wherein
- said DNA sequence comprises the nucleotide sequence depicted in SEQ ID NO:2
- said DNA sequence comprises the nucleotide sequence depicted in SEQ ID NO:3
- said DNA sequence comprises the nucleotide sequence depicted in SEQ ID NO:4
- said DNA sequence comprises the nucleotide sequence depicted in SEQ ID NO:5
- said DNA sequence comprises the nucleotide sequence depicted in SEQ ID NO:6
- said DNA sequence hybridizes under stringent conditions to any one of SEQ ID NO:1, SEQ ID NO:2, SEQ ID NO:3, SEQ ID NO:4, SEQ ID NO:5 or SEQ ID NO:6, in particular wherein the DNA sequence as mentioned hereinbefore comprises the nucleotide sequence depicted in SEQ ID NO:19 or SEQ ID NO:20.

The invention further provides DNA sequences comprising a consecutive stretch of at least 50 nt, preferably of between about 400 bases and about 650 bases, more preferably of between about 200 bases and about 400 bases and most preferably of about 350 bases in length of SEQ ID NO:1, wherein said DNA sequences are capable of driving expression of an associated nucleotide sequence.

In a particular embodiment of the invention said consecutive stretch of at least 50 nt, preferably of between about 400 bases and about 650 bases, more preferably of between about 200 bases and about 400 bases and most preferably of about 350 bases in length has at least 75%, preferably 80%, more preferably 90% and most preferably 95% sequence identity with a corresponding consecutive stretch of at least 50 nt, preferably of between about 400 bases and about 650 bases, more preferably of between about 200 bases and about 400 bases and most preferably of about 350 bases in length of SEQ ID NO:1.

The invention further provides recombinant DNA molecules comprising a full-length transcript promoter region isolated from Cestrum yellow leaf curling virus. In addition, the invention provides recombinant DNA molecules and DNA expression cassettes comprising a DNA sequence according to the invention operably linked to a nucleotide sequence of interest. The invention also provides DNA expression vectors comprising said recombinant DNA and expression cassettes, respectively.

In particular, recombinant DNA molecules and DNA expression cassettes are provided wherein the nucleotide sequence of interest comprises a coding sequence, particularly wherein
- the coding sequence encodes a desirable phenotypic trait
- the coding sequence encodes a selectable or screenable marker gene
- the coding sequence encodes a protein conferring antibiotic resistance, virus resistance, insect resistance, disease resistance, or resistance to other pests, herbicide tolerance, improved nutritional value, improved performance in an industrial process or altered reproductive capability
- the coding sequence encodes a protein which confers a positive selective advantage to cells that have been transformed with said coding sequence
- the coding sequence encodes a protein which confers a metabolic advantage to cells that have been transformed with said coding sequence consisting of being able to metabolize a compound, wherein said compound is mannose or xylose or a derivative or a precursor of these, or a substrate of the protein, or is capable of being metabolized by cells transformed with said coding sequence into such a substrate
- the coding sequence encodes an enzyme selected from the group of xyloisomerases, phosphomanno-isomerase, mannose-6-phosphate isomerase, mannose-1-phosphate isomerase, phosphomanno mutase, mannose epimerase, mannose or xylose phosphatase, mannose-6-phosphatase, mannose-1-phosphatase and mannose or xylose permease
- the coding sequence encodes a phosphomanno isomerase
- the coding region is non-translatable
- the non-translatable coding region is from a viral gene, in particular from TSWV, more particularly from the TSWV NP gene
- the coding sequence encodes commercially valuable enzymes or metabolites in the plant
- the coding sequence is in antisense orientation

The invention also provides DNA expression vectors comprising a DNA sequence or a recombinant DNA molecule as mentioned hereinbefore. In a particular embodiment of the invention, said DNA expression vector is pNOV2819 or pNOV2819. Further are provided DNA expression vectors comprising a first DNA sequence according to the invention operably linked to a nucleotide sequence of interest, and a second DNA sequence according to the invention operably linked to a nucleotide sequence of interest. In a specific embodiment of the invention the DNA expression vectors as mentioned hereinbefore are capable of altering the expression of a viral genome.

In an even more specific embodiment, the DNA expression vector as mentioned hereinbefore comprises a first DNA sequence capable of expressing in a cell a sense RNA fragment of said viral genome or portion thereof and a second DNA sequence capable of expressing in a cell an antisense RNA fragment of said viral genome or portion thereof, wherein said sense RNA fragment and said antisense RNA fragment are capable of forming a double-stranded RNA.

Expression vectors as mentioned hereinbefore are provided wherein
- said virus is selected from the group consisting of tospoviruses, potyviruses, potexviruses, tobamoviruses, luteoviruses, cucumoviruses, bromoviruses, closteorviruses, tombusviruses and furoviruses
- said DNA sequences comprises a nucleotide sequence derived from a viral coat protein gene, a viral nucleocapsid protein gene, a viral replicase gene, or a viral movement protein gene or portions thereof
- said DNA sequence is derived from tomato spotted wilt virus (TSWV)
- said DNA is derived from a nucleocapsid protein gene

The invention further provides
host cells stably transformed with a DNA sequence, a recombinant DNA molecule or a DNA expression vector according to the invention. In particular, wherein
- the host cell is a bacterium
- the host cell is a plant cell
- the host cell is a plant cell selected from the group consisting of rice, maize, wheat, barley, rye, sweet potato, sweet corn, bean, pea, chicory, lettuce, cabbage, cauliflower, broccoli, turnip, radish, spinach, asparagus, onion, garlic, pepper, celery, squash, pumpkin, hemp, zucchini, apple, pear, quince, melon, plum, cherry, peach, nectarine, apricot, strawberry, grape, raspberry, blackberry, pineapple, avocado, papaya, mango, banana, soybean, tomato, sorghum, sugarcane, sugar-beet, sunflower, rapeseed, clover, tobacco, carrot, cotton, alfalfa, potato, eggplant, cucumber, *Arabidopsis thaliana,* and woody plants such as coniferous and deciduous trees, but particularly rice, maize, wheat, barley, cabbage, cauliflower, pepper, squash, melon, soybean, tomato, sugar-beet, sunflower or cotton, rice, maize, wheat, *Sorghum bicolor,* orchardgrass, sugar beet and soybean cells
- the host cell is a plant cell from a dicotyledonous plant
- the host cell is a plant cell from a dicotyledonous plant selected from the group consisting of soybean, cotton, tobacco, sugar beet and oilseed rape
- the host cell is a plant cell from a monocotyledonous plant
- the host cell is a plant cell from a monocotyledonous plant selected from the group consisting of maize, wheat, sorghum, rye, oats, turf grass, rice, and barley.

In addition, plants and the progeny thereof including seeds are provided stably transformed with a DNA sequence, a recombinant DNA molecule or a DNA expression vector according to the invention. In particular, wherein
- the plant is selected from the group consisting of rice, maize, wheat, barley, rye, sweet potato, sweet corn, bean, pea, chicory, lettuce, cabbage, cauliflower, broccoli, turnip, radish, spinach, asparagus, onion, garlic, pepper, celery, squash, pumpkin, hemp, zucchini, apple, pear, quince, melon, plum, cherry, peach, nectarine, apricot, strawberry, grape, raspberry, blackberry, pineapple, avocado, papaya, mango, banana, soybean, tomato, sorghum, sugarcane, sugar-beet, sunflower, rapeseed, clover, tobacco, carrot, cotton, alfalfa, potato, eggplant, cucumber, *Arabidopsis thaliana,* and woody plants such as coniferous and deciduous trees, but particularly rice, maize, wheat, barley, cabbage, cauliflower, pepper, squash, melon, soybean, tomato, sugar-beet, sunflower or cotton, rice, maize, wheat, *Sorghum bicolor,* orchardgrass, sugar beet and soybean.

The present invention further discloses
- the use of the DNA sequence according to the invention to express a nucleotide sequence of interest
- a method of producing a DNA sequence according to the invention, wherein the DNA is produced by a polymerase chain reaction wherein at least one oligonucleotide used comprises a sequence of nucleotides which represents a consecutive stretch of 15, 18, 20, 22, 24 or more base pairs of SEQ ID NO:1.

In order to ensure a clear and consistent understanding of the specification and the claims, the following definitions are provided:
CmYLCV: Cestrum yellow leaf curling virus.
DNA shuffling: DNA shuffling is a method to rapidly, easily and efficiently introduce rearrangements, preferably randomly, in a DNA molecule or to generate exchanges of DNA sequences between two or more DNA molecules, preferably randomly. The DNA molecule resulting from DNA shuffling is a shuffled DNA molecule that is a non-naturally occurring DNA molecule derived from at least one template DNA molecule.
Expression: refers to the transcription and/or translation of an endogenous gene or a transgene in plants. In the case of antisense constructs, for example, expression may refer to the transcription of the antisense DNA only.
Functionally equivalent sequence: refers to a DNA sequence which has promoter activity substantially similar to the CmYLCV full-length transcript promoter or parts thereof and which under stringent hybridizing conditions hybridizes with the said promoter sequences.
Gene: refers to a coding sequence and associated regulatory sequence wherein the coding sequence is transcribed into RNA such as mRNA, rRNA, tRNA, snRNA, sense RNA or antisense RNA. Examples of regulatory sequences are promoter sequences, 5'- and 3'-untranslated sequences and termination sequences. Further elements that may be present are, for example, introns.
Gene of interest: refers to any gene which, when transferred to a plant, confers upon the plant a desired characteristic such as antibiotic resistance, virus resistance, insect resistance, disease resistance, or resistance to other pests, herbicide tolerance, improved nutritional value, improved performance in an industrial process or altered reproductive capability. The "gene of interest" may also be one that is transferred to plants for the production of commercially valuable enzymes or metabolites in the plant.
Heterologous as used herein means of different natural or of synthetic origin. For example, if a host cell is transformed with a nucleic acid sequence that does not occur in the untransformed host cell, that nucleic acid sequence is said to be heterologous with respect to the host cell. The transforming nucleic acid may comprise a heterologous promoter, heterologous coding sequence, or heterologous termination sequence. Alternatively, the transforming nucleic acid may be completely heterologous or may comprise any possible combination of heterologous and endogenous nucleic acid sequences.
Leader region: region in a gene between transcription start site and translation start site.
Marker gene: refers to a gene encoding a selectable or screenable trait.
Operably linked to/associated with: a regulatory DNA sequence is said to be "operably linked to" or "associated with" a DNA sequence that codes for an RNA or a protein if the two sequences are situated such that the regulatory DNA sequence affects expression of the coding DNA sequence.
Plant: refers to any plant, particularly to seed plants
Plant cell: structural and physiological unit of the plant, comprising a protoplast and a cell wall. The plant cell may be in form of an isolated single cell or a cultured cell, or as a part of higher organized unit such as, for example, a plant tissue, or a plant organ.
Plant material: refers to leaves, stems, roots, flowers or flower parts, fruits, pollen, pollen tubes, ovules, embryo sacs, egg cells, zygotes, embryos, seeds, cuttings, cell or tissue cultures, or any other part or product of a plant
Promoter: refers to a DNA sequence that initiates transcription of an associated DNA sequence. The promoter region may also include elements that act as regulators of gene expression such as activators, enhancers, and/or repressors and may include all or part of the 5' non-translated leader sequence.
Recombinant DNA molecule: a combination of DNA sequences that are joined together using recombinant DNA technology.
Recombinant DNA technology: procedures used to join together DNA sequences as described, for example, in Sambrook et al., 1989, Cold Spring Harbor, NY: Cold Spring Harbor Laboratory Press.
Screenable marker gene: refers to a gene whose expression does not confer a selective advantage to a transformed cell, but whose expression makes the transformed cell phenotypically distinct from untransformed cells.
Selectable marker gene: refers to a gene whose expression in a plant cell gives the cell a selective advantage. The selective advantage possessed by the cells transformed with the selectable marker gene may be due to their ability to grow in the presence of a negative selective agent, such as an antibiotic or a herbicide, compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell in the presence of the selective agent, compared to the absence of the selective agent, has a positive effect on the transformed plant cell and a negative effect on the un-transformed plant cell, for example with respect to growth, and thus gives the transformed plant cell a positive selective advantage.
Sequence identity: the percentage of sequence identity is determined using computer programs that are based on dynamic programming algorithms. Computer programs that are preferred within the scope of the present invention include the BLAST (Basic Local Alignment Search Tool) search programs designed to explore all of the available sequence databases regardless of whether the query is protein or DNA. Version BLAST 2.0 (Gapped BLAST) of this search tool has been made publicly available on the Internet (currently http://www.ncbi.nlm.nih.gov/BLAST/). It uses a heuristic algorithm, which seeks local as opposed to global alignments and is therefore able to detect relationships among sequences, which share only isolated regions. The scores assigned in a BLAST search have a well-defined statistical interpretation. Said programs are preferably run with optional parameters set to the default values.
Transformation: refers to the introduction of a nucleic acid into a cell. In particular, it refers to the stable integration of a DNA molecule into the genome of an organism of interest TSWV: tomato spotted wilt virus

The present invention relates to DNA sequences obtainable from the genome of the Cestrum yellow leaf curling virus (CmYLCV). Preferred are DNA sequences which are obtainable from the CmYLCV full-length transcript promoter which are capable of driving expression of an associated nucleotide sequence of interest. DNA sequences comprising functional and/or structural equivalents thereof are also embraced by the invention. The present invention thus relates to DNA sequences that function as promoters of transcription of associated nucleotide sequences. The promoter region may also include elements that act as regulators of gene expression such as activators, enhancers, and/or repressors and may include the 5' non-translated leader sequence of the transcribed mRNA.

In a preferred embodiment of the invention, said DNA sequence confers constitutive expression to an associated nucleotide sequence. Constitutive expression means that the nucleotide sequence of interest is expressed at all times and in most tissues and organs. When tested in association with a GUS or CAT reporter gene in transient expression assays, the DNA sequence according to the invention confers high level of expression of the GUS or CAT reporter gene. Thus, the DNA sequence according to the invention is useful for high level expression of an associated nucleotide sequence of interest, which preferably is a coding sequence. It is known to the skilled artisan that the associated coding sequence of interest can be expressed in sense or in antisense orientation. Further, the coding sequence of interest may be of heterologous or homologous origin with respect to the plant to be transformed. In case of a homologous coding sequence, the DNA sequence according to the invention is useful for ectopic expression of said sequence. In one particular embodiment of the invention expression of the coding sequence of interest under control of a DNA sequence according to the invention suppresses its own expression and that of the original copy of the gene by a process called cosuppression.

The promoters of the present invention can be obtained from Cestrum yellow leaf curling virus (CmYLCV) genomic DNA, which can be isolated, for example, from infected *Cestrum parqui* plants. CmYLCV-infected *Cestrum parqui* plants are identified by their leaf mosaic and crinkled leaf malformation. This DNA is then sequenced and analyzed. Sequence comparison with sequences in the database shows that the genomic organization of the CmYLCV is related to the one of other members of the Caulimovirus family. CmYLCV contains 8 open reading frames. Sequence comparison of the CmYLCV gene products shows that gene I product is involved in intracellular movements: gene A encodes a small protein of unknown function, gene B codes for the aphid transmission factor; gene C encodes a virion associated protein; the gene IV is the major capsid protein; gene V codes for a reverse transcriptase and the gene VI activates in trans the translation of viral genes on full-length transcript. Of particular interest is the so-called CmYLCV full-length transcript promoter.

It is known to the skilled artisan that various regions of the CmYLCV full-length transcript promoter can be used according to the invention. One particular embodiment of the invention is the CmYLCV full-length transcript promoter region shown in SEQ ID NO:1, called the CmpD promoter. This sequence contains 350 bp of CmYLCV full-length transcript promoter and 320 bp of the CmYLCV full-length transcript 5' non-translated leader sequence. This sequence is obtained by sequencing the CmYLCV genomic DNA. The promoter and leader sequences respectively, are identified by comparison to sequences in the database. A variant of SEQ ID NO:1, called CmpE, wherein the CmYLCV full-length transcript 5' non-translated leader sequence is 318 bp instead of 320 bp in length, is shown in SEQ ID NO:19.

One preferred embodiment of the invention is the DNA sequence depicted in SEQ ID NO:2, called the CmpC promoter. The CmpC promoter is a fragment of the sequence shown in SEQ ID NO:1 and contains 346 bp of CmYLCV full-length transcript promoter, corresponding to base 5 to 350 of SEQ ID NO:1. This DNA sequence is obtainable by PCR with genomic DNA from Cestrum yellow leaf curling virus or from Cestrum yellow leaf curling virus-infected *Cestrum parqui* plants using forward primer Cmp1 (SEQ ID NO:13) and reverse primer CmpC2 (SEQ ID NO:14). The putative TATA-box of the CmpC promoter is located from base 308 to base 315 of SEQ ID NO:2. The CmpC promoter contains at least 3 putative enhancer regions. Enhancer region 1 having the nucleotide sequence CAAT is located from base 232 to base 235 of SEQ ID NO:2, and enhancer region 2 also having the nucleotide sequence CAAT is located from base 243 to base 246 of SEQ ID NO:2. Enhancer region 3 is located from base 246 to base 253 of SEQ ID NO:2 and has the nucleotide sequence TGACGTAA.

Another preferred embodiment of the invention is the DNA shown in SEQ ID NO:3, called the CmpS promoter. The CmpS promoter also is a fragment of the sequence shown in SEQ ID NO: 1 and contains the 346 bp of SEQ ID NO:2 plus 54 bp from the leader region of the CmYLCV full-length transcript promoter. The leader region is a nucleotide sequence preceding the coding region which is transcribed but not translated into protein. It is known to the skilled artisan that the leader region can contain regulatory elements with important functions in gene expression. The CmpS promoter is obtainable by PCR with genomic DNA from Cestrum yellow leaf curling virus or from Cestrum yellow leaf curling virus-infected *Cestrum parqui* plants using forward primer Cmp1 (SEQ ID NO:13) and reverse primer CmpS2 (SEQ ID NO:15). The CmpS promoter contains at least 2 putative enhancer regions in the leader region. Enhancer region 4 (GAGAGA) is located at base 354 to base 359 of SEQ ID NO:3 and enhancer region 5 (GAGAGAGA) is located at base 368 to base 375 of SEQ ID NO:3.

Yet another preferred embodiment of the invention is the sequences depicted in SEQ ID NO:4, called the CmpL promoter. As the preceding promoters, the CmpL promoter is a fragment of the sequence shown in SEQ ID NO: 1 and contains the 346 bp of SEQ ID NO:2 plus 288 bp of the CmYLCV full-length transcript 5' non-translated leader sequence. The CmpL promoter is obtainable by PCR with genomic DNA from Cestrum yellow leaf curling virus or from Cestrum yellow leaf curling virus-infected *Cestrum parqui* plants using forward primer Cmp1 (SEQ ID NO:13) and reverse primer CmpL2 (SEQ ID NO:16). A variant of SEQ ID NO:4, called CmpF, wherein the CmYLCV full-length transcript 5' non-translated leader sequence is 286 bp instead of 288 bp in length, is shown in SEQ ID NO:20.

It is known to the skilled artisan that the nucleotide sequences shown in SEQ ID NOs:1, 2, 3, 4, 19 and 20 can be extended at their 5-' and 3'-ends with homologous or heterologous nucleotide sequences. For example, the 5'-end of SEQ ID NOs:1, 2, 3, 4, 19 and 20 can be extended by all or part of the nucleotides shown in SEQ ID NO:6. SEQ ID NO:6 is naturally found upstream of SEQ ID NOs: 2, 3, 4 and 20 and contains part of ORF VI of CmYLCV (base 1 to base 100 of SEQ ID NO:6) as well as part of the CmYLCV full-length transcript promoter (base 101 to base 104 of SEQ ID NO:6). Likewise, the 3'-ends of SEQ ID NOs:2, 3, 4 and 20 can be extended by all or part of the nucleotides shown in SEQ ID NO:5, representing the nucleotide sequence naturally found at the 3'-end of SEQ ID NOs:4 and 20 and comprising part of the CmYLCV full-length transcript leader sequence.

As described above, the DNA sequences of the invention can be obtained, for example, by PCR with genomic DNA from Cestrum yellow leaf curling virus or from Cestrum yellow leaf curling virus-infected *Cestrum parqui* plants. Alternatively, the DNA sequences of the invention can be obtained from any other virus of the Caulimovirus family comprising homologues of the DNA sequence of the invention using sequence specific primers.

It is apparent to the skilled artisan that, based on the nucleotide sequences shown in SEQ ID NO:1 to SEQ ID NO:6 and SEQ ID Nos 19 and 20, any primer combination of interest can be chosen to PCR amplify DNA fragments of various lengths that can be used according to the invention. The invention thus includes fragments derived from the CmYLCV full-length transcript promoters that function according to the invention, i.e. are capable of conferring expression of an associated nucleotide sequence.

This can be tested by generating such promoter fragments, fusing them to a selectable or screenable marker gene and assaying the fusion constructs for retention of promoter activity. Such assays are within the ordinary skill of the person skilled in the art. Preferred DNA fragments of the invention are of at least about 50 bases, preferably of between about 400 bases and about 650 bases, more preferably of between about 200 bases and about 400 bases and most preferably of about 350 bases in length.

It is also clear to the skilled artisan that mutations, insertions, deletions and/or substitutions of one or more nucleotides can be introduced into the DNA sequences of SEQ ID NOs:1 , 2, 3, 4, 5 and 6 or longer or shorter fragments derived from the sequence information thereof using methods known in the art. In addition, an unmodified or modified nucleotide sequence of the present invention can be varied by shuffling the sequence of the invention. To test for a function of variant DNA sequences according to the invention, the sequence of interest is operably linked to a selectable or screenable marker gene and expression of the marker gene is tested in transient expression assays with protoplasts or in stably transformed plants. It is known to the skilled artisan that DNA sequences capable of driving expression of an associated nucleotide sequence are build in a modular way. Accordingly, expression levels from shorter DNA fragments may be different than the one from the longest fragment and may be different from each other. For example, deletion of a down-regulating upstream element will lead to an increase in the expression levels of the associated nucleotide sequence while deletion of an up-regulating element will decrease the expression levels of the associated nucleotide sequence. It is also known to the skilled artisan that deletion of development-specific or a tissue-specific element will lead to a temporally or spatially altered expression profile of the associated nucleotide sequence. Embraced by the present invention are also functional equivalents of the promoters of the present invention, i.e. nucleotide sequences that hybridize under stringent conditions to any one of SEQ ID NO:1 , SEQ ID NO:2, SEQ ID NO:3, SEQ ID NO:4, SEQ ID NO:5 or SEQ ID NO:6, such as, for example, the sequences shown in SEQ ID NO:19 and SEQ ID NO:20. A stringent hybridization is performed at a temperature of 65°C, preferably 60°C and most preferably 55°C in double strength (2X) citrate buffered saline (SSC) containing 0.1 % SDS followed by rinsing of the support at the same temperature but with a buffer having a reduced SSC concentration. Such reduced concentration buffers are typically one tenth strength SSC (0.1X SSC) containing 0.1% SDS, preferably 0.2X SSC containing 0.1% SSC and most preferably half strength SSC (0.5X SSC) containing 0.1% SDS. In fact, functional equivalents to all or part of the CmYLCV full-length transcript promoter from other organisms can be found by hybridizing any one of SEQ ID NO:1, SEQ ID NO:2, SEQ ID NO:3, SEQ ID NO:4, SEQ ID NO:5, SEQ ID NO:6, SEQ ID NO:19 or SEQ ID NO:20 with genomic DNA isolated from an organism of interest, particularly from another Caulimovirus. The skilled artisan knows how to proceed to find such sequences as there are many ways known in the art to identify homologous sequences in other organisms. Such newly identified DNA molecules then can be sequenced and the sequence can be compared to any one of SEQ ID NO:1, SEQ ID NO:2, SEQ ID NO:3, SEQ ID NO:4, SEQ ID NO:5, SEQ ID NO:6, SEQ ID NO: 19 or SEQ ID NO:20 and tested for promoter activity. Within the scope of the present invention are DNA molecules having at least 75%, preferably 80%, more preferably 90%, and most preferably 95% sequence identity to the nucleotide sequence of any one of SEQ ID Nos:1, 2, 3, 4 or 6 over a length of at least 50 nucleotides. The percentage of sequence identity is determined using computer programs that are based on dynamic programming algorithms. Computer programs that are preferred within the scope of the present invention include the BLAST (Basic Local Alignment Search Tool) search programs designed to explore all of the available sequence databases regardless of whether the query is protein or DNA. Version BLAST 2.0 (Gapped BLAST) of this search tool has been made publicly available on the internet (currently http://www.ncbi.nlm.nih.gov/BLAST/). It uses a heuristic algorithm which seeks local as opposed to global alignments and is therefore able to detect relationships among sequences which share only isolated regions. The scores assigned in a BLAST search have a well-defined statistical interpretation. Said programs are preferably run with optional parameters set to the default values.

It is another object of the present invention to provide recombinant DNA molecules comprising a DNA sequence according to the invention operably linked to a nucleotide sequence of interest. The nucleotide sequence of interest can, for example, code for a ribosomal RNA, an antisense RNA or any other type of RNA that is not translated into protein. In another preferred embodiment of the invention the nucleotide sequence of interest is translated into a protein product. The nucleotide sequence associated with the promoter sequence may be of homologous or heterologous origin with respect to the plant to be transformed. The sequence may also be entirely or partially synthetic. Regardless of the origin, the associated DNA sequence will be expressed in the transformed plant in accordance with the expression properties of the promoter to which it is linked. In case of homologous nucleotide sequences associated with the promoter sequence, the promoter according to the invention is useful for ectopic expression of said homologous sequences. Ectopic expression means that the nucleotide sequence associated with the promoter sequence is expressed in tissues and organs and/or at times where said sequence may not be expressed under control of its own promoter. In one particular embodiment of the invention, expression of nucleotide sequence associated with the promoter sequence suppresses its own expression and that of the original copy of the gene by a process called cosuppression.

In a preferred embodiment of the invention the associated nucleotide sequence may code for a protein that is desired to be expressed throughout the plant at all times and in most tissues and organs. Such nucleotide sequences preferably encode proteins conferring a desirable phenotypic trait to the plant transformed therewith. Examples are nucleotide sequences encoding proteins conferring antibiotic resistance, virus resistance, insect resistance, disease resistance, or resistance to other pests, herbicide tolerance, improved nutritional value, improved performance in an industrial process or altered reproductive capability. The associated nucleotide sequence may also be one that is transferred to plants for the production of commercially valuable enzymes or metabolites in the plant. Embraced by the present invention are also selectable or screenable marker genes, i.e. genes comprising a DNA sequence of the invention operably linked to a coding region encoding a selectable or screenable trait.

Examples of selectable or screenable marker genes are described below. For certain target species, different antibiotic or herbicide selection markers may be preferred. Selection markers used routinely in transformation include the *npt*II gene which confers resistance to kanamycin, paromomycin, geneticin and related antibiotics (Vieira and Messing, 1982, Gene 19: 259-268; Bevan et al., 1983, Nature 304:184-187) the bacterial *aad*A gene (Goldschmidt-Clermont, 1991, Null, Acids Res. 19: 4083-4089), encoding aminoglycoside 3'-adenylyltransferase and conferring resistance to streptomycin or spectinomycin, the *hph* gene which confers resistance to the antibiotic hygromycin (Blochlinger and Diggelmann, 1984, Mol. Cell. Biol. 4: 2929-2931), and the dhfr gene, which confers resistance to methotrexate (Bourouis and Jarry, 1983, EMBO J. 2: 1099-1104). Other markers to be used include a phosphinothricin acetyltransferase gene, which confers resistance to the herbicide phosphinothricin (White et al., 1990, Nucl. Acids Res. 18: 1062; Spencer et al. 1990, Theor. Appl. Genet. 79: 625-631), a mutant EPSP synthase gene encoding glyphosate resistance (Hinchee et al., 1988, Bio/Technology 6: 915-922), a mutant acetolactate synthase (ALS) gene which confers imidazolione or sulfonylurea resistance (Lee et al., 1988, EMBO J. 7: 1241-1248), a mutant psbA gene conferring resistance to atrazine (Smeda et al., 1993, Plant Physiol. 103: 911-917), or a mutant protoporphyrinogen oxidase gene as described in EP 0 769 059. Identification of transformed cells may also be accomplished through expression of screenable marker genes such as genes coding for chloramphenicol acetyl transferase (CAT), β-glucuronidase (GUS), luciferase (LUC), and green fluorescent protein (GFP) or any other protein that confers a phenotypically distinct trait to the transformed cell. Selection markers resulting in positive selection, such as a phosphomannose isomerase gene, as described in patent application WO 93/05163, are also used. Other genes to be used for positive selection are described in WO 94/20627 and encode xyloisomerases and phosphomanno-isomerases such as mannose-6-phosphate isomerase and mannose-1-phosphate isomerase; phosphomanno mutase; mannose epimerases such as those which convert carbohydrates to mannose or mannose to carbohydrates such as glucose or galactose; phosphatases such as mannose or xylose phosphatase, mannose-6-phosphatase and mannose-1-phosphatase, and permeases which are involved in the transport of mannose, or a derivative, or a precursor thereof into the cell. The agent which reduces the toxicity of the compound to the cells is typically a glucose derivative such as methyl-3-O-glucose or phloridzin. Transformed cells are identified without damaging or killing the non-transformed cells in the population and without co-introduction of antibiotic or herbicide resistance genes. As described in WO 93/05163, in addition to the fact that the need for antibiotic or herbicide resistance genes is eliminated, it has been shown that the positive selection method is often far more efficient than traditional negative selection.

Therefore, the promoters of the present invention are preferably operably linked to a nucleotide sequence which encodes a protein which comprises a region which: (a) encodes a protein which is involved in the metabolism of the compound, and/or (b) regulates the activity of the gene encoding the protein, wherein the compound is mannose or xylose or a derivative or a precursor of these, or a substrate of the protein, or is capable of being metabolized by the transformed cells into such a substrate. Said nucleotide sequence may encode a mannose-6-phosphate isomerase and the compound may be mannose. Alternatively, the nucleotide sequence may encode a phosphosugar-isomerase, a phosphosugar-mutase such as phosphomanno mutase, a phosphatase such as mannose-6-phosphatase, a sugar-epimerase, a sugar-permease, a phosphosugar-permease or a xylose isomerase, and the compound may be mannose, mannose-6-phosphate, D-mannoseamine or xylose.

In a preferred embodiment of the invention, the promoters of the invention are operably linked to the coding region of the *E*. *coli* manA gene (Joersbo and Okkels, 1996, Plant Cell Reports 16:219-221, Negrotto et al., 2000, Plant Cell Reports 19:798-803), encoding a phosphomannose isomerase (PMI), and a Nos (nopaline synthetase) terminator obtained from Agrobacterium tumefaciens T-DNA (Depicker et al., J. Mol. Appl. Genet. 1 (6), 561-573 (1982)). The skilled artisan knows that the Nos terminator may be substituted for by any other terminator that functions in plants. The promoter of the invention may be the CmpD, CmpC, CmpS, CmpL, CmpB, CmpA, CmpE or CmpF promoter, depicted in SEQ ID Nos 1 to 6 and 19 to 20, or any promoter derived therefrom. In an even more preferred embodiment of the invention, the promoter is the CmpS promoter depicted in SEQ ID NO:3.

The promoters of the present invention may also be used to provide resistance or tolerance to viruses by operatively linking the promoters of the invention with coding regions of genes from viruses to be controlled.

For virus control of negative strand viruses such as tomato spotted wilt virus (TSWV), the viral nucleocapsid protein (NP) and the movement protein (MP) gene sequences can be used, and for viruses belonging to the Alpha-like supergroup of viruses such as TMV and CMV, RNA-dependent RNA-polymerase (RdRp) and movement protein (MP) gene sequences can be used. For viruses belonging to the Picorna-like supergroup of viruses, including potyviruses, any viral sequence may be linked to the promoters of the invention. Finally, for all other viruses, including DNA viruses, RNA-dependent RNA-polymerase (RdRp) or replicase-associated gene sequences may be used. Such constructs for virus control can be constructed as exemplified in Example 8 and Example 9 of WO 00/68374. It is within the ordinary skill of the person skilled in the art to replace the promoters disclosed in WO 00/68374 with the promoters of the present invention. Following the teaching of WO 00/68374 the skilled artisan also knows how to prepare constructs comprising the promoters of the invention operably linked to a viral sequences as mentioned hereinbefore to confer resistance or tolerance to said virus.

Instead of expressing double stranded viral RNA in transgenic plants, as disclosed in WO 00/68374, the viral RNA can also be expressed as a non-translatable RNA plus-sense viral RNA molecule as described, for example, in US 558302 or in Examples 12 and 13 of the present invention.

It is a further objective of the invention to provide recombinant expression vectors comprising a DNA sequence of the invention fused to an associated nucleotide sequence of interest. In these vectors, foreign DNA can be inserted into a polylinker region such that these exogenous sequences can be expressed in a suited host cell which may be, for example, of bacterial or plant origin. For example, the plasmid pBI101 derived from the *Agrobacterium tumefaciens* binary vector pBIN19 allows cloning and testing of promoters using beta-glucuronidase (GUS) expression signal (Jefferson et al, 1987, EMBO J 6: 3901-3907). The size of the vector is 12.2 kb. It has a low-copy RK2 origin of replication and confers kanamycine resistance in both bacteria and plants. There are numerous other expression vectors known to the person skilled in the art that can be used according to the invention.

It is a further objective of the present invention to provide transgenic plants comprising the recombinant DNA sequences of the invention. The invention thus relates to plant cells, to plants derived from such cells, to plant material, to the progeny and to seeds derived from such plants, and to agricultural products with improved properties obtained by any one of the transformation methods described below. Plants transformed in accordance with the present invention may be monocots or dicots and include, but are not limited to, rice, maize, wheat, barley, rye, sweet potato, sweet corn, bean, pea, chicory, lettuce, cabbage, cauliflower, broccoli, turnip, radish, spinach, asparagus, onion, garlic, pepper, celery, squash, pumpkin, hemp, zucchini, apple, pear, quince, melon, plum, cherry, peach, nectarine, apricot, strawberry, grape, raspberry, blackberry, pineapple, avocado, papaya, mango, banana, soybean, tomato, sorghum, sugarcane, sugar-beet, sunflower, rapeseed, clover, tobacco, carrot, cotton, alfalfa, potato, eggplant, cucumber, *Arabidopsis thaliana,* and woody plants such as coniferous and deciduous trees. Preferred plants to be transformed are rice, maize, wheat, barley, cabbage, cauliflower, pepper, squash, melon, soybean, tomato, sugar-beet, sunflower or cotton, but especially rice, maize, wheat, *Sorghum bicolor,* orchardgrass, sugar beet and soybean. The recombinant DNA sequences of the invention can be introduced into the plant cell by a number of well-known methods. Those skilled in the art will appreciate that the choice of such method might depend on the type of plant which is targeted for transformation, i.e., monocot or dicot. Suitable methods of transforming plant cells include microinjection (Crossway et al., 1986, Bio Techniques 4:320-334), electroporation (Riggs and Bates, 1986, Proc. Natl. Acad. Sci., USA 83:5602-5606), Agrobacterium-mediated transformation (Hinchee et al., 1988, Bio/Technology 6:915-922; EP 0 853 675), direct gene transfer (Paszkowski et al., 1984, EMBO J. 3:2717-2722), and ballistic particle acceleration using devices available from Agracetus, Inc., Madison, Wisconsin and Dupont, Inc., Wilmington, Delaware (see, for example, US Patent No. 4,945,050 and McCabe et al., 1988, Bio/Technology 6:923-926). The cells to be transformed may be differentiated leaf cells, embryogenic cells, or any other type of cell.

In the direct transformation of protoplasts, the uptake of exogenous genetic material into a protoplast may be enhanced by the use of a chemical agent or an electric field. The exogenous material may then be integrated into the nuclear genome. The previous work is conducted in dicot tobacco plants, which resulted in the foreign DNA being incorporated and transferred to progeny plants (Paszkowski et al., 1984, EMBO J. 3:2712-2722; Potrykus et al., 1985, Mol. Gen. Genet 199:169-177). Monocot protoplasts, for example, of Triticum monococcum, Lolium multiflorum (Italian rye grass), maize, and Black Mexican sweet corn, are transformed by this procedure. An additional preferred embodiment is the protoplast transformation method for maize as disclosed in EP 0 292 435, as well as in EP 0 846 771. For maize transformation also see Koziel et al., 1993, Bio/Technology 11:194-200. Transformation of rice can be carried out by direct gene transfer techniques utilizing protoplasts or particle bombardment. Protoplast-mediated transformation is described for Japonica-types and Indica-types (Zhang et al., 1988, Plant Cell Rep., 7:379-384; Shimamoto et al., 1989, Nature 338:274-276; Datta et al., 1990, Bio/Technology 8:736-740). Both types described above are also routinely transformable using particle bombardment (Christou et al., 1991, Bio/Technology 9:957-962). Patent application No. EP 0 332 581 describes techniques for the generation, transformation and regeneration of Pooideae protoplasts. These techniques allow the transformation of all Pooideae plants including Dactylis and wheat. Furthermore, wheat transformation is described in patent application No. EP 0 674 715; and by Weeks et al., 1993 (Plant Physiol. 102:1077-1084). The thus-constructed plant expression vector can, for example, be introduced into the calli of rice according to the conventional plant transformation method, and the differentiation of roots and leaves is induced therefrom, and then, can be transferred to a flowerpot for cultivation, thereby obtaining the transformed rice.

The plants resulting from transformation with the DNA sequences or vectors of the present invention will express a nucleotide sequence of interest throughout the plant and in most tissues and organs.

The genetic properties engineered into the transgenic plants described above are passed on by sexual reproduction or vegetative growth and can thus be maintained and propagated in progeny plants. Generally said maintenance and propagation make use of known agricultural methods developed to fit specific purposes such as tilling, sowing or harvesting. Specialized processes such as hydroponics or greenhouse technologies can also be applied. Use of the advantageous genetic properties of the transgenic plants according to the invention can further be made in plant breeding that aims at the development of plants with improved properties such as tolerance of pests, herbicides, or stress, improved nutritional value, increased yield, or improved structure causing less loss from lodging or shattering. The various breeding steps are characterized by well-defined human intervention such as selecting the lines to be crossed, directing pollination of the parental lines, or selecting appropriate progeny plants. Depending on the desired properties different breeding measures are taken. The relevant techniques are well known in the art and include but are not limited to hybridization, inbreeding, backcross breeding, multiline breeding, variety blend, interspecific hybridization, aneuploid techniques, etc. Hybridization techniques also include the sterilization of plants to yield male or female sterile plants by mechanical, chemical or biochemical means. Cross pollination of a male sterile plant with pollen of a different line assures that the genome of the male sterile but female fertile plant will uniformly obtain properties of both parental lines. Thus, the transgenic plants according to the invention can be used for the breeding of improved plant lines that for example increase the effectiveness of conventional methods such as herbicide or pesticide treatment or allow to dispense with said methods due to their modified genetic properties. Alternatively new crops with improved stress tolerance can be obtained that, due to their optimized genetic "equipment", yield harvested product of better quality than products that were not able to tolerate comparable adverse developmental conditions.

It is another objective of the present invention to provide DNA sequences that can be used to express a nucleotide sequence of interest in a desired organism. This organism can be a bacterium, a plant or any other organism of interest.

Furthermore, the disclosure of SEQ ID NOs:1 to 6 enables a person skilled in the art to design oligonucleotides for polymerase chain reactions which attempt to amplify DNA fragments from templates comprising a sequence of nucleotides characterized by any continuous sequence of 15 and preferably 20 to 30 or more base pairs in SEQ ID Nos:1, 2, 3, 4, 5 or 6. Said nucleotides comprise a sequence of nucleotides which represents 15 and preferably 20 to 30 or more base pairs of SEQ ID Nos:1, 2, 3, 4, 5 or 6. Polymerase chain reactions performed using at least one such oligonucleotide and their amplification products constitute another embodiment of the present invention.

### BRIEF DESCRIPTION OF THE SEQUENCES IN THE SEQUENCE LISTING

| | |
|---|---|
| SEQ ID NO:1 | CmpD |
| SEQ ID NO:2 | CmpC |
| SEQ ID NO:3 | CmpS |
| SEO ID NO:4 | CmpL |
| SEQ ID NO:5 | CmpB |
| SEQ ID NO:6 | CmpA |
| SEQ ID NO:7 | S1 forward primer |
| SEQ ID NO:8 | S2 reverse primer |
| SEQ ID NO:9 | GUS forward primer |
| SEQ ID NO:10 | GUS reverse primer |
| SEQ ID NO:11 | CAT forward primer |
| SEQ ID NO:12 | CAT reverse primer |
| SEQ ID NO:13 | Cmp1 forward primer |
| SEQ ID NO:14 | CmpC2 reverse primer |
| SEQ ID NO:15 | CmpS2 reverse primer |
| SEQ ID NO:16 | CmpL2 reverse primer |
| SEQ ID NO:17 | PA forward primer |
| SEQ ID NO:18 | PA reverse primer |
| SEQ ID NO:19 | CmpE |
| SEQ ID NO:20 | CmpF |
| SEQ ID NO:21 | NOS terminator forward primer |
| SEQ ID NO:22 | NOS terminator reverse primer |
| SEQ ID NO:23 | TSWV NP gene forward primer |
| SEQ ID NO:24 | TSWV NP gene reverse primer |
| SEQ ID NO:25 | CmYLCV promoter forward primer |
| SEQ ID NO:26 | CmYLCV promoter reverse primer |
| SEQ ID NO:27 | AGLINK multicloning site |
| SEQ ID NO:28 | BIGLINK multicloning site |
| SEQ ID NO:29 | Cmpf-B primer |
| SEQ ID NO:30 | CmpS-B primer |
| SEQ ID NO:31 | pNOV2804; binary vector, contains a promoterless PMI-nos terminator expression cassette |
| SEQ ID NO:32 | pNOV3604; vector for transformation by biolistics, contains a promoterless PMI-nos terminator expression cassette |
| SEQ ID NO:33 | CmpMr reverse primer |
| SEQ ID NO:34 | CmpMf forward primer |
| SEQ ID NO:35 | CmpMrs reverse primer |
| SEQ ID NO:36 | CmpMfs forward primer |
| SEQ ID NO:37 | synGFPI; plant optimized GFP gene with intron |
| SEQ ID NO:38 | GIG; GUS gene with intron |
| SEQ ID NO:39 | Cmp-C reverse primer |
| SEQ ID NO:40 | CmpS-synGFPI-nos expression cassette |
| SEQ ID NO:41 | CmpS-GIG-nos expression cassette |
| SEQ ID NO:42 | CmpC-synGFPI-nos expression cassette |
| SEQ ID NO:43 | CmpC-GIG-nos expression cassette |
| SEQ ID NO:44 | pNOV2117 vector |
| SEQ ID NO:45 | pNOV4200 vector |
| SEQ ID NO:46 | ZmUbi-GFP-35S term expression cassette |
| SEQ ID NO:47 | ZmUbi-GIG-nos expression cassette |
| SEQ ID NO:48 | Ubq3(At)-synGFPI-nos expression cassette |
| SEQ ID NO:49 | Ubq3(At)-GIG-nos expression cassette |

### EXAMPLES

Standard recombinant DNA and molecular cloning techniques used here are well known in the art and are described, for example, by Sambrook et al., 1989, "Molecular Cloning", Cold Spring Harbor, Cold Spring Harbor Laboratory Press, NY and by Ausubel et al. ,1994, "Current protocols in molecular biology", John Wiley and Sons, New York.

### Example 1: Virus cloning

1. DNA extraction Virus genomic DNA is extracted from Cestrum yellow leaf curling virus-infected *Cestrum parqui* plants. Five grams of infected leaves are homogenized in 30 ml of grinding buffer (0.2 M Tris pH 7.0, 0.02 M EDTA, 2 M Urea) for 60 sec at maximum speed in a Brinkman Polytron homogenizer with PT10 probe and gently shaken at 4°C overnight with Triton X-100 (2% final concentration). Virus is purified from crude homogenate by low-speed centrifugation (10,000 rpm for 20 min in a Sorvall SS-34 rotor) and from the obtained supernatant by high-speed centrifugation (27,000 rpm for 2 hr in a Beckman SW-28 rotor) through a sucrose cushion (3 ml of 15% sucrose). The pellet-containing virus is then resuspended in 0.1 M Tris, pH 7.4, 2.5 mM MgCl₂. DNase I (Sigma) and RNase A (Sigma) are added to a concentration of 10 mg/ml each. After 30 min at 37°C, the reaction is stopped with the addition of EDTA to 10 mM. Virus DNA is isolated from CmYLCV particles by treating with protease K (E. Merck, 0.5 mg/ml final concentration) in the presence of 1% SDS at 65°C for 15 min. The viral DNA is then purified by phenol extraction and by ethanol precipitation. The virus DNA contained in the final ethanol precipitate is dissolved in water.
2. DNA amplification and cloning Hundred nanograms of the obtained DNA are used as a template for PCR amplification in a 50 µl reaction volume containing 10 µM of each primer, 25 mM each dNTP, 5 µl of pfu reaction buffer (Stratagene) and 2.5 units of pfu turbo DNA polymerase (Stratagene). A S1 forward primer (gaccacaaacatcagaag, SEQ ID NO:7) and a S2 reverse primer (caaacttattgggtaatc, SEQ ID NO:8) are used for the PCR reaction. Cycling parameters for PCR are: 1x (94°C for 1 min); 30x (94°C for 1 min, 50°C for 1 min, 72°C for 1 min); 1x (72°C for 10 min) Each single DNA fragment is cloned in pPCR-Script™ Amp SK(+) plasmid (Stratagene) according to the manufacturer's instructions.

### Example 2: DNA Sequencing

Sequencing of the DNA virus clones is carried out using the automated ABI PRISM 377 DNA sequencer (Perkin Elmer) and ABI PRISM dRhodamine terminator cycle sequencing kit (Perkin Elmer) according to the manufacturer's instructions. The described S1 primer and S2 primers (Example 1) are used for the sequencing reactions as well as the universal M13-20 and Reverse primers.

### Example 3: Construction of plasmids for transient expression

All the PCR reactions are carried out with Pfu polymerase (Stratagene) as described in Example 1.
1. Reporter genes amplification The beta-glucuronidase (GUS) and the chloramphenicol acetyltransferase (CAT) reporter genes are amplified. For GUS gene amplification GUS forward (cagggtaccactaaaatcacc, SEQ ID NO:9) and GUS reverse (aggggatccccaattcccc, SEQ ID NO:10) oligonucleotides are used at the annealing temperature of 60°C. CAT forward (aggggtaccatcgatatggag, SEQ ID NO:11) and Cat reverse (ttaggatccgccctgccac, SEQ ID NO:12) oligonucleotides are annealed at 62°C. The forward primers are designed to contain a *Kpn*I recognition site and the reverse ones a *BamH*I site.
2. CmYLCV promoter amplification Three different promoter fragments are selected. CmpC (SEQ ID NO:2), CmpS (SEQ ID NO:3), and CmpL (SEQ ID NO:4). Cmp1 forward primer (cttctagacaaagtggcagac, SEQ ID NO:13) is used for all the three amplifications at the annealing temperature of 52°C. It is modified (shown in bold) from the original sequence to contain a Xbal recognition site. The CmpC2 reverse primer (ttggtaccttaacaatgaggg, SEQ ID NO:14) is used for CmpC fragment amplification and the CmpS2 reverse primer (ctacttctaggtaccttgctc, SEQ ID NO:15) is used for the CmpS fragment. Both of them are modified to contain a *Kpn*I recognition site. The CmpL2 reverse primer (ttggtaccttaacaatgaggg, SEQ ID NO:16) is used for CmpL fragment amplification and it is modified to contain a *Cla*I restriction site.
3. Polyadenylation signal amplification The polyadenylation signal fragments is amplified from the cauliflower mosaic virus (CaMV) infectious clone (Franck et al., Cell 21 (1), 285-294, 1980). PA (polyadenylation signal amplification) forward (ggggatccccagtctctctc, SEQ ID NO:17) and PA reverse (gtgaattcgagctcggta, SEQ ID NO:18) oligonucleotides are used for PCR and annealed at 60°C. The forward primer is designed to contain a *BamH*I recognition site and the reverse one an *EcoR*I site.
4. Produced constructs
   - pCmpCG (CmpC promoter fragment + GUS gene + polyA)
   - pCmpSG (CmpS promoter fragment + GUS gene + polyA)
   - pCmpCC (CmpC promoter fragment + CAT gene + polyA)
   - pCmpSC (CmpS promoter fragment + CAT gene + polyA)
   - pCmpLC (CmpL promoter fragment + CAT gene + polyA)

   The cassettes containing the promoter fragment, the reporter genes and the polyadenylation signal are inserted in a pUC19 vector (Stratagene) restricted with *Xbal* and *EcoR*I.
5.Constructs used for comparison
   - pCapG (Cap promoter fragment + GUS gene + polyA)
   - pCapSG (CapS promoter fragment + GUS gene + polyA)
   - pCapC (Cap promoter fragment + CAT gene + polyA)

   Promoter fragments contained in these constructs are obtained from CaMV (Franck et al., Cell 21 (1), 285-294, 1980, see GenBank accession number V00141). GUS, CAT and polyA fragments are identical to those used for the CmYLCV constructs. Cap corresponds to the fragment from base -227 to base + 33 from TATA-box (base 7175 to base 7438 of the CaMV genome, see GenBank accession number V00141) and CapS corresponds to the fragment from base -227 to base + 82 from TATA-box (base 7175 to base 7486 of the CaMV genome, see GenBank accession number V00141). These positions correspond approximately to those chosen for the CmYLCV fragments.

### Example 4: Transient expression experiments

1. Suspension cultures and protoplast preparation
   *Orychophragmus violaceus.* Suspension cultures are maintained in 40 ml of MS medium (Murashige and Skoog, Physiol Plant 15, 474-497, 1962) including 100 mg/ml inositol, 2% sucrose, and 0.1 mg/ml 2,4D. Protoplasts are isolated from 4- to 5-day-old-cultures. Cell walls are digested at 26°C for 1 hr in 0.1% pectolyase Y23 (Seishin Pharmaceutical Co., Japan), 1% cellulase Onozuka R10 (Yakult Honsha Co., Japan), 0.4 M D-mannitol, and 0.1% MES, pH 5.5. Protoplasts are filtered through a 50 µm sieve and washed twice with electroporation (EP) solution (10 mM HEPES, 150 mM NaCl, 5 mM CaCl₂, 0.2 M mannitol, pH7.1).
   *Nicotiana plumbaginifolia.* Plants are maintained axenically on RPM2 medium (Blonstein et al., Mol Gen Genet 211, 252-259, 1988) plus 7 g/l bacto agar, pH 5.6. For protoplasts preparation, leaves are cut and incubated overnight at 26°C in a solution of 0.5% driselase (Fluka), 0.25 mM PVP 10 (polyvinylpyrrolidone MW 10000), 3.85 mM CaCl₂, 6 mg/l NAA, 2 mg/l BAP, and 0.5 M sucrose, pH 5.7. Protoplasts are filtered through a 100 µm sieve. Sucrose solution (0.6 M sucrose, 0.1% MES, and 15 mM CaCl₂, pH 5.7) is added to the protoplast suspension for the first purification step, and the suspension is overlaid with W5 solution (150 mM NaCl, 125 mM CaCl₂, 5 mM KCI, 6 mM glucose; Menczel et al., Theor Appl Genet 59, 191-195, 1981). Protoplasts are then washed once with W5 solution and finally with EP solution.
   *Oryza sativa.* Protoplasts are prepared from a morphogenic rice suspension culture established from *O. sativa* cv. Nipponbare as described (Datta et al., 1990, Bio/Technology 8:736-740).
2. Transient expression experiment Transfection by electroporation of 2x10⁶ *Orychophragmus violaceus* protoplasts in 0.66 ml EP buffer is carried out by discharging a 960 µF capacitor through a distance of 4 mm of protoplast suspension. The capacitor is loaded at the 450 Volts. Electroporation is performed in the presence of 5-10 µg of plasmid DNA, then protoplasts are cultivated 16 to 24 hours at 25°C. Transfection of 2x10⁶ *Nicotiana plumbaginifolia* protoplast in 0.3 ml suspension is carried out in the presence of 0.3 ml PEG (40% polyetyleneglycole 6000) and 5-10 µg of plasmid DNA. Protoplasts are cultivated in 0.4 ml K3 medium (Godall et al., Methods Enzymol 181, 148-161, 1990) for 16 to 24 hours at 25°C and added with 10 ml W5 buffer before harvesting.

Protein extracts are prepared by at least three cycles of freezing and thawing, and clarified by centrifugation.

### Example 5: CAT and GUS assays

For detection of CAT gene expression double antibody sandwich (DAS)-ELISA is carried out using a CAT ELISA kit (Boehringer) according to the manufacturer's instructions. CAT activity is measured with a Dynex MRXII apparatus. Samples for GUS assay are diluted in GUS buffer (0.05 M NaPO₄, pH 7, 0.01 M EDTA, 0.1% Triton-X-100, 0.1% Sarkosyl). The reaction is carried out in the presence of an equal amount of GUS-reaction buffer (100 ml/l 10x GUS buffer, 200 mg/l BSA, 705 mg/l 4-methylumbelliferyl-glucuronide) at 37°C and stopped with 2 M Ammediol. The activity is measured in a Titertek Fluoroskan II.

Both CAT and GUS assay results are normalized to a standard clone value. Results obtained from ten different experiments show that the various CmYLCV promoter fragments function as highly active promoters.

### N. plumbaginifolia protoplast transient expression

### GUS reporter gene

| | | |
|---|---|---|
| CmpCG | 100 | |
| CmpSG | 86,9 | +/-20% |
| CapG | 9 | +/-20% |
| CapSG | 38,9 | +/-15% |

### CAT reporter gene

| | | |
|---|---|---|
| CmpCC | 100 | |
| CmpSC | 78 | /-20% |
| CapSC | 89,5 | +/-15% |
| CmpLC | 9 | +/-20% |

### O.violaceus protoplast transient expression

### GUS reporter gene

| | | |
|---|---|---|
| CmpCG | 100 | |
| CmpSG | 84,6 | +/-15% |
| CapG | 9,6 | +/-15% |
| CapSG | 40,7 | +/-15% |

### CAT reporter gene

| | | |
|---|---|---|
| CmpCC | 100 | |
| CmpSC | 255 | +/-20% |
| CapSC | 546 | +/-15% |
| CmpLC | 10 | +/-20% |

### O. sativa protoplast transient expression

### GUS reporter gene

| | | |
|---|---|---|
| CmpCG | 100 | |
| CmpSG | 84,6 | ⁺/-15% |
| CapG | 9,6 | ⁺/-15% |
| CapSG | 40,7 | ⁺/-15% |

### Example 6: Preparation of Solutions and Media for Plant Regeneration and Transformation

Culture media GM, CIM and SIM are the media described by Valvekens et al. (1988, Proc. Natl. Acad. Sci. USA. 85: 5536-5540).

Culture medium GM contains the mineral salts of Murashige and Skoog (1962, Physiol. Plant. 15:473-497), 1.0 mg/l thiamine (stock 1 mg/ml), 0.5 mg/l pyridoxine HCl (stock 1 mg/ml), 0.5 mg/l nicotinic acid (stock 1 mg/ml), 0.5 g/l 2-(N-morpholino)ethanesulfonic acid (MES), 10 g/l sucrose, 8 g/l agar, with the pH adjusted to 5.8 with 1 N KOH. CIM contains the mineral salts and vitamins of B5 medium (Gamborg et al., 1968, Exp. Cell Res. 50:151-158), 0.5 g/l 2-(N-morpholino)ethanesulfonic acid (MES), 20 g/l glucose, 0.5 mg/l 2,4-dichlorophenoxyacetic acid (2,4-D) (stock 10 mg/ml in DMSO), 0.05 mg/l kinetin (stock 5 mg/ml in DMSO), pH 5.8. Solid CIM medium contains 8 g/l agar. SIM contains the mineral salts and vitamins of B5 medium (Gamborg et al., 1968, supra), 0.5 g/l 2-(N-morpholino)ethanesulfonic acid (MES), 20 g/l glucose, 5 mg/l N6-(2-isopentenyl)adenine (2iP) (stock 20 mg/ml in DMSO), 0.15 mg/l indole-3-acetic-acid (IAA) (stock 1.5 mg/ml in DMSO), 8 g/l agar, pH 5.8. SIM V750 K100 is SIM medium supplemented with 750 mg/l vancomycin and 100 mg/l kanamycin. SIM V500 K100 is SIM medium supplemented with 500 mg/l vancomycin and 100 mg/l kanamycin. GM K50 is GM medium supplemented with 50 mg/l kanamycin.

The culture media are all sterilized by autoclaving (20 min, 121°C). Vitamins are dissolved in water and added to media before autoclaving. Hormones are dissolved in dimethyl sulfoxide (DMSO). Antibiotics are dissolved in water and sterilized by filtration (0.22 µm). Hormones and antibiotics are added after autoclaving and cooling of the media to 65°C. In all cases 9-cm Petri dishes (Falcon, 3003) are used, except for GM and GM K50 which are usually poured into 15-cm Petri dishes (Falcon, 3025).

Plates with solid media are dried before usage in laminar flow to remove condensate.

### Example 7: Arabidopsis strain and growth conditions

*Arabidopsis thaliana* seeds ecotype Columbia (Col-0) wild type are purchased from Lehle Seeds, USA (1102 South Industrial Blvd. Suite D, Round Rock TX 78681, USA). Plants are grown at 22°C 16/8 hour light/dark cycle in pots in the mixture of 4 parts sand, 4 parts garden soil and 1 part agrilit.

### Example 8: Agrobacterium strain and culture

Vector plasmids are introduced into recipient *Agrobacterium tumefaciens* strain LBA4404 (Clontech) by triparental mating according to the protocol described by Walkerpeach and Velten ("Agrobacterium-mediated gene transfer to plant cells: Cointegrate and binary vector systems". in: Plant Molecular Biology Manual, B1: 1-19, 1994. Eds.: S.B. Gelvin, R.A., Schilperoort, Kluvers Acad. Publishers.). Mobilizing strain used is *E*. *coli* HB101 harboring conjugation plasmid pRK2013 (Ditta et al., 1980, Broad host range DNA cloning system from Gram-negative bacteria. Construction of gene bank of Rhizobium meliloti. Proc. Natl. Acad. Sci. USA 77: 7347-7351). Agrobacteria used for root transformation are grown in LB medium (Sambrook et al., 1989, "Molecular Cloning", Cold Spring Harbor, Cold Spring Harbor Laboratory Press, NY) without antibiotics at 28°C and 200 rpm.

### Example 9: Seed sterilization

Seeds are placed in 70% EtOH/0.05% Tween 20 for 1 minute in a 2 ml Eppendorf tube. 70% EtOH/0.05% Tween 20 is removed with a pipette and replaced with 5% NaOCI/0.05% Tween 20 for 15 minutes. Seeds are shaken regularly. The solution is removed in sterile conditions and the seeds are washed in sterile, distilled water 3 times for 10 minutes each. After the last wash seeds are keep in 0.5 - 1 ml water. Seeds can be used immediately or stored at 4°C for two - three weeks. Sterilized seeds (20-30) are transferred with forceps on GM medium in 15-cm Petri dishes. Seedlings are grown in vertically placed plates in a growth chamber (22°C; 16/8 hour light/dark cycle).

### Example 10: Transformation of root explants of Arabidopsis thaliana

Roots of three-week-old seedlings are used in the transformation procedure. Roots should not be green or brown. Green parts of seedlings are removed with scalpel and forceps. Remaining roots are collected and approximately 5 entire root systems are placed per plate with solid CIM medium. Roots are pressed gently onto the surface of the plate to ensure full contact with the medium, but they should not be dipped into the agar. Roots are incubated for three days in a growth chamber (22°C; 16/8 hour light/dark cycle). Roots are then transferred to a sterile Petri dish with filter paper wetted with liquid CIM medium and cut with a scalpel in 0.5-1 cm pieces. Root explants are then transferred to a 50 ml sterile Falcon tube containing 10 ml of liquid CIM medium. To this, 0.5 ml of an overnight *Agrobacterium* culture (OD 0.6-1) is added and incubate for 1-2 minutes while shaking gently. The liquid is poured out of the tube through sterile metal screens (50 mesh, Sigma, S-0895), which are kept with forceps. Roots usually remain on the wall of the tube close to its edge. Then the root explants are transferred to a sterile Petri dish with filter paper and briefly blotted dry to remove excess of liquid. Root explants are put onto plates with solid CIM medium and incubated in a growth chamber for 2 days under dim light (1.5-2 klux). Slight traces of overgrowth with *Agrobacterium* should be visible after the period of cocultivation. Root explants are then transferred to sterile 50 ml Falcon tubes with 20 ml of liquid CIM medium, supplemented with 1000 mg/l vancomycin. The Falcon tubes are then gently vortexed to remove the *Agrobacteria.* The liquid is poured out of the tube as described above and the explants are briefly blotted dry on filter paper. Explants are then transferred to plates containing SIM V750 K100 medium. Roots should be in a close contact with the medium. The explants are incubated in a growth chamber in normal conditions for one week and then transferred to SIM V500 K100 medium and incubated for an additional week. Then the amount of vancomycin is reduced to 250 mg/l. First shoots should appear at the end of the third week of cultivation on SIM media. Shoots are excised when 0.3-0.5-cm long, any residual callus is removed, and the shoots are transferred to 15-cm plates containing GM K50 medium. Max. 3 shoots are placed per plate. To get more shoots, the remaining root explants can be transferred to fresh SIM plates supplemented with 125 mg/l vancomycin and 100 mg/l kanamycin for additional two weeks. Rooted shoots can be transferred to soil to allow seed set. Shoots that do not root are transferred to Magenta jars (one per jar) containing GM medium to produce seeds *in vitro.*

Seeds from individual transgenic plants are germinated on GM K50 medium in growth chamber for 2 weeks. Phenotypically normal kanamycin resistant seedlings, which form green true leaves and branched root system, are selected for further analyses.

### Example 11: Histochemical B-glucuronidase (GUS) assay

*In vitro* grown seedlings or plants grown in soil are used in GUS assays. Either whole seedlings or dissected organs are dipped into GUS staining solution. GUS staining solution contains 1 mM 5-bromo-4-chloro-3-indolyl glucuronide (X-Gluc, Duchefa, 20 mM stock in DMSO), 100 mM Na-phosphate buffer pH 7.0, 10 mM EDTA pH 8.0, and 0.1 % Triton X100. Tissue samples are incubated at 37°C for 1-16 hours. If necessary samples can be cleared with several washes of 70% EtOH to remove chlorophyll.

### Example 12: Construction of tobacco transformation vector pZU627

All the PCR reactions are carried out with Platinum Pfx DNA Polymerase (Life Technologies) according to manufacturer's instructions.

### 1. NOS terminator amplification and cloning

The NOS terminator is amplified from pBINl9 (Bevan *et al* 1984) using the NOS terminator forward primer (SEQ ID NO:21) and NOS terminator reverse primer (SEQ ID NO:22). The forward primer is modified to contain a Pstl site and the reverse primer is modified to contain a HindIII site. The NOS terminator amplification product is cloned as Pstl / Hindlll fragment in same sites of pBluescript (Stratagene) resulting in plasmid pZU400A.

### 2. Amplification of the tomato spotted wilt virus nucleocapsid protein (TSWV NP) gene amplification and cloning

In order to obtain a non-translatable TSWV NP gene, modified TSWV NP gene primers are used to amplify the TSWV NP gene. The TSWV NP gene forward primer introduces a BamHl, a Sphl, a start and a stop codon (SEQ ID NO:23). The reverse primer is modified to introduce a PstI site (SEQ ID NO:24). The amplification product is cloned as BamHl / PstI fragment into the BamHl / PstI site of pZU400A upstream of and in same direction as the NOS terminator. In the resulting clone pZU400b the PstI site between the TSWV NP gene and the NOS terminator is removed using a T4 DNA polymerase treatment (Life Technologies) according to manufacturer's instructions. From the resulting clone denoted pZU400C, the TSWV NP gene and NOS terminator are cloned as a Sphl / HindIII fragment into the Sphl / HindIII site of shuttle vector pZO1560 resulting in plasmid pZU400. pZO1560 is a pbluescript derivative in which the original multicloning site is replaced by the AGLINK multicloning site (SEQ ID NO:27).

### 3. CmYLCV Promoter amplification and cloning

The CmYLCV viral promoter is amplified using CmYLCV promoter forward primer (SEQ ID NO:25) and CmYLCV promoter reverse primer (SEQ ID NO:26). The forward primer is modified to contain a SstI site and the reverse primer is modified to contain a PstI site. The amplified CmYLCV viral promoter is cloned as SstI / PstI fragment, in same direction as and upstream of the TSWV-N gene, into the SstI / PstI site of pZU400. This results in clone pZU625 that contains a viral gene cassette that produces a non-translatable TSWV NP RNA.

### 4. Cloning of viral gene cassette to pVictorHiNK

The viral gene cassette is cloned as AscI / PacI fragment from pZU625 into the AscI / PacI site of pVictorHiNK vector pZU494. This results in the plant transformation vector pZU627. pVictorHiNK is a binary vector comprising an origin of replication (ORI) derived from *Pseudomonas aeruginosa* plasmid pVS1 which is known to be highly stable in *A. tumefaciens* (Itoh et al., 1984. Plasmid 11:206-220; Itoh and Haas, 1985. Gene 36;27-36). The pVS1 ORI is only functional in *Agrobacterium* and can be mobilized by the helper plasmid pRK2013 from *E.coli* into *A. tumefaciens* by means of a triparental mating procedure (Ditta et al, 1980. Proc. Natl. Acad. Sci. USA 77:7347-7351).

This binary vector also comprises a ColEl origin of replication which is functional in *E. coli* and derived from pUC19 (Yannisch-Perron et al., 1985. Gene 33:103-119).

For maintenance in *E.coli* and *A. tumefaciens* this vector contains a bacterial resistance gene to spectomycin and streptomycin encoded by a 0.93 kb gene from transposon Tn7 (Fling et al., 1985. Nucl. Acid Res. 13:7095) which functions as selection marker for maintenance of the vector in *E. coli* and *A. tumefaciens.* The gene is fused to the tac promoter for efficient bacterial expression (Amman et al., 1983. Gene 25: 167-178).

The right and left T-DNA border fragments of 1.9 kb and 0.9 kb, respectively that comprise the 24 bp border repeats, are derived from the Ti-plasmid of the nopaline type *A. tumefaciens* strains pTil37 (Yadev et al., 1982. Proc. Natl. Acad. Sci. USA. 79:6322-6326). Subsequently the T-DNA region between the borders is modified by deleting the M13 derived sequences and, to improve its cloning versatility, the BIGLINK multicloning site (SEQ ID NO:28) is introduced yielding pVictorHiNK.

PVictorHiNK contains an NPTII gene cassette for selection of transformants during the plant transformation process. This gene cassette contains a NOS promoter, the NPTII gene and the NOS terminator, obtained from *A. tumefaciens.*

### Example 13: Plant transformation and screening for resistance

### 1. Transformation of binary vectors to plant material

Methods to transfer binary vectors to plant material are well established and known to a person skilled in the art. Variations in procedures exist due to for instance differences in used *Agrobacterium* strains, different sources of explant material, differences in regeneration systems depending on as well the cultivar as the plant species used. Binary vector pZU627 is used in plant transformation experiments according to the following procedures. PZU627 is transferred by tri-parental mating to an acceptor *Agrobacterium tumefaciens* strain, followed by Southern blot analysis of the ex-conjugants for verification of proper transfer of the construct to the acceptor strain, inoculation and co-cultivation of axenic explant material with the recombinant *Agrobacterium tumefaciens* strain, selective killing of the Agrobacterium tumefaciens strain using the appropriate antibiotics, selection of transformed cells by growing on selective media containing kanamycine, transfer of plantlets to soil, assaying for the intactness of the integrated T-DNA by Southern blot analysis of isolated chromosomal DNA of the plant.

### 2. Resistance of plants against TSWV infections

Transformed plants are grown in the greenhouse under standard quarantine conditions in order to prevent any infection by pathogens. At a four-leaf stage the plants are infected with TSWV by mechanical inoculation. Tissue from plants systemically infected with TSWV is ground in 5 volumes of ice-cold inoculation buffer (10 mM phosphate buffer supplemented with 1% Na₂SO₃) and rubbed in the presence of carborundum powder on the first two fully extended new leaves. The inoculated plants are monitored for symptom development during three weeks after inoculation. Plants containing pZU627 sequences do not show TSWV symptoms, whereas untransformed control plants show severe systemic TSWV symptoms within 7 days after inoculation. Resistant plants are self-pollinated and the seeds harvested. Progeny plants are analyzed for segregation of the inserted gene and subsequently re-screened for resistance against TSWV infection as described above.

### Example 14: Construction of CmpS-phosphomannose isomerase-nos constructs for plant transformation

The CmpS promoter is PCR amplified using the Cmpf-B (cgc gga tcc tgg cag aca aag tgg cag a; SEQ ID NO:29) and the CmpS-B (cgc gga tcc tac ttc tag gct act tg, SEQ ID NO:30) primers having flanking BamHI sites. The resulting PCR fragment is cloned into the BamHl site of pBluescript KS (+) to form pNOV4211.

To create a binary vector for transformation via *Agrobacterium tumefaciens*, the CmpS promoter is excised from pNOV4211 using BamHl and inserted into BamHI-digested pNOV2804 (SEQ ID NO:31) upstream of the PMI gene, and the resulting vector called pNOV2819. pNOV2804 (SEQ ID NO:31) is a binary vector with VS1 origin of replication, a copy of the *Agrobacterium* virG gene in the backbone, and a promoterless PMI-nos terminator expression cassette between the left and right borders of T-DNA. PMI (phosphomannose isomerase) is the coding region of the *E.coli* manA gene (Joersbo and Okkels, 1996, Plant Cell Reports 16:219-221, Negrotto et al., 2000, Plant Cell Reports 19:798-803). The nos (nopaline synthase) terminator is obtained from *Agrobacterium tumefaciens* T-DNA (Depicker et al., 1982, J. Mol. Appl. Genet. 1 (6), 561-573. The phosphomannose isomerase coding region and the nos terminator are located at nt 290 to nt 1465 and nt 1516 to 1789 respectively, of pNOV2804 (SEQ ID NO:31).

To create a vector for biolistic transformation, the CmpS promoter is excised from pNOV4211 using BamHl and inserted into BamHl-digested pNOV3604 (SEQ ID NO:32) to form pNOV2820, thus creating a PMI expression cassette driven by the CmpS promoter. pNOV3604 is a vector for preparing biolistic fragments and is based on pUC19 with an ColEl origin of replication and a beta-lactamase gene conferring ampicillin-resistance. As pNOV2804, pNOV3604 also contains a promoterless PMI-nos terminator expression cassette (see above). The phosphomannose isomerase coding region and the nos terminator are located at nt 42 to nt 1217 and nt 1268 to 1541 respectively, of pNOV3604 (SEQ ID NO:32).

Binary vector pNOV2819 is used for transformation via *Agrobacterium tumefaciens* and vector pNOV2820 is used for biolistic transformation.

### Example 15: Construction of CmpC-GUS plasmids for stable expression in planta.

### 1.Construction of the binary vector

The vector pCambia 1302 (Cambia, Canberra, Australia; Roberts, C.S., Rajagopal, S., Smith, L., Nguyen, T., Yang, W., Nugroho, S., Ravi, K.S. Cao, M.L., Vijayachandra, K., et al. A comprehensive set of modular vectors for advanced manipulation and efficient transformation of plants. Presented at the Rockefeller Foundation Meeting of the international Program on Rice Biotechnology, Malacca, Malaysia, September 15-19, 1997) is chosen and modified for the experiments: The CaMV 35S promoter in front of the GFP gene is removed by digestion at position 9788 with *Hind*III endonuclease and at position 1 with Ncol endonuclease and the vector is relegated at the two compatible ends. Digestion with XhoI endonuclease at positions 7613 and BstXl at position 9494 excise the hygromycin gene and the CaMV 35S promoter in front of it. The 35S promoter sequences are eliminated from the vector to exclude any risk of homology-mediated gene silencing.

The bar gene driven by the 1' promoter (Mengiste et al., Plant J, 1997, 12(4): 945-948) is introduced in the *Eco*RI site at position 9737 as a selectable marker. The obtained vector is named pCamBar.

### 2. Produced constructs

The cassettes CmpCG and CapG described in Example 3 are inserted in the pCamBar vector between *Xba*I site at position 9764 and *Eco*RI at position 9737 to obtain the pCamBarCmpCG and the pCamBarCapG plasmids, respectively. The two constructs are used to transform *Agrobacterium tumefaciens* cells.

### Example 16: Stable expression in Arabidopsis thaliana

### 1. Plant production and transfection

*Arabidopsis thaliana* (Columbia 0) wt seeds are sown, cold-treated 3 days at 4 °C in the dark to synchronize germination and transferred to growth room (22°C/ 24h light). Germinated plants are infiltrated when they have produced a maximum number of unopened buds. The infiltration is carried out according to the protocol described by Clough and Bent, Plant J. 16: 735-743, 1987.

### 2. Selection of transgenic plants

Seedlings obtained from T1-generation seeds are selected by spraying with the BASTA herbicide (Plüss-Staufer AG/SA) (150 mg/l) every five days for three times. Twenty pCamBarCmpCG and nine pCamBarCapG resistant plants are collected after selection. They are grown under the described condition to obtain the T2-generation seeds. These seeds undergo the same procedure as described above for the wt, the T2 seedlings are selected by BASTA spraying, and the resistant mutants analyzed for GUS-gene expression.

### 3. Histochemical GUS expression in stably transformed Arabidolosis plants

Histochemical GUS staining is done as described in Example 11. The analysis is performed in 15 ml culture tubes by immersion of the transformed seedlings in the X-gluc solution, application of 130 mBar pressure for 10 min and incubation at 37°C overnight. Results obtained from Arabidopsis plants transformed with pCamBarCmpCG indicate a constitutive expression of the GUS reporter gene driven by the CmpC promoter in all vegetative organs.

### 4. Quantitative enzymatic GUS assays in stably transformed Arabidopsis plants

### A. Sample preparation

Four plants per transgenic line and one leaf per each of these plants are selected for the test. The four leaves are collected in the same eppendorf tube, frozen with liquid nitrogen and ground to fine powder with disposable grinders. This is diluted in 150 µl GUS buffer (0.05M NaPO₄, pH7, 0.01 M EDTA, 0.1% Triton-X-100, 0.1% Sarkosyl), incubated 5 min at 37°C and spun in a microcentrifuge for 5 min at maximum speed. The supernatant is transferred to a new eppendorf tube and these samples are used for the enzymatic test. Protein in these plant extracts is estimated according to the Bradford method (Bradford, M.M. Anal. Biochem. 72: 248-254, 1976) using BSA as a standard. The presence of the promoter and GUS gene in the mutant lines is confirmed by PCR analysis using the extracted samples as a template.

### B. Fluorometric GUS assay

The fluorometric GUS assay is done as described in Example 5. For detection of GUS gene expression samples are diluted in GUS buffer. The reaction is carried out in the presence of an equal amount of GUS-reaction buffer at 37°C and stopped with 2 M Ammediol. The activity is measured in a Titertek Fluoroskan II.

Fifteen out of the twenty selected lines transformed with pCamBarCmpCG and eight out of nine lines transformed with pCamBarCapG show GUS enzymatic activity in leaves. The level of activity in several CmpCG transformed lines is comparable to CapG. However the results are variable for the different transgenic lines due to the difference in their genotypes (number of loci and number of transgene insertions per locus).

### Example 17: Construction of CmpC promoter variants

All the PCR reactions are carried out with Pfu polymerase (Stratagene) as described in Example 1.
1. DNA amplification and cloning Two variants, pCmpMG and pCmpMsG, of the pCmpCG plasmid (Example 3) are produced. The former deletes the sequence "GTGGTCCC" in the CmpC promoter and the latter mutates it to the sequence "GTGCTCGC".

To obtain pCmpMG three PCR products are amplified:
CmpM1 using the Cmp1 forward primer (see Example 3, SEQ ID NO:13), the CmpMr reverse primer (CCATCGTGGTATTTGGTATTG, SEQ ID NO:33) and the pCmpCG plasmid as a template.
CmpM2 using the CmpMf forward primer (CAATACCAAATACCACGATGG; SEQ ID NO:34), the CmpC2 reverse primer (see Example 3; SEQ ID NO:14) and the pCmpCG plasmid as a template.
CmpM using the Cmp1 forward primer, the CmpC2 reverse primer and an equimolar mix of CmpM1 and CmpM2 PCR products as a template.

To obtain pCmpMsG three PCR product are amplified:
CmpMs1 using the Cmp1 forward primer, the CmpMrs reverse primer (CGTGGTAGCGAGCACTTTGGT, SEQ ID NO:35) and the pCmpCG plasmid as a template.
CmpMs2 using the CmpMfs forward primer (AAGTGCTCGCTACCACGATGG, SEQ ID NO:36), the Cmp2 reverse primer and the pCmpCG plasmid as a template.
CmpsM using the Cmp1 forward primer, the Cmp2 reverse primer and an equimolar mix of CmpMs1 and CmpMs2 PCR products as a template.

To obtain the pCmpMG and the pCmpMsG plasmids the original pCmpCG plasmid is restricted with *Xba*I and *BamH*I endonucleases to eliminate the CmpC fragment and to insert in place of it the CmpM and the CmpMs PCR products.

### Example 18: Transient expression experiments with pCmpM and pCmpMs constructs

### 1. Suspension cultures and protoplast preparation

Suspension cultures and protoplasts are produced as described in Example 4.

### 2. GUS assays

Transfection and protein extract preparation are carried out as described in the Example 4, and the GUS assay is carried out as described in Example 5.

GUS assay results are obtained from the average of ten different experiments and normalized to a standard clone value. The deletion of the "GTGGTCCC" sequence in the CmpCG construct reduces expression of the GUS reporter gene to about 50% of the GUS reporter gene expression from CmpC in all protoplast species (see below). The effect induced by the "GTGCTCGC" mutation depends on the plant species. In *O. violaceus* and *O. sativa* protoplasts the level of GUS gene expression is decreased to 65.2% and 73.6%, respectively. In *N. plumbaginifolia* protoplasts the CmpMs promoter activity is similar to the CmpM promoter.

### N. plumbaginifolia

| | | |
|---|---|---|
| CmpCG | 100 | |
| CmpMG | 52 | ⁺/-12% |
| CmpMsG | 53.7 | ⁺/-16% |
| CapG | 28.3 | ⁺/-14% |

### O. violaceus

| | | |
|---|---|---|
| CmpCG | 100 | |
| CmpMG | 55.2 | ⁺/-12% |
| CmpMsG | 73.6 | ⁺/-10% |
| CapG | 5 | ⁺/- 1% |

### O. sativa

| | | |
|---|---|---|
| CmpCG | 100 | |
| CmpMG | 59.6 | ⁺/-21% |
| CmpMsG | 65.2 | ⁺/-20% |
| CapG | 42.5 | ⁺/-16% |

### Example 19: Construction of plant transformation vectors containing 5'-promoter fragments operably linked to GFP or GUS reporter genes

### 1. Promoter amplification and cloning

A chimeric gene is constructed that includes a DNA sequence from the CmYLCV full-length transcript promoter (SEQ ID NO:1) fused to the plant optimized GFP with intron (synGFPI, SEQ ID NO:37) or GUS reporter gene with intron (GIG; SEQ ID NO:38) sequence. The GIG gene contains the ST-LS1 intron from *Solanum tuberosum* at nt 385 to nt 576 of SEQ ID NO: 38 (obtained from Dr. Stanton Gelvin, Purdue University, and described in Narasimhulu, et al 1996, Plant Cell, 8: 873-886.). SynGFPI is a plant optimized GFP gene into which the ST-LS1 intron is introduced (nt 278 to nt 465 of SEQ ID NO:37). For the promoters, the CmYLCV genomic DNA is used as a template for the polymerase chain reaction (PCR). Gene specific primers are designed to amplify the DNA sequence. A gene fragment corresponding to CmpC (SEQ ID NO:2) is isolated using the Cmpf-B forward primer (SEQ ID NO:29) in combination with the 5' to 3' primer CGCGGATTGCTCCCTTAACAATGAGG (SEQ ID NO:39) as reverse primer. A gene fragment corresponding to CmpS (SEQ ID NO:3) is isolated using the Cmpf-B (SEQ ID NO:29) forward primer in combination with the CmpS-B reverse primer (SEQ ID NO:30). All three primers contain the BamHI restriction enzyme recognition site CGCGGA at their 5' end. All PCR reactions are carried out with Pfu polymerase according to manufacturers recommendations (Stratagene). A thermocycler (DNA Engine, MJResearch, Inc. Waltham, MA USA) is used to amplify the promoter fragments using the following PCR conditions: [(94°C:10s):(94°C:10s/56°C:30s/72°C:1min)X20]:(72°C:1.5m)]. Following restriction digestion with BamHl the promoter fragments are ligated into pUC-based vectors with the GIG gene or synGFPI gene to create the promoter-reporter gene fusions operatively linked with a nos terminator.

### 2. Produced promoter-reporter cassettes

- CmpS promoter fragment + synGFPI gene + nos (SEQ ID NO:40)
- CmpS promoter fragment + GIG gene + nos (SEQ ID NO:41)
- CmpC promoter fragment + synGFPI gene + nos (SEQ ID NO:42)
- CmpC promoter fragment + GIG gene + nos (SEQ ID NO:43)

| SEQ ID NO | Promoter | Gene (synGFPI or GIG) | terminator |
|---|---|---|---|
| 40 | nt 1 to nt 402 | nt 411 to nt 1331 | nt 1343 to nt 1618 |
| 41 | nt 1 to nt 405 | nt 425 to nt 2425 | nt 2479 to nt 2751 |
| 42 | nt 1 to nt 354 | nt 380 to nt 1292 | nt 1304 to nt 1577 |
| 43 | nt 1 to nt 354 | nt 399 to nt 2399 | nt 2453 to nt 2725 |

### 3. Subcloning into Agrobacterium binary vector

The promoter-reporter cassettes (SEQ ID NO:40 to 43) containing the promoter fragment, the reporter gene and the nos terminator are inserted into binary vector pNOV2117 for maize and binary vector pNOV4200 for tomato transformation. pNOV2117 (SEQ ID NO:44) is a binary vector with VS1 origin of replication, a copy of the *Agrobacterium* virG gene in the backbone, and a Maize Ubiquitin promoter- PMI gene-nos terminator expression cassette between the left and right borders of T-DNA. PMI (phosphomannose isomerase) is the coding region of the *E.coli* manA gene (Joersbo and Okkels, 1996, Plant Cell Reports 16:219-221, Negrotto et al., 2000, Plant Cell Reports 19:798-803). The nos (nopaline synthase) terminator is obtained from *Agrobacterium tumefaciens* T-DNA (Depicker et al., 1982, J. Mol. Appl. Genet. 1 (6), 561-573). The maize ubiquitin promoter, the phosphomannose isomerase coding region and the nos terminator are located at nt 31 to nt 2012, nt 2109 to nt 3212 and nt 3273 to 3521 respectively, of pNOV2117 (SEQ ID NO:44). The reporter-promoter cassettes are inserted closest to the right border. The selectable marker expression cassette in the binary vectors is closest to the left border.

For tomato transformation, pNOV4200 (SEQ ID NO:45) is used as the binary vector, which contains the VS1 origin of replication, a copy of the *Agrobacterium* virG gene in the backbone, and a hygromycin selectable marker cassette between the left and right border sequences. The hygromycin selectable marker cassette comprises the Arabidopsis Ubiquitin 3 promoter (Ubq3(At), Callis et al., J. Biol. Chem. 265:12486-12493 (1990)) operably linked to the gene encoding hygromycin resistance (denoted here as "HYG", synthetic hygromycin B phosphotransferase gene from *E.coli,* Patent: JP 1986085192-A1 30-APR-1986) and the nos terminator (Depicker et al., J. Mol. Appl. Genet. 1 (6), 561-573 (1982). The Arabidopsis ubiquitin promoter, HYG gene and nos terminator are located at nt 162 to nt 11494, nt 1897 to nt 2939 and nt 2939 to nt 3236, respectively of pNOV4200 (SEQ ID NO:45). The reporter-promoter cassettes (SEQ ID NO:40 to 43) are inserted closest to the right border. The selectable marker expression cassette in the binary vectors is closest to the left border.

Shown below are the orientations of the selectable marker and promoter-reporter cassettes in the binary vector constructs.

### 4. Binary Vector Constructs:

- NOV4215 (RB CmpS promoter fragment + synGFPI gene + nos - ZmUbi + PMI gene + nos LB)
- NOV4216 (RB nos + synGFPI gene + CmpS promoter fragment - ZmUbi + PMI gene+ nos LB)
- NOV4217 (RB CmpS promoter fragment + synGFPI gene + nos - Ubq3(At) + HYG gene + nos LB)
- OV4220 (RB CmpS promoter fragment + GIG gene + nos - Ubq3(At) + HYG gene + nos LB)
- NOV4224 (RB CmpC promoter fragment + GIG gene + nos - ZmUbi + HYG gene + nos LB)
- NOV4226 (RB CmpC promoter fragment + synGFPI gene + nos - ZmUbi + PMI gene + nos LB)

Additional cassettes are constructed with known promoters to be used for comparison. To this end, a promoter cassette, ZmUbi-GFP-35S term (SEQ ID NO:46), comprising the maize Ubiquitin promoter (Christensen *et al.* Plant Molec. Biol. 12: 619-632 (1989)) operatively linked with synGFP and the 35S terminator (35S term) is constructed and a promoter cassette comprising the maize ubiquitin promoter operatively linked with the GIG gene and the nos terminator (NOV 3612, SEQ ID NO:47) is also constructed The ZmUbi promoter, GFP gene and 35S terminator are located at nt 1 to nt 2019, nt 2020 to nt 2751 and nt 2876 to nt 2949, respectively of SEQ ID NO:46. The ZmUbi promoter, GIG gene and nos terminator are located at nt 1 to nt 1982, nt 2015 to nt 4015 and nt 4069 to nt 4341, respectively of SEQ ID NO:47. The ZmUbi-GIG nos cassette (SEQ ID NO: 47) is cloned into a pUC-based vector. The ZmUbi- GFP-nos cassette (SEQ ID NO:46 ) is cloned into the pNOV2117 binary vector for maize transformation, as described above. Promoter cassettes comprising the Arabidopsis Ubiquitin3 promoter (Ubq3(At)) operatively linked to synGFPI and the nos terminator (SEQ ID NO:48) and the Arabidopsis Ubiquitin3 promoter operatively linked to GIG gene and the nos terminator (SEQ ID NO:49) are constructed. The Ubiqutin3 promoter, synGFPI and the nos terminator are located at nt 1 to nt 1332, nt 1738 to nt 2658 and nt 2670 to nt 2943, respectively of SEQ ID NO: 48. The Ubiqutin3 promoter, GIG gene and the nos terminator are located at nt 1 to nt 1335, nt 1746 to nt 3746 and nt 3800 to nt 4072, respectively of SEQ ID NO: 49. As described above, the promoter cassettes are cloned into the pNOV4200 binary vector containing a hygromycin selectable marker cassette for tomato transformation. Shown below are the orientations of the selectable marker and promoter-reporter cassettes in the binary vector constructs.

### 5. Binary vector constructs

- NOV2110 (RB ZmUbi Promoter + synGFP gene + 35S term - ZmUbi + PMI gene + nos LB)
- NOV4209 (RB Ubq3(At) promoter + synGFPI gene + nos - Ubq3(At) + HYG gene + nos LB)
- NOV4208 (RB Ubq3(At) promoter + GUS -Intron -GUS gene + nos - Ubq3(At) + HYG gene + nos LB)

### Example 20: Agrobacterium-mediated transformation of maize

Transformation of immature maize embryos is performed essentially as described in Negrotto et al., (2000) Plant Cell Reports 19: 798-803. For this example, all media constituents are as described in Negrotto et al., *supra.* However, various media constituents described in the literature may be substituted.

### 1. Transformation plasmids and selectable marker

The genes used for transformation are cloned into a vector suitable for maize transformation as described in Example 19. Vectors used contain the phosphomannose isomerase (PMI) gene (Negrotto et al. (2000) Plant Cell Reports 19: 798-803).

### 2. Preparation of Agrobacterium tumefaciens

*Agrobacterium* strain LBA4404 (pSB1) containing the plant transformation plasmid is grown on YEP (yeast extract (5 g/L), peptone (10g/L), NaCl (5g/L),15g/l agar, pH 6.8) solid medium for 2 to 4 days at 28°C. Approximately 0.8X 10⁹ *Agrobacteria* are suspended in LS-inf media supplemented with 100 µM acetosyringone (As) (Negrotto et al.,(2000) Plant Cell Rep 19: 798-803). Bacteria are pre-induced in this medium for 30-60 minutes.

### 3. Inoculation

Immature embryos from A188 or other suitable maize genotypes are excised from 8 - 12 day old ears into liquid LS-inf + 100 µM As. Embryos are rinsed once with fresh infection medium. *Agrobacterium* solution is then added and embryos are vortexed for 30 seconds and allowed to settle with the bacteria for 5 minutes. The embryos are then transferred scutellum side up to LSAs medium and cultured in the dark for two to three days. Subsequently, between 20 and 25 embryos per petri plate are transferred to LSDc medium supplemented with cefotaxime (250 mg/l) and silver nitrate (1.6 mg/l) and cultured in the dark for 28°C for 10 days.

### 4. Selection of transformed cells and regeneration of transformed plants

Immature embryos producing embryogenic callus are transferred to LSD1M0.5S medium. The cultures are selected on this medium for 6 weeks with a subculture step at 3 weeks. Surviving calli are transferred either to LSD1M0.5S medium to be bulked-up or to Reg1 medium. Following culturing in the light (16 hour light/ 8 hour dark regiment), green tissues are then transferred to Reg2 medium without growth regulators and incubated for 1-2 weeks. Plantlets are transferred to Magenta GA-7 boxes (Magenta Corp, Chicago III.) containing Reg3 medium and grown in the light. Plants that are PCR positive for the promoter-reporter cassette are transferred to soil and grown in the greenhouse.

### Example 21: Stable transformation of tomato

Transformation of tomato is performed essentially as described in Meissner et al., The Plant Journal 12: 1465-1472,1997.

### 1. Transformation plasmids and selectable marker

The genes used for transformation are cloned into a binary vector suitable for tomato transformation as described in Example 19. Vectors contain the hygromycin resistance gene for selection.

### 2. Preparation of Agrobacterium tumefaciens

*Agrobacterium* strain GV3101 containing the plant transformation plasmid is grown on LB (yeast extract (5 g/L), peptone (10g/L), NaCl (5g/L), 15g/l agar, pH 6.8) liquid media for 2 days at 22°C. Approximately 0.8X 10⁹ *Agrobacteria* are suspended in KCMS inoculation media (MS salts (4.3g/l), Myo-Inositol, Thiamine (1.3 ug/ml), 2,4-D (0.2 ug/ml), Kinetin 0.1ug/ml and 3% Sucrose, pH 5.5) supplemented with 100 µM acetosyringone. Bacteria are pre-induced in this medium for 60 minutes.

### 3. Seeds sterilization and germination.

Seeds of Micro-Tom cultivar of tomato (Meissner et al., The Plant Journal 12: 1465-1472,1997) and ZTV-840 cultivars are soaked in 20% bleach solution with Tween for 20 min. Seeds are then washed three times with sterile water and plated on TSG media (½ MS saltes, 1% Sucrose, 1% PhytoAgar, pH 5.8) in Magenta boxes (Magenta Corp, Chicago III) for germination. Cotyledons are excised and cotyledon petioles are cut into 5 mm segments 7 to 10 days after germination.

### 4. Inoculation

Seven to ten day old cotyledons and cotyledonal petioles segments are incubated for 24 hrs on tobacco BY-2 feeder cells plates (MS salts, Myo-inisitol 0.1 mg/ml, Casein Hydrolysate 0.2 mg/ml, Thiamine -HCl 1.3 µg/ml, 3% Sucrose, pH 5.5) prior to inoculation with *Agrobacterium.*

Cotyledons are dipped into liquid KCMS Agrobacterium suspension for 5 minutes. Between 20 and 25 cotyledons are then transferred abaxial side upward on the same feeder cell plates and cultured in the dark for two to three days.

### 5. Selection of transformed cotyledons and petiols and regeneration of transformed plants

Cotyledons and petiole fragments are transferred onto selection media TSM-1 (4.3g/l MS salts, 1X B5 Vitamins, 2% Sucrose, 1% Glucose, 1 µg/ml Zeatin, 0.05 µg/ml IAA, 5 µg/ml Hygromicine and 250 µg/ml Carbenicillin, pH 5.8) two to three days after inoculation with *Agrobacterium* in the light. Cotyledons and petiole segments producing embryogenic callus are transferred to TSM-2 medium (MS Salts 4.3 g/l, 1X MS Vitamins, 2% Sucrose, 1 µg/ml Zeatin, 5 µg/ml Hygromicine and 250 µg/ml Carbenicillin, pH 5.8). Surviving calli forming plantlets are transferred to Magenta GA-7 boxes (Magenta Corp, Chicago III.) containing TRM medium (4.3 g/l MS salt, 1X MS Vitamins, 2% Sucrose pH 5.8) and grown in the light. After rootformation primary transformants are transferred to soil.

### Example 22: Green Fluorescent Protein (GFP) fluorometric assay

For detection of synGFP gene expression the fluorometric assay is performed.

Leaf discs of stably transformed maize and tomato are frozen on dry ice. Frozen tissue is throughly ground and mixed with 300 µl of GFP Extraction Buffer (10 mM Tris-HCl (pH 7.5), 100 mM NaCl, 1 mM MgCl2, 10 mM DTT and 0.1% Sarcosyl). The extracts are separated from the leaf debris by centrifugation followed by transfer into 96 well black plate with clear bottom (Costar 3615). Relative fluoresence units (RFU) are measured by Spectra Fluor Plus Plate Reader at 465 nm excitation and 512 nm emission. GFP activity is normalized to total protein measured using BCA Assay (according to Pierce).

### Example 23: Expression analysis of stably transformed maize and tomato plants

### 1. Zea mays transgenic To plants

GFP expression is 10 to 20 times higher under the control of the CmpS promoter (NOV4215, NOV4216) compared to the ZmUbi promoter (NOV2110). These results are obtained from leaf samples of 42 independent To lines representing a total of 93 To plants.

### 2. GFP Elisa assay

A quantitative sandwich immunoassay is carried out for the detection of green fluorescent protein (GFP). Two commercially available polyclonal antibodies are used. The goat anti-GFP IAP (Rockland, #600-1-1-215) is immunoaffinity purified and the rabbit anti-GFP antibody (Torrey Pines Biolabs, #TP401) is protein A purified. The GFP protein in the plant extract is captured onto the solid phase microtiter by the rabbit antibody. Then a "sandwich" is formed between the solid phase rabbit antibody, the GFP protein, and the secondary goat antibody that is in the well. After a wash step, where unbound secondary antibody is removed, the bound antibody is detected using an alkaline phosphatase-labeled antibody. Substrate for the enzyme is added and color development is measured by reading the absorbance of each well. The reading is then fit to a standard curve to plot the GFP concentrations versus the absorbance

### synGFP reporter gene

| | **GFP Activity** | **ELISA** |
|---|---|---|
| **Binary vector Cassette** | **(Average)** | **(Average)** |
| | RFU/mf protein | ng GFP/mg protein |
| NOV4215 (CmpS) | 4722.3 | 657.1 |
| NOV4216 (CmpS) | 2795.8 | 246.1 |
| NOV2110 (ZmUbi) | 272.0 | 41.4 |

### 2. Lycopersicon esculentum To plants

The CmpS fragment of CmYLCV promoter (NOV 4217) results in synGFP expression level that is significantly higher then the expression level of Ubq3 promoter of *Arabidopsis* (NOV 4209).

### synGFP reporter gene

The results are obtained from evaluating of intensity of synGFP fluorescence by microscopy in callus and primary shoots of 5 independent stably transformed callus lines transformed with the NOV4217 cassette and 2 independent stably transformed callus lines transformed with the NOV4209 cassette.

| | Callus ID | GFP Fluorescence Intensity |
|---|---|---|
| Binary vector Cassette | | |
| (Promoter) | | |
| NOV4217 (CmpS) | NOV4217-1 | +++++ |
| NOV4217 (CmpS) | NOV4217-2 | ++++ |
| NOV4217 (CmpS) | NOV4217-3 | ++++ |
| NOV4217(CmpS) | NOV4217-4 | +++ |
| NOV4217 (CmpS) | NOV4217-5 | +++ |
| NOV4209 (Ubq3 (At)) | NOV4209-1 | + |
| NOV4209 (Ubq3 (At)) | NOV4209-2 | + |

### Gus-Intron-GUS reporter gene

The results are obtained from evaluating of intensity of GUS staining in 11 independent stably transformed callus lines containing the CmpS promoter-reporter cassettes (lines NOV4220-1 through NOV4220-11). Two stably transformed callus lines containing CmpC promoter fragment of CmYLCV promoter (NOV4224-1, NOV4224-2) are evaluated. For comparison two stably transformed callus lines containing the Arabidopsis Ubiquitin 3 (Ubq3) promoter (NOV4208-1, NOV4208-2) are evaluated.

| Binary vector cassette | Callus ID | GUS Staining |
|---|---|---|
| (Promoter) | | |
| NOV4220 (CmpS) | NOV4220-1 | ++++ |
| NOV4220 (CmpS) | NOV4220-2 | +++ |
| NOV4220 (CmpS) | NOV4220-3 | ++++ |
| NOV4220 (CmpS) | NOV4220-4 | +++++ |
| NOV4220 (CmpS) | NOV4220-5 | +++++ |
| NOV4220 (CmpS) | NOV4220-6 | +++ |
| NOV4220 (Cmps) | NOV4220-7 | ++++ |
| NOV4220 (CmpS) | NOV4220-8 | +++++ |
| NOV4220 (CmpS) | NOV4220-9 | ++++ |
| NOV4220 (CmpS) | NOV4220-10 | +++ |
| NOV4220 (CmpS) | NOV4220-11 | ++++ |
| NOV4224 (CmpC) | NOV4224-1 | ++++ |
| NOV4224 (CmpC) | NOV4224-2 | +++ |
| NOV4208 (Ubq3 (At)) | NOV4208-1 | + |
| NOV4208 (Ubq3(At)) | NOV4208-2 | ++ |

### Example 24: Transient expression experiments in maize

### 1. Embryos preparation

Immature embryos from A188 or other suitable maize genotypes are excised from 8 - 12 day old ears into liquid 2DG4 + 0.5d (Duncan's D medium modified to contain 20mg/l glucose and supplemented with 10g/l mannose) and cultured for 1 to 2 days.

### 2. Plasmolysis

Immature embryos were incubated in 12% sucrose solution for 3-4 hour prior bombardment. Embryos are arranged in 8-10 mm circles on a plate.

### 3. Bombardment

Transfection by Particle Bombardment of Maize embryos is performed in the presence of 5 µg of plasmid DNA at 650 psi as described (Wright et al, 2001, Plant Cell Reports, In Press) In case an 'in press' reference won't do, here are the details lifted from the paper:

Fragment DNA is precipitated onto gold microcarriers (<1 um) as described in the DuPont Biolistics Manual. Genes are delivered to the target tissue cells using the PDS-1000He Biolisitcs device. The settings on the device are as follows: 8mm between the rupture disk and the macrocarrier, 10mm between the macrocarrier and the stopping screen and 7cm between the stopping screen and the target. The plates are shot twice with DNA-coated particles using 650 psi-rupture disks. To reduce tissue damage from the shock wave of the helium blast, a stainless steel mesh, 200 openings per lineal inch horizontally and vertically (McMaster-Carr, New Brunswick, NJ) is placed between the stopping screen and the target tissue.. Embryos are cultivated on the plates for 48 hrs in the dark at 25°C. Protein extracts are prepared by lysing cells in GUS lysis buffer and clarified by centrifugation.

### Example 25: GUS assays

For detection of GUS gene expression the histochemical and chemiluminescent assays are performed.

### 1. Histochemical B-glucuronidase (GUS) assay

Maize embryos and tomato *in vitro* callus lines are used in GUS assays. Either whole embryos or small pieces of callus are dipped into GUS staining solution. GUS staining solution contains 1 mM 5-bromo-4-chloro-3-indolyl glucuronide (X-Gluc, Duchefa, 20 mM stock in DMSO), 100 mM Na-phosphate buffer pH 7.0, 10 mM EDTA pH 8.0, and 0.1% Triton X100. Tissue samples are incubated at 37°C for 1-16 hours. If necessary, samples are cleared with several washes of 70% EtOH to remove chlorophyll

### 2. B-glucuronidase (GUS) chemilluminescent assay

For quantitive analysis of Gus expression the GUS chemilluminescent assay is performed using GUS-Light Kit (Tropix. Inc) according to manufacturer's instructions.

The activity is measured in a Luminometer.

The GUS results with construct pCmpS and pCmpMG (Example 17) are normalized to a comparison clone value (pNOV3612, Example 19). Results obtained from two different experiments show that the 8 bp deletion in CmYLCV promoter (pCmpMG, Example 17) does not significantly affect the expression levels of the GUS reporteras compared to pCmpS.

Transient expression in *Z.mays* embryos

### GUS Activity at 48 and 72 Hours after transfection

| **Construct ID** | **GUS Activity** RFU/mg protein | |
|---|---|---|
| | 48hr | 72hr |
| pCmpS | **103.7** | **51.7** |
| pCmpMG | **81.4** | **70.8** |
| ZmUbi (pNOV3612) | **52.6** | **70.2** |

## Claims

1. DNA sequence comprising
(a) a nucleotide sequence selected from the group consisting of SEQ ID NO: 1, SEQ ID NO: 2, SEQ ID NO: 3, SEQ LD NO: 4, SEQ ID NO: 19 and SEQ ID NO: 20, or
(b) the complement of a DNA sequence which hybridizes under stringent conditions to any one of SEQ ID NO: 1, SEQ ID NO: 2, SEQ ID NO: 3 or SEQ ID NO: 4, wherein said complement functions as a promoter of transcription of an associated nucleotide sequence,
(c) a DNA sequence comprising a consecutive stretch of at least 50 nt of SEQ ID NO: 1, wherein said DNA sequence functions as a promoter of transcription of an associated nucleotide sequence.

2. The DNA sequence according to claim 1 wherein said DNA sequence comprises a nucleotide sequence selected from the group consisting of SEQ ID NO: 2, SEQ ID NO: 3, SEQ ID NO: 4 and (SEQ ID NO: 20 and wherein said DNA sequence additionally comprises at its 3' end the nucleotide sequence depicted in SEQ ID NO: 5.

3. The DNA sequence according to claim 1 or claim 2, additionally comprising at its 5'-end the nucleotide sequence depicted in SEQ ID NO: 6.

4. Recombinant DNA molecule comprising a DNA sequence according to any one of claims 1 to 3 operably linked to a nucleotide sequence of interest.

5. The recombinant DNA molecule of claim 4, wherein the nucleotide sequence of interest comprises a coding sequence.

6. The recombinant DNA molecule of claim 5, wherein the coding sequence encodes a desirable phenotypic trait.

7. The recombinant DNA molecule of claim 6, wherein the coding sequence encodes a protein which confers a positive selective advantage to cells that have been transformed with said coding sequence.

8. The recombinant DNA molecule of claim 6 or 7, wherein the coding sequence encodes a protein which confers a metabolic advantage to cells that have been transformed with said coding sequence consisting of being able to metabolise a compound, wherein said compound is (i) mannose or xylose of a derivative or 8. precursor of these, or (ii) a substrate of the protein, or (iii) is capable of being metabolised by cells transformed with said coding sequence into such a substrate.

9. The recombinant DNA molecule of claim 8, wherein said coding sequence encodes an enzyme selected from the group of xyloisomerases, phosphomanno-isomerase, mannose-6-phosphate isomerase, mannose-1-phosphate isomerase, phosphomanno mutase, mannose epimerase, mannose or xylose phosphatase, mannose-6-phosphatase, mannose-1-phosphatase and mannose or xylose permease.

10. The recombinant molecule of claim 9, wherein the coding sequence encodes a phosphomanno isomerase.

11. The recombinant DNA according to claim 5, wherein the coding region is non-translatable.

12. The recombinant DNA according to claim 11, wherein the non-translatable coding region is from a viral gene.

13. The recombinant DNA according to claim 12, wherein the viral gene is derived from TSWV in particular from the TSWV NP gene.

14. The recombinant DNA molecule of claim 5, wherein the coding sequence is in antisense orientation.

15. A DNA expression vector comprising a DNA sequence according to any one of claims 1 to 3 or a recombinant DNA molecule of any one of claims 4 to 14.

16. A DNA expression vector of claim 15 comprising a first DNA sequence according to any one of claims 1 to 3 operably linked to a nucleotide sequence of interest, and a second DNA sequence according to any one of claims 1 to 3 linked to a nucleotide sequence of interest.

17. A DNA expression vector according to claim 16 capable of altering the expression of a viral genome.

18. The DNA expression vector according to claim 17 comprising a first DNA sequence capable of expressing in a cell a sense RNA fragment of said viral genome or portion thereof and a second DNA sequence capable of expressing in a cell an antisense RNA fragment of said viral genome or portion thereof, wherein said sense RNA fragment and said antisense RNA fragment are capable of forming a double-stranded RNA.

19. The DNA expression vector according to claim 18, wherein said virus is selected from the group consisting of tospoviruses, potyviruses, potexviruses, tobamoviruses, luteoviruses, cucumoviruses, bromoviruses, closteoviruses, tombusviruses and furoviruses.

20. The DNA expression vector of claim 19, wherein said DNA sequences comprises a nucleotide sequence derived from a viral coat protein gene, a viral nucleocapsid protein gene, a viral replicase gene, or a viral movement protein gene or portions thereof.

21. The DNA expression vector of claim 20, wherein said DNA sequence is derived from tomato spotted wilt virus (TSWV).

22. The DNA expression vector of claim 21, wherein said DNA is derived from a nucleocapsid protein gene.

23. A host cell stably transformed with a DNA sequence according to any one of claims 1 to 3 or a recombinant DNA molecule of any one of claims 4 to 14 or a DNA expression vector according to anyone of claims 15 to 22.

24. The host cell of claim 23, wherein said host cell is a plant cell.

25. A plant and the progeny thereof stably transformed with a DNA sequence according to any one of claims 1 to 3 or a recombinant DNA molecule of any one of claims 4 to 14 or a DNA expression vector according to anyone of claims 15 to 22.

26. The plant of claim 25, wherein said plant is selected from the group consisting of maize, wheat, sorghum, rye, oats, turf grass, rice, barley, soybean, cotton, tobacco, sugar beet and oilseed rape.

27. Use of the DNA sequence of any one of claims 1 to 3 to express a nucleotide sequence of interest.

28. A method of producing a DNA sequence according to claim 1, wherein the DNA is produced by a polymerase chain reaction wherein at least one oligonucleotide used comprises a sequence of nucleotides which represents a consecutive stretch of 15 or more base pairs of SEQ ID NO: 1.

## Patentansprüche

1. DNA-Sequenz umfassend
(a) eine Nucleotidsequenz, die aus der Gruppe ausgewählt ist, die aus SEQ ID Nr. 1, SEQ ID Nr. 2, SEQ ID Nr. 3, SEQ ID Nr. 4, SEQ ID Nr. 19 und SEQ ID Nr. 20 besteht, oder
(b) das Komplement einer DNA-Sequenz, die unter stringenten Bedingungen mit irgendeiner von SEQ ID Nr. 1, SEQ ID Nr. 2, SEQ ID Nr. 3 oder SEQ ID Nr. 4 hybridisiert, wobei dieses Komplement als Promotor zur Transkription einer damit verbundenen Nucleotidsequenz dient,
(c) eine DNA-Sequenz, die eine konsekutive Abfolge von mindestens 50 Nt der SEQ ID Nr. 1 umfasst, wobei die DNA-Sequenz als Promotor zur Transkription einer damit verbundenen Nucleotidsequenz dient.

2. Die DNA-Sequenz gemäß Anspruch 1, wobei die DNA-Sequenz eine Nucleotidsequenz umfasst, die aus der Gruppe ausgewählt ist, die aus SEQ ID Nr. 2, SEQ ID Nr. 3, SEQ ID Nr. 4 und SEQ ID Nr. 20 besteht, und wobei die DNA-Sequenz zusätzlich an ihrem 3'-Ende die Nucleotidsequenz, die in SEQ ID Nr. 5 wiedergegeben ist, umfasst.

3. DNA-Sequenz gemäß Anspruch 1 und Anspruch 2, welche zusätzlich an ihrem 5'-Ende die in SEQ ID Nr. 6 wiedergegebene Nucleotidsequenz umfasst.

4. Rekombinantes DNA-Molekül, das eine DNA-Sequenz gemäß irgendeinem der Ansprüche 1 bis 3, operativ verbunden mit einer Nucleotidsequenz von Interesse, umfasst.

5. Rekombinantes DNA-Molekül nach Anspruch 4, wobei die Nucleotidsequenz von Interesse eine kodierende Sequenz umfasst.

6. Rekombinantes DNA-Molekül nach Anspruch 5, wobei die kodierende Sequenz ein gewünschtes phänotypisches Merkmal kodiert.

7. Rekombinantes DNA-Molekül nach Anspruch 6, wobei die kodierende Sequenz ein Protein kodiert, welches Zellen einen positiven Selektionsvorteil verleiht, die mit dieser kodierenden Sequenz transformiert worden sind.

8. Rekombinantes DNA-Molekül nach Anspruch 6 oder 7, wobei die kodierende Sequenz ein Protein kodiert, welches einen metabolischen Vorteil an Zellen verleiht, die mit dieser kodierenden Sequenz transformiert worden sind, was darin besteht, dass es in der Lage ist, eine Verbindung zu metabolisieren, wobei die Verbindung (i) Mannose oder Xylose oder ein Derivat oder Vorläufer hiervon ist, oder (ii) ein Substrat des Proteins oder (iii) in der Lage ist, von Zellen, die mit dieser kodierenden Sequenz transformiert worden sind, in ein solches Substrat metabolisiert zu werden.

9. Rekombinantes DNA-Molekül nach Anspruch 8, wobei die kodierende Sequenz ein Enzym kodiert, welches aus der Gruppe ausgewählt ist, die aus Xylosiomerasen, Phosphomanno-isomerase, Mannose-6-phosphatisomerase, Mannose-1-phosphatisomerase, Phosphomannomutase, Mannoseepimerase, Mannose- oder Xylosephosphatase, Mannose-6-phosphatase, Mannose-1-phosphatase und Mannose- oder Xylosepermease.

10. Rekombinantes Molekül nach Anspruch 9, wobei die kodierende Sequenz eine Phosphomannoisomerase kodiert.

11. Rekombinante DNA gemäß Anspruch 5, wobei die kodierende Region nicht translatierbar ist.

12. Rekombinante DNA gemäß Anspruch 11, wobei die nicht translatierbare kodierende Region aus einem viralen Gen stammt.

13. Rekombinante DNA gemäß Anspruch 12, wobei das virale Gen von TSWV stammt, insbesondere vom TSWV NP-Gen.

14. Rekombinantes DNA-Molekül nach Anspruch 5, wobei die kodierende Sequenz in Gegensinn-Richtung vorliegt.

15. DNA-Expressionsvektor umfassend eine DNA-Sequenz gemäß irgendeinem der Ansprüche 1 bis 3, oder ein rekombinantes DNA-Molekül irgendeines der Ansprüche 4 bis 14.

16. DNA-Expressionsvektor nach Anspruch 15, umfassend eine erste DNA-Sequenz gemäß irgendeinem der Ansprüche 1 bis 3, operativ verbunden mit einer Nucleotidsequenz von Interesse und eine zweite DNA-Sequenz gemäß irgendeinem der Ansprüche 1 bis 3, welche mit einer Nucleotidsequenz von Interesse verbunden ist.

17. DNA-Expressionsvektor gemäß Anspruch 16, welcher in der Lage ist, die Expression eines viralen Genoms zu verändern.

18. DNA-Expressionsvektor gemäß Anspruch 17, umfassend eine erste DNA-Sequenz, die in der Lage ist, ein Sinn-RNA-Fragment dieses viralen Genoms oder einen Teil davon zu exprimieren, und eine zweite DNA-Sequenz, die in der Lage ist, in einer Zelle ein Gegensinn-RNA-Fragment dieses viralen Genoms oder eines Teils davon zu exprimieren, wobei dieses Sinn-RNA-Fragment und dieses Gegensinn-RNA-Fragment in der Lage sind, eine doppelsträngige RNA zu bilden.

19. DNA-Expressionsvektor gemäß Anspruch 18, wobei das Virus aus der Gruppe, bestehend aus Tospoviren, Potyviren, Potexviren, Tobamoviren, Luteoviren, Cucumoviren, Bromoviren, Closteoviren, Tombusviren und Furoviren, ausgewählt ist.

20. DNA-Expressionsvektor nach Anspruch 19, wobei die DNA-Sequenzen eine Nucleotidsequenz umfassen, die von einem viralen Hüllproteingen, einem viralen Nucleocapsidproteingen, einem viralen Replicasegen oder einem viralen Bewegungsproteingen oder Teilen davon stammt.

21. DNA-Expressionsvektor nach Anspruch 20, wobei diese DNA-Sequenz aus dem Tomatenbronzefleckenvirus (TSWV) stammt.

22. DNA-Expressionsvektor nach Anspruch 21, wobei diese DNA aus einem Nucleocapsidproteingen stammt.

23. Wirtszelle, die mit einer DNA-Sequenz gemäß irgendeinem der Ansprüche 1 bis 3, oder einem rekombinanten DNA-Molekül nach irgendeinem der Ansprüche 4 bis 14 oder einem DNA-Expressionsvektor gemäß irgendeinem der Ansprüche 15 bis 22 stabil transformiert ist.

24. Wirtszelle nach Anspruch 23, wobei die Wirtszelle eine Pflanzenzelle ist.

25. Pflanze oder Nachkommen davon, die mit einer DNA-Sequenz gemäß irgendeinem der Ansprüche 1 bis 3 oder einem rekombinanten DNA-Molekül nach irgendeinem der Ansprüche 4 bis 14 oder einem DNA-Expressionsvektor gemäß irgendeinem der Ansprüche 15 bis 22 stabil transformiert sind.

26. Pflanze nach Anspruch 25, wobei diese Pflanze ausgewählt ist aus der Gruppe, die aus Mais, Weizen, Hirse, Roggen, Hafer, Rasengras, Reis, Gerste, Sojabohnen, Baumwolle, Tabak, Zuckerrüben und Ölsaatraps besteht.

27. Verwendung der DNA-Sequenz gemäß irgendeinem der Ansprüche 1 bis 3, um eine Nucleotidsequenz von Interesse zu exprimieren.

28. Verfahren zum Herstellen einer DNA-Sequenz gemäß Anspruch 1, wobei die DNA durch eine Polymerase-Kettenreaktion hergestellt wird, worin mindestens ein Oligonucleotid, das verwendet wird, eine Sequenz aus Nucleotiden umfasst, welche ein konsekutive Abfolge von 15 oder mehr Basenpaaren der SEQ ID Nr. 1 darstellen.

## Revendications

1. Séquence d'ADN comprenant:
(a) une séquence de nucléotides choisie dans le groupe constitué par SEQ ID NO: 1, SEQ ID NO: 2, SEQ ID NO: 3, SEQ ID NO: 4, SEQ ID NO: 19 et SEQ ID NO: 20, ou
(b) le complément d'une séquence d'ADN qui s'hybride dans des conditions stringentes à l'une quelconque de SEQ ID NO: 1, SEQ ID NO: 2, SEQ ID NO: 3 ou SEQ ID NO: 4, ledit complément fonctionnant comme promoteur de la transcription d'une séquence de nucléotides associée,
(c) une séquence d'ADN comprenant un segment consécutif d'au moins 50 nt de SEQ ID NO: 1, ladite séquence d'ADN fonctionnant comme promoteur de la transcription d'une séquence de nucléotides associée.

2. Séquence d'ADN selon la revendication 1, ladite séquence d'ADN comprenant une séquence de nucléotides choisie dans le groupe constitué par SEQ ID NO: 2, SEQ ID NO: 3, SEQ ID NO: 4 et SEQ ID NO: 20, et ladite séquence d'ADN comprenant en outre à son extrémité 3' la séquence de nucléotides représentée par SEQ ID NO: 5.

3. Séquence d'ADN selon la revendication 1 ou la revendication 2, comprenant en outre à son extrémité 5' la séquence de nucléotides représentée par SEQ ID NO: 6.

4. Molécule d'ADN recombinée comprenant une séquence d'ADN selon l'une quelconque des revendications 1 à 3 fonctionnellement liée à une séquence de nucléotides d'intérêt.

5. Molécule d'ADN recombinée selon la revendication 4, dans laquelle la séquence de nucléotides d'intérêt comprend une séquence codante.

6. Molécule d'ADN recombinée selon la revendication 5, dans laquelle la séquence codante code pour un caractère phénotypique avantageux.

7. Molécule d'ADN recombinée selon la revendication 6, dans laquelle la séquence codante code pour une protéine qui confère un avantage sélectif positif aux cellules qui ont été transformées avec ladite séquence codante.

8. Molécule d'ADN recombinée selon la revendication 6 ou 7, dans laquelle la séquence codante code pour une protéine qui confère aux cellules qui ont été transformées avec ladite séquence codante un avantage métabolique consistant en la capacité de métaboliser un composé, ledit composé étant (i) le mannose ou le xylose ou un de leurs dérivés ou de leurs précurseurs, ou (ii) un substrat de la protéine, ou (iii) un composé pouvant être métabolisé en un tel substrat par des cellules transformées avec ladite séquence codante.

9. Molécule d'ADN recombinée selon la revendication 8, dans laquelle ladite séquence codante code pour une enzyme choisie dans le groupe constitué par les xylo-isomérases, les phosphomanno-isomérases, les mannose-6-phosphate-isomérases, les mannose-1-phosphate-isomérases, les phosphomanno-mutases, les mannose-épimérases, les mannose- ou xylose-phosphatases, les mannose-6-phosphatases, les mannose-1-phosphatases et les mannose- ou xylose-perméases.

10. Molécule recombinée selon la revendication 9, dans laquelle la séquence codante code pour une phosphomanno-isomérase.

11. ADN recombiné selon la revendication 5, dans lequel la région codante est non traduisible.

12. ADN recombiné selon la revendication 11, dans lequel la région codante non traduisible provient d'un gène viral.

13. ADN recombiné selon la revendication 12, où le gène viral provient du TSWV, en particulier du gène NP de TSWV.

14. Molécule d'ADN recombinée selon la revendication 5, dans laquelle la séquence codante se trouve dans l'orientation antisens.

15. Vecteur d'expression d'ADN comprenant une séquence d'ADN selon l'une quelconque des revendications 1 à 3 ou une molécule d'ADN recombinée selon l'une quelconque des revendications 4 à 14.

16. Vecteur d'expression d'ADN selon la revendication 15, comprenant une première séquence d'ADN selon l'une quelconque des revendications 1 à 3 fonctionnellement liée à une séquence de nucléotides d'intérêt, et une deuxième séquence d'ADN selon l'une quelconque des revendications 1 à 3 liée à une séquence de nucléotides d'intérêt.

17. Vecteur d'expression d'ADN selon la revendication 16, capable d'altérer l'expression d'un génome viral.

18. Vecteur d'expression d'ADN selon la revendication 17, comprenant une première séquence d'ADN capable d'exprimer un fragment d'ARN sens dudit génome viral ou une portion de celui-ci dans une cellule, et une deuxième séquence d'ADN capable d'exprimer un fragment d'ARN antisens dudit génome viral ou une portion de celui-ci dans une cellule, ledit fragment d'ARN sens et ledit fragment d'ARN antisens étant capables de former un ARN double brin.

19. Vecteur d'expression d'ADN selon la revendication 18, où ledit virus est choisi dans le groupe constitué par les tospovirus, les potyvirus, les potexvirus, les tobamovirtus, les lutéovirus, les cucumovirus, les bromovirus, les clostérovirus, les tombusvirus et les furovirus.

20. Vecteur d'expression d'ADN selon la revendication 19, dans lequel lesdites séquences d'ADN comprennent une séquence de nucléotides provenant d'un gène de protéine d'enveloppe virale, d'un gène de protéine de nucléocapside virale, d'un gène de réplicase virale, ou d'un gène de protéine de mouvement virale ou de portions de ceux-ci.

21. Vecteur d'expression d'ADN selon la revendication 20, dans lequel ladite séquence d'ADN provient du virus de la maladie bronzée de la tomate (TSWV).

22. Vecteur d'expression d'ADN selon la revendication 21, dans lequel ledit ADN provient d'un gène de protéine de nucléocapside.

23. Cellule hôte transformée de manière stable avec une séquence d'ADN selon l'une quelconque des revendications 1 à 3 ou une molécule d'ADN recombinée selon l'une quelconque des revendications 4 à 14 ou un vecteur d'expression d'ADN selon l'une quelconque des revendications 15 à 22.

24. Cellule hôte selon la revendication 23, ladite cellule hôte étant une cellule végétale.

25. Plante et sa descendance transformées de manière stable avec une séquence d'ADN selon l'une quelconque des revendications 1 à 3 ou une molécule d'ADN recombinée selon l'une quelconque des revendications 4 à 14 ou un vecteur d'expression d'ADN selon l'une quelconque des revendications 15 à 22.

26. Plante selon la revendication 25, ladite plante étant choisie dans le groupe constitué par le maïs, le blé, le sorgho, le seigle, l'avoine, le gazon, le riz, l'orge, le soja, le coton, le tabac, la betterave à sucre et le colza.

27. Utilisation de la séquence d'ADN selon l'une quelconque des revendications 1 à 3 pour l'expression d'une séquence de nucléotides d'intérêt.

28. Procédé de production d'une séquence d'ADN selon la revendication 1, dans lequel l'ADN est produit par une réaction d'amplification en chaîne par polymérase dans laquelle au moins un oligonucléotide utilisé comprend une séquence de nucléotides qui représente un segment consécutif d'au moins 15 paires de bases de SEQ ID NO: 1.


### SEQUENCE LISTING

<110> Syngenta Participations AG
<120> Cestrum yellow leaf curling virus promoters
<130> S-31359A
<140>
   <141>
<150> GB 0007427.8
   <151> 2000-03-27
<150> GB 0010486.9
   <151> 2000-04-28
<150> EP 01101802.5
   <151> 2001-01-26
<150> US
   <151> 2001-02-28
<160> 49
<170> PatentIn Ver. 2.2
<210> 1
   <211> 670
   <212> DNA,
   <213> Cestrum yellow leaf curling virus
<400> 1
   attactggca gacaaagtgg cagacatact gtcccacaaa tgaagatgga atctgtaaaa 60
   gaaaacgcgt gaaataatgc gtctgacaaa ggttaggtcg gctgccttta atcaatacca 120
   aagtggtccc taccacgatg gaaaaactgt gcagtcggtt tggctttttc tgacgaacaa 180
   ataagattcg tggccgacag gtgggggtcc accatgtgaa ggcatcttca gactccaata 240
   atggagcaat gacgtaaggg cttacgaaat aagtaagggt agtttgggaa atgtccactc 300
   acccgtcagt ctataaatac ttagcccctc cctcattgtt aagggagcaa aatctcagag 360
   agatagtcct agagagagaa agagagcaag tagcctagaa gtagtcaagg cggcgaagta 420
   ttcaggcagg gtggccagga agaagaaaag ccaagacgac gaaaacaggt aagagctaag 480
   ctttctcatc tcaaagatga ttcttgatga tttttgtctc cacggtccgt ataggatcca 540
   ctgaattgat aaatatcata tggtttgtat aaaacccgat atttaaatct gtatcattct 600
   gtttgaataa aacttgatac tttgttggag tcgtttgtaa aaacataaac aattataatc 660
   tgttaaaaac                                                        670
<210> 2
   <211> 346
   <212> DNA
   <213> Cestrum yellow leaf curling virus
<400> 2
   ctggcagaca aagtggcaga catactgtcc cacaaatgaa gatggaatct gtaaaagaaa 60
   acgcgtgaaa taatgcgtct gacaaaggtt aggtcggctg cctttaatca ataccaaagt 120
   ggtccctacc acgatggaaa aactgtgcag tcggtttggc tttttctgac gaacaaataa 180
   gattcgtggc cgacaggtgg gggtccacca tgtgaaggca tcttcagact ccaataatgg 240
   agcaatgacg taagggctta cgaaataagt aagggtagtt tgggaaatgt ccactcaccc 300
   gtcagtctat aaatacttag cccctccctc attgttaagg gagcaa                 346
<210> 3
   <211> 400
   <212> DNA,
   <213> Cestrum yellow leaf curling virus
<400> 3
   ctggcagaca aagtggcaga catactgtcc cacaaatgaa gatggaatct gtaaaagaaa 60
   acgcgtgaaa taatgcgtct gacaaaggtt aggtcggctg cctttaatca ataccaaagt 120
   ggtccctacc acgatggaaa aactgtgcag tcggtttggc tttttctgac gaacaaataa 180
   gattcgtggc cgacaggtgg gggtccacca tgtgaaggca tcttcagact ccaataatgg 240
   agcaatgacg taagggctta cgaaataagt aagggtagtt tgggaaatgt ccactcaccc 300
   gtcagtctat aaatacttag cccctccctc attgttaagg gagcaaaatc tcagagagat 360
   agtcctagag agagaaagag agcaagtagc ctagaagtag                       400
<210> 4
   <211> 634
   <212> DNA
   <213> Cestrum yellow leaf curling virus
<400> 4
   ctggcagaca aagtggcaga catactgtcc cacaaatgaa gatggaatct gtaaaagaaa 60
   acgcgtgaaa taatgcgtct gacaaaggtt aggtcggctg cctttaatca ataccaaagt 120
   ggtccctacc acgatggaaa aactgtgcag tcggtttggc tttttctgac gaacaaataa 180
   gattcgtggc cgacaggtgg gggtccacca tgtgaaggca tcttcagact ccaataatgg 240
   agcaatgacg taagggctta cgaaataagt aagggtagtt tgggaaatgt ccactcaccc 300
   gtcagtctat aaatacttag cccctccctc attgttaagg gagcaaaatc tcagagagat 360
   agtcctagag agagaaagag agcaagtagc ctagaagtag tcaaggcggc gaagtattca 420
   ggcagggtgg ccaggaagaa gaaaagccaa gacgacgaaa acaggtaaga gctaagcttt 480
   ctcatctcaa agatgattct tgatgatttt tgtctccacg gtccgtatag gatccactga 540
   attgataaat atcatatggt ttgtataaaa cccgatattt aaatctgtat cattctgttt 600
   gaataaaact tgatactttg ttggagtcgt ttgt                             634
<210> 5
   <211> 32
   <212> DNA
   <213> Cestrum yellow leaf curling virus
<400> 5
   aaaaacataa acaattataa tctgttaaaa ac 32
<210> 6
   <211> 104
   <212> DNA
   <213> Cestrum yellow leaf curling virus
<400> 6
   ggcacagctg tcagttgtgc aaatccgagt catctggacc acaaacatca gaagagggtc 60
   tacaagagtc agaagacgaa gacttttcgg tgctagttta atta 104
<210> 7
   <211> 18
   <212> DNA
   <213> Artificial Sequence
<220>
   <223> Description of Artificial Sequence: oligonucleotide
<400> 7
   gaccacaaac atcagaag 18
<210> 8
   <211> 18
   <212> DNA
   <213> Artificial Sequence
<220>
   <223> Description of Artificial Sequence: oligonucleotide
<400> 8
   caaacttatt gggtaatc 18
<210> 9
   <211> 21
   <212> DNA
   <213> Artificial Sequence
<220>
   <223> Description of Artificial Sequence: oligonucleotide
<400> 9
   cagggtacca ctaaaatcac c 21
<210> 10
   <211> 19
   <212> DNA
   <213> Artificial Sequence
<220>
   <223> Description of Artificial Sequence: oligonucleotide
<400> 10
   aggggatccc caattcccc 19
<210> 11
   <211> 21
   <212> DNA
   <213> Artificial Sequence
<220>
   <223> Description of Artificial Sequence: oligonucleotide
<400> 11
   aggggtacca tcgatatgga g 21
<210> 12
   <211> 19
   <212> DNA
   <213> Artificial Sequence
<220>
   <223> Description of Artificial Sequence: oligonucleotide
<400> 12
   ttaggatccg ccctgccac 19
<210> 13
   <211> 21
   <212> DNA
   <213> Artificial Sequence
<220>
   <223> Description of Artificial Sequence: oligonucleotide
<400> 13
   cttctagaca aagtggcaga c 21
<210> 14
   <211> 21
   <212> DNA
   <213> Artificial Sequence
<220>
   <223> Description of Artificial Sequence: oligonucleotide
<400> 14
   ttggtacctt aacaatgagg g 21
<210> 15
   <211> 21
   <212> DNA
   <213> Artificial Sequence
<220>
   <223> Description of Artificial Sequence: oligonucleotide
<400> 15
   ctacttctag gtaccttgct c 21
<210> 16
   <211> 21
   <212> DNA
   <213> Artificial Sequence
<220>
   <223> Description of Artificial Sequence: oligonucleotide
<400> 16
   ttggtacctt aacaatgagg g 21
<210> 17
   <211> 20
   <212> DNA
   <213> Artificial Sequence
<220>
   <223> Description of Artificial Sequence: oligonucleotide
<400> 17
   ggggatcccc agtctctctc 20
<210> 18
   <211> 18
   <212> DNA
   <213> Artificial Sequence
<220>
   <223> Description of Artificial Sequence: oligonucleotide
<400> 18
   gtgaattcga gctcggta 18
<210> 19
   <211> 668
   <212> DNA
   <213> Cestrum yellow leaf curling virus
<400> 19
   attactggca gacaaagtgg cagacatact gtcccacaaa tgaagatgga atctgtaaaa 60
   gaaaacgcgt gaaataatgc gtctgacaaa ggttaggtcg gctgccttta atcaatacca 120
   aagtggtccc taccacgatg gaaaaactgt gcagtcggtt tggctttttc tgacgaacaa 180
   ataagattcg tggccgacag gtgggggtcc accatgtgaa ggcatcttca gactccaata 240
   atggagcaat gacgtaaggg cttacgaaat aagtaagggt agtttgggaa atgtccactc 300
   acccgtcagt ctataaatac ttagcccctc cctcattgtt aagggagcaa aatctcagag 360
   agatagtcct agagagagaa agagagcaag tagcctagaa gtagtcaagg cggcgaagta 420
   ttcaggcagg tggccaggaa gaagaaaagc caagacgacg aaaacaggta agagctaagc 480
   tttctcatct caaagatgat tcttgatgat ttttgtctcc acggtccgta taggatcact 540
   gaattgataa atatcatatg gtttgtataa aacccgatat ttaaatctgt atcattctgt 600
   ttgaataaaa cttgatactt tgttggagtc gtttgtaaaa acataaacaa ttataatctg 660
   ttaaaaac                                                          668
<210> 20
   <211> 632
   <212> DNA
   <213> Cestrum yellow leaf curling virus
<400> 20
   ctggcagaca aagtggcaga catactgtcc cacaaatgaa gatggaatct gtaaaagaaa 60
   acgcgtgaaa taatgcgtct gacaaaggtt aggtcggctg cctttaatca ataccaaagt 120
   ggtccctacc acgatggaaa aactgtgcag tcggtttggc tttttctgac gaacaaataa 180
   gattcgtggc cgacaggtgg gggtccacca tgtgaaggca tcttcagact ccaataatgg 240
   agcaatgacg taagggctta cgaaataagt aagggtagtt tgggaaatgt ccactcaccc 300
   gtcagtctat aaatacttag cccctccctc attgttaagg gagcaaaatc tcagagagat 360
   agtcctagag agagaaagag agcaagtagc ctagaagtag tcaaggcggc gaagtattca 420
   ggcaggtggc caggaagaag aaaagccaag acgacgaaaa caggtaagag ctaagctttc 480
   tcatctcaaa gatgattctt gatgattttt gtctccacgg tccgtatagg atcactgaat 540
   tgataaatat catatggttt gtataaaacc cgatatttaa atctgtatca ttctgtttga 600
   ataaaacttg atactttgtt ggagtcgttt gt 632
<210> 21
   <211> 28
   <212> DNA
   <213> Artificial Sequence
<220>
   <223> Description of Artificial Sequence: oligonucleotide
<400> 21
   cccgctgcag atcgttcaaa catttggc 28
<210> 22
   <211> 27
   <212> DNA
   <213> Artificial Sequence
<220>
   <223> Description of Artificial Sequence: oligonucleotide
<400> 22
   cccgaagctt tctagagatc tagtaac 27
<210> 23
   <211> 41
   <212> DNA
   <213> Artificial Sequence
<220>
   <223> Description of Artificial Sequence: oligonucleotide
<400> 23
   gggcggatcc gcatgcatgt cttaaggtaa gctcactaag g 41
<210> 24
   <211> 34
   <212> DNA
   <213> Artificial Sequence
<220>
   <223> Description of Artificial Sequence: oligonucleotide
<400> 24
   ccgcgctgca ggctgctttc aagcaagttc tgcg 34
<210> 25
   <211> 35
   <212> DNA
   <213> Artificial Sequence
<220>
   <223> Description of Artificial Sequence: oligonucleotide
<400> 25
   ttgagagctc gtttaattac tggcagacaa agtgg 35
<210> 26
   <211> 34
   <212> DNA
   <213> Artificial Sequence
<220>
   <223> Description of Artificial Sequence: oligonucleotide
<400> 26
   ttgactgcag gtttatgttt ttacaaacga ctcc 34
<210> 27
   <211> 137
   <212> DNA
   <213> Artificial Sequence
<220>
   <223> Description of Artificial Sequence: AGLINK multicloning site
<400> 27
   gcggccgctc cggattcgaa ttaattaacg tacgaagctt gcatgcctgc agtgatcacc 60
   atggtcgact ctagaggatc cccgggtacc gagctcgaat tcggcgcgcc caattgattt 120
   aaatggccgc tgcggcc 137
<210> 28
   <211> 216
   <212> DNA
   <213> Artificial Sequence
<220>
   <223> Description of Artificial Sequence: BIGLINK multicloning site
<400> 28
   ggccgcagcg gccatttaaa tcaattgggc gcgccgaatt cgagctcggt acccggggat 60
   cctctagagt cgaccatggt gatcactgca ggcatgcaag cttcgtacgt taattaattc 120
   gaatccggag cggccgcacg cgtgggcccg tttaaacctc gagagatctg ctagccctgc 180
   aggaaattta ccggtgcccg ggcggccagc atggcc 216
<210> 29
   <211> 28
   <212> DNA
   <213> Artificial Sequence
<220>
   <223> Description of Artificial Sequence: oligonucleotide
<400> 29
   cgcggatcct ggcagacaaa gtggcaga 28
<210> 30
   <211> 26
   <212> DNA
   <213> Artificial Sequence
<220>
   <223> Description of Artificial Sequence: oligonucleotide
<400> 30
   cgcggatcct acttctaggc tacttg 26
<210> 31
   <211> 7195
   <212> DNA
   <213> Artificial Sequence
<220>
   <223> Description of Artificial Sequence: vector pNOV2804
<400> 31
   gtttacccgc caatatatcc tgtcaaacac tgatagttta aactgaaggc gggaaacgac 60
   aatctgatca tgagcggaga attaagggag tcacgttatg acccccgccg atgacgcggg 120
   acaagccgtt ttacgtttgg aactgacaga accgcaacgc tgcaggaatt ggccgcagcg 180
   gccatttaaa tcaattgggc gcgtacgtag cactagtgcg cgatcgctta attaagcggc 240
   gcgcctaaag cttgcatgcc tgcaggtcga ctctagagga tccccgatca tgcaaaaact 300
   cattaactca gtgcaaaact atgcctgggg cagcaaaacg gcgttgactg aactttatgg 360
   tatggaaaat ccgtccagcc agccgatggc cgagctgtgg atgggcgcac atccgaaaag 420
   cagttcacga gtgcagaatg ccgccggaga tatcgtttca ctgcgtgatg tgattgagag 480
   tgataaatcg actctgctcg gagaggccgt tgccaaacgc tttggcgaac tgcctttcct 540
   gttcaaagta ttatgcgcag cacagccact ctccattcag gttcatccaa acaaacacaa 600
   ttctgaaatc ggttttgcca aagaaaatgc cgcaggtatc ccgatggatg ccgccgagcg 660
   taactataaa gatcctaacc acaagccgga gctggttttt gcgctgacgc ctttccttgc 720
   gatgaacgcg tttcgtgaat tttccgagat tgtctcccta ctccagccgg tcgcaggtgc 780
   acatccggcg attgctcact ttttacaaca gcctgatgcc gaacgtttaa gcgaactgtt 840
   cgccagcctg ttgaatatgc agggtgaaga aaaatcccgc gcgctggcga ttttaaaatc 900
   ggccctcgat agccagcagg gtgaaccgtg gcaaacgatt cgtttaattt ctgaatttta 960
   cccggaagac agcggtctgt tctccccgct attgctgaat gtggtgaaat tgaaccctgg 1020
   cgaagcgatg ttcctgttcg ctgaaacacc gcacgcttac ctgcaaggcg tggcgctgga 1080
   agtgatggca aactccgata acgtgctgcg tgcgggtctg acgcctaaat acattgatat 1140
   tccggaactg gttgccaatg tgaaattcga agccaaaccg gctaaccagt tgttgaccca 1200
   gccggtgaaa caaggtgcag aactggactt cccgattcca gtggatgatt ttgccttctc 1260
   gctgcatgac cttagtgata aagaaaccac cattagccag cagagtgccg ccattttgtt 1320
   ctgcgtcgaa ggcgatgcaa cgttgtggaa aggttctcag cagttacagc ttaaaccggg 1380
   tgaatcagcg tttattgccg ccaacgaatc accggtgact gtcaaaggcc acggccgttt 1440
   agcgcgtgtt tacaacaagc tgtaagagct tactgaaaaa attaacatct cttgctaagc 1500
   tgggagctct agatccccga atttccccga tcgttcaaac atttggcaat aaagtttctt 1560
   aagattgaat cctgttgccg gtcttgcgat gattatcata taatttctgt tgaattacgt 1620
   taagcatgta ataattaaca tgtaatgcat gacgttattt atgagatggg tttttatgat 1680
   tagagtcccg caattataca tttaatacgc gatagaaaac aaaatatagc gcgcaaacta 1740
   ggataaatta tcgcgcgcgg tgtcatctat gttactagat cgggaattgg gtaccatgcc 1800
   cgggcggcca gcatggccgt atccgcaatg tgttattaag ttgtctaagc gtcaatttgt 1860
   ttacaccaca atatatcctg ccaccagcca gccaacagct ccccgaccgg cagctcggca 1920
   caaaatcacc actcgataca ggcagcccat cagaattaat tctcatgttt gacagcttat 1980
   catcgactgc acggtgcacc aatgcttctg gcgtcaggca gccatcggaa gctgtggtat 2040
   ggctgtgcag gtcgtaaatc actgcataat tcgtgtcgct caaggcgcac tcccgttctg 2100
   gataatgttt tttgcgccga catcataacg gttctggcaa atattctgaa atgagctgtt 2160
   gacaattaat catccggctc gtataatgtg tggaattgtg agcggataac aatttcacac 2220
   aggaaacaga ccatgaggga agcgttgatc gccgaagtat cgactcaact atcagaggta 2280
   gttggcgtca tcgagcgcca tctcgaaccg acgttgctgg ccgtacattt gtacggctcc 2340
   gcagtggatg gcggcctgaa gccacacagt gatattgatt tgctggttac ggtgaccgta 2400
   aggcttgatg aaacaacgcg gcgagctttg atcaacgacc ttttggaaac ttcggcttcc 2460
   cctggagaga gcgagattct ccgcgctgta gaagtcacca ttgttgtgca cgacgacatc 2520
   attccgtggc gttatccagc taagcgcgaa ctgcaatttg gagaatggca gcgcaatgac 2580
   attcttgcag gtatcttcga gccagccacg atcgacattg atctggctat cttgctgaca 2640
   aaagcaagag aacatagcgt tgccttggta ggtccagcgg cggaggaact ctttgatccg 2700
   gttcctgaac aggatctatt tgaggcgcta aatgaaacct taacgctatg gaactcgccg 2760
   cccgactggg ctggcgatga gcgaaatgta gtgcttacgt tgtcccgcat ttggtacagc 2820
   gcagtaaccg gcaaaatcgc gccgaaggat gtcgctgccg actgggcaat ggagcgcctg 2880
   ccggcccagt atcagcccgt catacttgaa gctaggcagg cttatcttgg acaagaagat 2940
   cgcttggcct cgcgcgcaga tcagttggaa gaatttgttc actacgtgaa aggcgagatc 3000
   accaaagtag tcggcaaata aagctctagt ggatctccgt acccccgggg gatctggctc 3060
   gcggcggacg cacgacgccg gggcgagacc ataggcgatc tcctaaatca atagtagctg 3120
   taacctcgaa gcgtttcact tgtaacaacg attgagaatt tttgtcataa aattgaaata 3180
   cttggttcgc atttttgtca tccgcggtca gccgcaattc tgacgaactg cccatttagc 3240
   tggagatgat tgtacatcct tcacgtgaaa atttctcaag cgctgtgaac aagggttcag 3300
   attttagatt gaaaggtgag ccgttgaaac acgttcttct tgtcgatgac gacgtcgcta 3360
   tgcggcatct tattattgaa taccttacga tccacgcctt caaagtgacc gcggtagccg 3420
   acagcaccca gttcacaaga gtactctctt ccgcgacggt cgatgtcgtg gttgttgatc 3480
   taaatttagg tcgtgaagat gggctcgaga tcgttcgtaa tctggcggca aagtctgata 3540
   ttccaatcat aattatcagt ggcgaccgcc ttgaggagac ggataaagtt gttgcactcg 3600
   agctaggagc aagtgatttt atcgctaagc cgttcagtat cagagagttt ctagcacgca 3660
   ttcgggttgc cttgcgcgtg cgccccaacg ttgtccgctc caaagaccga cggtcttttt 3720
   gttttactga ctggacactt aatctcaggc aacgtcgctt gatgtccgaa gctggcggtg 3780
   aggtgaaact tacggcaggt gagttcaatc ttctcctcgc gtttttagag aaaccccgcg 3840
   acgttctatc gcgcgagcaa cttctcattg ccagtcgagt acgcgacgag gaggtttatg 3900
   acaggagtat agatgttctc attttgaggc tgcgccgcaa acttgaggca gatccgtcaa 3960
   gccctcaact gataaaaaca gcaagaggtg ccggttattt ctttgacgcg gacgtgcagg 4020
   tttcgcacgg ggggacgatg gcagcctgag ccaattccca gatccccgag gaatcggcgt 4080
   gagcggtcgc aaaccatccg gcccggtaca aatcggcgcg gcgctgggtg atgacctggt 4140
   ggagaagttg aaggccgcgc aggccgccca gcggcaacgc atcgaggcag aagcacgccc 4200
   cggtgaatcg tggcaagcgg ccgctgatcg aatccgcaaa gaatcccggc aaccgccggc 4260
   agccggtgcg ccgtcgatta ggaagccgcc caagggcgac gagcaaccag attttttcgt 4320
   tccgatgctc tatgacgtgg gcacccgcga tagtcgcagc atcatggacg tggccgtttt 4380
   ccgtctgtcg aagcgtgacc gacgagctgg cgaggtgatc cgctacgagc ttccagacgg 4440
   gcacgtagag gtttccgcag ggccggccgg catggccagt gtgtgggatt acgacctggt 4500
   actgatggcg gtttcccatc taaccgaatc catgaaccga taccgggaag ggaagggaga 4560
   caagcccggc cgcgtgttcc gtccacacgt tgcggacgta ctcaagttct gccggcgagc 4620
   cgatggcgga aagcagaaag acgacctggt agaaacctgc attcggttaa acaccacgca 4680
   cgttgccatg cagcgtacga agaaggccaa gaacggccgc ctggtgacgg tatccgaggg 4740
   tgaagccttg attagccgct acaagatcgt aaagagcgaa accgggcggc cggagtacat 4800
   cgagatcgag ctagctgatt ggatgtaccg cgagatcaca gaaggcaaga acccggacgt 4860
   gctgacggtt caccccgatt actttttgat cgatcccggc atcggccgtt ttctctaccg 4920
   cctggcacgc cgcgccgcag gcaaggcaga agccagatgg ttgttcaaga cgatctacga 4980
   acgcagtggc agcgccggag agttcaagaa gttctgtttc accgtgcgca agctgatcgg 5040
   gtcaaatgac ctgccggagt acgatttgaa ggaggaggcg gggcaggctg gcccgatcct 5100
   agtcatgcgc taccgcaacc tgatcgaggg cgaagcatcc gccggttcct aatgtacgga 5160
   gcagatgcta gggcaaattg ccctagcagg ggaaaaaggt cgaaaaggtc tctttcctgt 5220
   ggatagcacg tacattggga acccaaagcc gtacattggg aaccggaacc cgtacattgg 5280
   gaacccaaag ccgtacattg ggaaccggtc acacatgtaa gtgactgata taaaagagaa 5340
   aaaaggcgat ttttccgcct aaaactcttt aaaacttatt aaaactctta aaacccgcct 5400
   ggcctgtgca taactgtctg gccagcgcac agccgaagag ctgcaaaaag cgcctaccct 5460
   tcggtcgctg cgctccctac gccccgccgc ttcgcgtcgg cctatcgcgg ccgctggccg 5520
   ctcaaaaatg gctggcctac ggccaggcaa tctaccaggg cgcggacaag ccgcgccgtc 5580
   gccactcgac cgccggcgct gaggtctgcc tcgtgaagaa ggtgttgctg actcatacca 5640
   ggcctgaatc gccccatcat ccagccagaa agtgagggag ccacggttga tgagagcttt 5700
   gttgtaggtg gaccagttgg tgattttgaa cttttgcttt gccacggaac ggtctgcgtt 5760
   gtcgggaaga tgcgtgatct gatccttcaa ctcagcaaaa gttcgattta ttcaacaaag 5820
   ccgccgtccc gtcaagtcag cgtaatgctc tgccagtgtt acaaccaatt aaccaattct 5880
   gattagaaaa actcatcgag catcaaatga aactgcaatt tattcatatc aggattatca 5940
   ataccatatt tttgaaaaag ccgtttctgt aatgaaggag aaaactcacc gaggcagttc 6000
   cataggatgg caagatcctg gtatcggtct gcgattccga ctcgtccaac atcaatacaa 6060
   cctattaatt tcccctcgtc aaaaataagg ttatcaagtg agaaatcacc atgagtgacg 6120
   actgaatccg gtgagaatgg caaaagctct gcattaatga atcggccaac gcgcggggag 6180
   aggcggtttg cgtattgggc gctcttccgc ttcctcgctc actgactcgc tgcgctcggt 6240
   cgttcggctg cggcgagcgg tatcagctca ctcaaaggcg gtaatacggt tatccacaga 6300
   atcaggggat aacgcaggaa agaacatgtg agcaaaaggc cagcaaaagg ccaggaaccg 6360
   taaaaaggcc gcgttgctgg cgtttttcca taggctccgc ccccctgacg agcatcacaa 6420
   aaatcgacgc tcaagtcaga ggtggcgaaa cccgacagga ctataaagat accaggcgtt 6480
   tccccctgga agctccctcg tgcgctctcc tgttccgacc ctgccgctta ccggatacct 6540
   gtccgccttt ctcccttcgg gaagcgtggc gctttctcat agctcacgct gtaggtatct 6600
   cagttcggtg taggtcgttc gctccaagct gggctgtgtg cacgaacccc ccgttcagcc 6660
   cgaccgctgc gccttatccg gtaactatcg tcttgagtcc aacccggtaa gacacgactt 6720
   atcgccactg gcagcagcca ctggtaacag gattagcaga gcgaggtatg taggcggtgc 6780
   tacagagttc ttgaagtggt ggcctaacta cggctacact agaagaacag tatttggtat 6840
   ctgcgctctg ctgaagccag ttaccttcgg aaaaagagtt ggtagctctt gatccggcaa 6900
   acaaaccacc gctggtagcg gtggtttttt tgtttgcaag cagcagatta cgcgcagaaa 6960
   aaaaggatct caagaagatc ctttgatctt ttctacgggg tctgacgctc agtggaacga 7020
   aaactcacgt taagggattt tggtcatgag attatcaaaa aggatcttca cctagatcct 7080
   tttgatccgg aattaattcc tgtggttggc atgcacatac aaatggacga acggataaac 7140
   cttttcacgc ccttttaaat atccgattat tctaataaac gctcttttct cttag 7195
<210> 32
   <211> 4224
   <212> DNA
   <213> Artificial Sequence
<220>
   <223> Description of Artificial Sequence: vector pNOV3604
<400> 32
   agcttgcatg cctgcaggtc gactctagag gatccccgat catgcaaaaa ctcattaact 60
   cagtgcaaaa ctatgcctgg ggcagcaaaa cggcgttgac tgaactttat ggtatggaaa 120
   atccgtccag ccagccgatg gccgagctgt ggatgggcgc acatccgaaa agcagttcac 180
   gagtgcagaa tgccgccgga gatatcgttt cactgcgtga tgtgattgag agtgataaat 240
   cgactctgct cggagaggcc gttgccaaac gctttggcga actgcctttc ctgttcaaag 300
   tattatgcgc agcacagcca ctctccattc aggttcatcc aaacaaacac aattctgaaa 360
   tcggttttgc caaagaaaat gccgcaggta tcccgatgga tgccgccgag cgtaactata 420
   aagatcctaa ccacaagccg gagctggttt ttgcgctgac gcctttcctt gcgatgaacg 480
   cgtttcgtga attttccgag attgtctccc tactccagcc ggtcgcaggt gcacatccgg 540
   cgattgctca ctttttacaa cagcctgatg ccgaacgttt aagcgaactg ttcgccagcc 600
   tgttgaatat gcagggtgaa gaaaaatccc gcgcgctggc gattttaaaa tcggccctcg 660
   atagccagca gggtgaaccg tggcaaacga ttcgtttaat ttctgaattt tacccggaag 720
   acagcggtct gttctccccg ctattgctga atgtggtgaa attgaaccct ggcgaagcga 780
   tgttcctgtt cgctgaaaca ccgcacgctt acctgcaagg cgtggcgctg gaagtgatgg 840
   caaactccga taacgtgctg cgtgcgggtc tgacgcctaa atacattgat attccggaac 900
   tggttgccaa tgtgaaattc gaagccaaac cggctaacca gttgttgacc cagccggtga 960
   aacaaggtgc agaactggac ttcccgattc cagtggatga ttttgccttc tcgctgcatg 1020
   accttagtga taaagaaacc accattagcc agcagagtgc cgccattttg ttctgcgtcg 1080
   aaggcgatgc aacgttgtgg aaaggttctc agcagttaca gcttaaaccg ggtgaatcag 1140
   cgtttattgc cgccaacgaa tcaccggtga ctgtcaaagg ccacggccgt ttagcgcgtg 1200
   tttacaacaa gctgtaagag cttactgaaa aaattaacat ctcttgctaa gctgggagct 1260
   ctagatcccc gaatttcccc gatcgttcaa acatttggca ataaagtttc ttaagattga 1320
   atcctgttgc cggtcttgcg atgattatca tataatttct gttgaattac gttaagcatg 1380
   taataattaa catgtaatgc atgacgttat ttatgagatg ggtttttatg attagagtcc 1440
   cgcaattata catttaatac gcgatagaaa acaaaatata gcgcgcaaac taggataaat 1500
   tatcgcgcgc ggtgtcatct atgttactag atcgggaatt gggtaccgaa ttcactggcc 1560
   gtcgttttac aacgtcgtga ctgggaaaac cctggcgtta cccaacttaa tcgccttgca 1620
   gcacatcccc ctttcgccag ctggcgtaat agcgaagagg cccgcaccga tcgcccttcc 1680
   caacagttgc gcagcctgaa tggcgaatgg cgcctgatgc ggtattttct ccttacgcat 1740
   ctgtgcggta tttcacaccg catatggtgc actctcagta caatctgctc tgatgccgca 1800
   tagttaagcc agccccgaca cccgccaaca cccgctgacg cgccctgacg ggcttgtctg 1860
   ctcccggcat ccgcttacag acaagctgtg accgtctccg ggagctgcat gtgtcagagg 1920
   ttttcaccgt catcaccgaa acgcgcgaga cgaaagggcc tcgtgatacg cctattttta 1980
   taggttaatg tcatgataat aatggtttct tagacgtcag gtggcacttt tcggggaaat 2040
   gtgcgcggaa cccctatttg tttatttttc taaatacatt caaatatgta tccgctcatg 2100
   agacaataac cctgataaat gcttcaatgg cgcgccgcgg ccgcttaaga atattgaaaa 2160
   aggaagagta tgagtattca acatttccgt gtcgccctta ttcccttttt tgcggcattt 2220
   tgccttcctg tttttgctca cccagaaacg ctggtgaaag taaaagatgc tgaagatcag 2280
   ttgggtgcac gagtgggtta catcgaactg gatctcaaca gcggtaagat ccttgagagt 2340
   tttcgccccg aagaacgttt tccaatgatg agcactttta aagttctgct atgtggcgcg 2400
   gtattatccc gtattgacgc cgggcaagag caactcggtc gccgcataca ctattctcag 2460
   aatgacttgg ttgagtactc accagtcaca gaaaagcatc ttacggatgg catgacagta 2520
   agagaattat gcagtgctgc cataaccatg agtgataaca ctgcggccaa cttacttctg 2580
   acaacgatcg gaggaccgaa ggagctaacc gcttttttgc acaacatggg ggatcatgta 2640
   actcgccttg atcgttggga accggagctg aatgaagcca taccaaacga cgagcgtgac 2700
   accacgatgc ctgtagcaat ggcaacaacg ttgcgcaaac tattaactgg cgaactactt 2760
   actctagctt cccggcaaca attaatagac tggatggagg cggataaagt tgcaggacca 2820
   cttctgcgct cggcccttcc ggctggctgg tttattgctg ataaatctgg agccggtgag 2880
   cgtgggtctc gcggtatcat tgcagcactg gggccagatg gtaagccctc ccgtatcgta 2940
   gttatctaca cgacggggag tcaggcaact atggatgaac gaaatagaca gatcgctgag 3000
   ataggtgcct cactgattaa gcattggtaa ctgtcagacc aagtttactc atatatactt 3060
   tagattgatt taaaacttca tttttaattt aaaaggatct aggtgaagat cctttttgat 3120
   aatctcatga ccaaaatccc ttaacgtgag ttttcgttcc actgagcgtc agaccccgta 3180
   gaaaagatca aaggatcttc ttgagatcct ttttttctgc gcgtaatctg ctgcttgcaa 3240
   acaaaaaaac caccgctacc agcggtggtt tgtttgccgg atcaagagct accaactctt 3300
   tttccgaagg taactggctt cagcagagcg cagataccaa atactgtcct tctagtgtag 3360
   ccgtagttag gccaccactt caagaactct gtagcaccgc ctacatacct cgctctgcta 3420
   atcctgttac cagtggctgc tgccagtggc gataagtcgt gtcttaccgg gttggactca 3480
   agacgatagt taccggataa ggcgcagcgg tcgggctgaa cggggggttc gtgcacacag 3540
   cccagcttgg agcgaacgac ctacaccgaa ctgagatacc tacagcgtga gctatgagaa 3600
   agcgccacgc ttcccgaagg gagaaaggcg gacaggtatc cggtaagcgg cagggtcgga 3660
   acaggagagc gcacgaggga gcttccaggg ggaaacgcct ggtatcttta tagtcctgtc 3720
   gggtttcgcc acctctgact tgagcgtcga tttttgtgat gctcgtcagg ggggcggagc 3780
   ctatggaaaa acgccagcaa cgcggccttt ttacggttcc tggccttttg ctggcctttt 3840
   gctcacatgt tctttcctgc gttatcccct gattctgtgg ataaccgtat taccgccttt 3900
   gagtgagctg ataccgctcg ccgcagccga acgaccgagc gcagcgagtc agtgagcgag 3960
   gaagcggaag agcttaagcg gccgcggcgc gccgcccaat acgcaaaccg cctctccccg 4020
   cgcgttggcc gattcattaa tgcagctggc acgacaggtt tcccgactgg aaagcgggca 4080
   gtgagcgcaa cgcaattaat gtgagttagc tcactcatta ggcaccccag gctttacact 4140
   ttatgcttcc ggctcgtatg ttgtgtggaa ttgtgagcgg ataacaattt cacacaggaa 4200
   acagctatga ccatgattac gcca                                        4224
<210> 33
   <211> 21
   <212> DNA
   <213> Artificial Sequence
<220>
   <223> Description of Artificial Sequence: oligonucleotide
<400> 33
   ccatcgtggt atttggtatt g 21
<210> 34
   <211> 21
   <212> DNA
   <213> Artificial Sequence
<220>
   <223> Description of Artificial Sequence: oligonucleotide
<400> 34
   caataccaaa taccacgatg g 21
<210> 35
   <211> 21
   <212> DNA
   <213> Artificial Sequence
<220>
   <223> Description of Artificial Sequence: oligonucleotide
<400> 35
   cgtggtagcg agcactttgg t 21
<210> 36
   <211> 21
   <212> DNA
   <213> Artificial Sequence
<220>
   <223> Description of Artificial Sequence: oligonucleotide
<400> 36
   aagtgctcgc taccacgatg g 21
<210> 37
   <211> 912
   <212> DNA
   <213> Artificial
<220>
   <223> artificial sequence synthetic GFP gene with intron
<400> 37
   atgagcaagg gcgaggagct gttcaccggc gtggtgccaa tcctggtgga gctggacggc 60
   gacgtgaacg gccacaagtt cagcgtgagc ggcgagggcg agggcgacgc gacctacggc 120
   aagctgaccc tgaagttcat ctgtaccacc ggcaagctcc cggtcccgtg gccgaccctg 180
   gtgaccacct tcacctacgg cgtgcagtgt ttcagccgct acccggacca catgaagcgc 240
   cacgacttct tcaagagcgc catgccggag ggctacgtaa gtttctgctt ctacctttga 300
   tatatatata ataattatca ttaattagta gtaatataat atttcaaata tttttttcaa 360
   aataaaagaa tgtagtatat agcaattgct tttctgtagt ttataagtgt gtatatttta 420
   atttataact tttctaatat atgaccaaaa tttgttgatg tgcaggtgca ggagcgcacc 480
   atcagcttca aggacgacgg caactacaag acccgcgccg aggtgaagtt cgagggcgac 540
   acactagtga accgcatcga gctgaagggc atcgacttca aggaggacgg caacatcctg 600
   ggccacaagc tggagtacaa ctacaacagc cacaacgtgt acatcaccgc ggacaagcag 660
   aagaacggca tcaaggcgaa cttcaagatc cgccacaaca tcgaggacgg cagcgtgcag 720
   ctggccgacc actaccagca gaacaccccg atcggcgacg gtcctgtgct gctgccggac 780
   aaccactacc tgagcaccca gagcgccctg agcaaggacc cgaacgagaa gcgcgaccac 840
   atggtgctgc tggagttcgt gaccgccgcc ggcatcaccc acggcatgga cgagctgtac 900
   aaggttaact ag 912
<210> 38
   <211> 2001
   <212> DNA
   <213> Artificial
<220>
   <223> artificial sequence GUS gene with intron
<400> 38
   atggtccgtc ctgtagaaac cccaacccgt gaaatcaaaa aactcgacgg cctgtgggca 60
   ttcagtctgg atcgcgaaaa ctgtggaatt gatcagcgtt ggtgggaaag cgcgttacaa 120
   gaaagccggg caattgctgt gccaggcagt tttaacgatc agttcgccga tgcagatatt 180
   cgtaattatg cgggcaacgt ctggtatcag cgcgaagtct ttataccgaa aggttgggca 240
   ggccagcgta tcgtgctgcg tttcgatgcg gtcactcatt acggcaaagt gtgggtcaat 300
   aatcaggaag tgatggagca tcagggcggc tatacgccat ttgaagccga tgtcacgccg 360
   tatgttattg ccgggaaaag tgtacgtaag tttctgcttc tacctttgat atatatataa 420
   taattatcat taattagtag taatataata tttcaaatat ttttttcaaa ataaaagaat 480
   gtagtatata gcaattgctt ttctgtagtt tataagtgtg tatattttaa tttataactt 540
   ttctaatata tgaccaaaat ttgttgatgt gcaggtatca ccgtttgtgt gaacaacgaa 600
   ctgaactggc agactatccc gccgggaatg gtgattaccg acgaaaacgg caagaaaaag 660
   cagtcttact tccatgattt ctttaactat gccggaatcc atcgcagcgt aatgctctac 720
   accacgccga acacctgggt ggacgatatc accgtggtga cgcatgtcgc gcaagactgt 780
   aaccacgcgt ctgttgactg gcaggtggtg gccaatggtg atgtcagcgt tgaactgcgt 840
   gatgcggatc aacaggtggt tgcaactgga caaggcacta gcgggacttt gcaagtggtg 900
   aatccgcacc tctggcaacc gggtgaaggt tatctctatg aactgtgcgt cacagccaaa 960
   agccagacag agtgtgatat ctacccgctt cgcgtcggca tccggtcagt ggcagtgaag 1020
   ggcgaacagt tcctgattaa ccacaaaccg ttctacttta ctggctttgg tcgtcatgaa 1080
   gatgcggact tgcgtggcaa aggattcgat aacgtgctga tggtgcacga ccacgcatta 1140
   atggactgga ttggggccaa ctcctaccgt acctcgcatt acccttacgc tgaagagatg 1200
   ctcgactggg cagatgaaca tggcatcgtg gtgattgatg aaactgctgc tgtcggcttt 1260
   aacctctctt taggcattgg tttcgaagcg ggcaacaagc cgaaagaact gtacagcgaa 1320
   gaggcagtca acggggaaac tcagcaagcg cacttacagg cgattaaaga gctgatagcg 1380
   cgtgacaaaa accacccaag cgtggtgatg tggagtattg ccaacgaacc ggatacccgt 1440
   ccgcaaggtg cacgggaata tttcgcgcca ctggcggaag caacgcgtaa actcgacccg 1500
   acgcgtccga tcacctgcgt caatgtaatg ttctgcgacg ctcacaccga taccatcagc 1560
   gatctctttg atgtgctgtg cctgaaccgt tattacggat ggtatgtcca aagcggcgat 1620
   ttggaaacgg cagagaaggt actggaaaaa gaacttctgg cctggcagga gaaactgcat 1680
   cagccgatta tcatcaccga atacggcgtg gatacgttag ccgggctgca ctcaatgtac 1740
   accgacatgt ggagtgaaga gtatcagtgt gcatggctgg atatgtatca ccgcgtcttt 1800
   gatcgcgtca gcgccgtcgt cggtgaacag gtatggaatt tcgccgattt tgcgacctcg 1860
   caaggcatat tgcgcgttgg cggtaacaag aaagggatct tcactcgcga ccgcaaaccg 1920
   aagtcggcgg cttttctgct gcaaaaacgc tggactggca tgaacttcgg tgaaaaaccg 1980
   cagcagggag gcaaacaatg a 2001
<210> 39
   <211> 26
   <212> DNA
   <213> Artificial
<220>
   <223> artificial sequence PCR primer
<400> 39
   cgcggattgc tcccttaaca atgagg 26
<210> 40
   <211> 1618
   <212> DNA
   <213> Artificial
<220>
   <223> artificial sequence CropS-synGFPI-nos expression cassette
<400> 40
   tcctggcaga caaagtggca gacatactgt cccacaaatg aagatggaat ctgtaaaaga 60
   aaacgcgtga aataatgcgt ctgacaaagg ttaggtcggc tgcctttaat caataccaaa 120
   gtggtcccta ccacgatgga aaaactgtgc agtcggtttg gctttttctg acgaacaaat 180
   aagattcgtg gccgacaggt gggggtccac catgtgaagg catcttcaga ctccaataat 240
   ggagcaatga cgtaagggct tacgaaataa gtaagggtag tttgggaaat gtccactcac 300
   ccgtcagtct ataaatactt agcccctccc tcattgttaa gggagcaaaa tctcagagag 360
   atagtcctag agagagaaag agagcaagta gcctagaagt aggatccacc atgctgcaga 420
   tgagcaaggg cgaggagctg ttcaccggcg tggtgccaat cctggtggag ctggacggcg 480
   acgtgaacgg ccacaagttc agcgtgagcg gcgagggcga gggcgacgcg acctacggca 540
   agctgaccct gaagttcatc tgtaccaccg gcaagctccc ggtcccgtgg ccgaccctgg 600
   tgaccacctt cacctacggc gtgcagtgtt tcagccgcta cccggaccac atgaagcgcc 660
   acgacttctt caagagcgcc atgccggagg gctacgtaag tttctgcttc tacctttgat 720
   atatatataa taattatcat taattagtag taatataata tttcaaatat ttttttcaaa 780
   ataaaagaat gtagtatata gcaattgctt ttctgtagtt tataagtgtg tatattttaa 840
   tttataactt ttctaatata tgaccaaaat ttgttgatgt gcaggtgcag gagcgcacca 900
   tcagcttcaa ggacgacggc aactacaaga cccgcgccga ggtgaagttc gagggcgaca 960
   cactagtgaa ccgcatcgag ctgaagggca tcgacttcaa ggaggacggc aacatcctgg 1020
   gccacaagct ggagtacaac tacaacagcc acaacgtgta catcaccgcg gacaagcaga 1080
   agaacggcat caaggcgaac ttcaagatcc gccacaacat cgaggacggc agcgtgcagc 1140
   tggccgacca ctaccagcag aacaccccga tcggcgacgg tcctgtgctg ctgccggaca 1200
   accactacct gagcacccag agcgccctga gcaaggaccc gaacgagaag cgcgaccaca 1260
   tggtgctgct ggagttcgtg accgccgccg gcatcaccca cggcatggac gagctgtaca 1320
   aggttaacta gagctcaaga tcccccgaat ttccccgatc gttcaaacat ttggcaataa 1380
   agtttcttaa gattgaatcc tgttgccggt cttgcgatga ttatcatcta atttctgttg 1440
   aattacgtta agcatgtaat aattaacatg taatgcatga cgttatttat gagatgggtt 1500
   tttatgatta gagtcccgca attatacatt taatacgcga tagaaaacaa aatatagcgc 1560
   gcaaactagg ataaattatc gcgcgcggtg tcatctatgt tactagatcc gggaattg 1618
<210> 41
   <211> 2730
   <212> DNA
   <213> Artificial
<220>
   <223> artificial sequence CmpS-GIG-nos expression cassette
<400> 41
   aggatcctgg cagacaaagt ggcagacata ctgtcccaca aatgaagatg gaatctgtaa      60
   aagaaaacgc gtgaaataat gcgtctgaca aaggttaggt cggctgcctt taatcaatac     120
   caaagtggtc cctaccacga tggaaaaact gtgcagtcgg tttggctttt tctgacgaac     180
   aaataagatt cgtggccgac aggtgggggt ccaccatgtg aaggcatctt cagactccaa     240
   taatggagca atgacgtaag ggcttacgaa ataagtaagg gtagtttggg aaatgtccac     300
   tcacccgtca gtctataaat acttagcccc tccctcattg ttaagggagc aaaatctcag     360
   agagatagtc ctagagagag aaagagagca agtagcctag aagtaggatc ccctcgaggt      420
   cgaccatggt ccgtcctgta gaaaccccaa cccgtgaaat caaaaaactc gacggcctgt     480
   gggcattcag tctggatcgc gaaaactgtg gaattgatca gcgttggtgg gaaagcgcgt     540
   tacaagaaag ccgggcaatt gctgtgccag gcagttttaa cgatcagttc gccgatgcag     600
   atattcgtaa ttatgcgggc aacgtctggt atcagcgcga agtctttata ccgaaaggtt     660
   gggcaggcca gcgtatcgtg ctgcgtttcg atgcggtcac tcattacggc aaagtgtggg     720
   tcaataatca ggaagtgatg gagcatcagg gcggctatac gccatttgaa gccgatgtca     780
   cgccgtatgt tattgccggg aaaagtgtac gtaagtttct gcttctacct ttgatatata     840
   tataataatt atcattaatt agtagtaata taatatttca aatatttttt tcaaaataaa     900
   agaatgtagt atatagcaat tgcttttctg tagtttataa gtgtgtatat tttaatttat     960
   aacttttcta atatatgacc aaaatttgtt gatgtgcagg tatcaccgtt tgtgtgaaca   1020
   acgaactgaa ctggcagact atcccgccgg gaatggtgat taccgacgaa aacggcaaga    1080
   aaaagcagtc ttacttccat gatttcttta actatgccgg aatccatcgc agcgtaatgc    1140
   tctacaccac gccgaacacc tgggtggacg atatcaccgt ggtgacgcat gtcgcgcaag    1200
   actgtaacca cgcgtctgtt gactggcagg tggtggccaa tggtgatgtc agcgttgaac    1260
   tgcgtgatgc ggatcaacag gtggttgcaa ctggacaagg cactagcggg actttgcaag    1320
   tggtgaatcc gcacctctgg caaccgggtg aaggttatct ctatgaactg tgcgtcacag 1380
   ccaaaagcca gacagagtgt gatatctacc cgcttcgcgt cggcatccgg tcagtggcag 1440
   tgaagggcga acagttcctg attaaccaca aaccgttcta ctttactggc tttggtcgtc 1500
   atgaagatgc ggacttgcgt ggcaaaggat tcgataacgt gctgatggtg cacgaccacg 1560
   cattaatgga ctggattggg gccaactcct accgtacctc gcattaccct tacgctgaag 1620
   agatgctcga ctgggcagat gaacatggca tcgtggtgat tgatgaaact gctgctgtcg 1680
   gctttaacct ctctttaggc attggtttcg aagcgggcaa caagccgaaa gaactgtaca 1740
   gcgaagaggc agtcaacggg gaaactcagc aagcgcactt acaggcgatt aaagagctga 1800
   tagcgcgtga caaaaaccac ccaagcgtgg tgatgtggag tattgccaac gaaccggata 1860
   cccgtccgca aggtgcacgg gaatatttcg cgccactggc ggaagcaacg cgtaaactcg 1920
   acccgacgcg tccgatcacc tgcgtcaatg taatgttctg cgacgctcac accgatacca 1980
   tcagcgatct ctttgatgtg ctgtgcctga accgttatta cggatggtat gtccaaagcg 2040
   gcgatttgga aacggcagag aaggtactgg aaaaagaact tctggcctgg caggagaaac 2100
   tgcatcagcc gattatcatc accgaatacg gcgtggatac gttagccggg ctgcactcaa 2160
   tgtacaccga catgtggagt gaagagtatc agtgtgcatg gctggatatg tatcaccgcg 2220
   tctttgatcg cgtcagcgcc gtcgtcggtg aacaggtatg gaatttcgcc gattttgcga 2280
   cctcgcaagg catattgcgc gttggcggta acaagaaagg gatcttcact cgcgaccgca 2340
   aaccgaagtc ggcggctttt ctgctgcaaa aacgctggac tggcatgaac ttcggtgaaa 2400
   aaccgcagca gggaggcaaa caatgaatca acaactctcc tggcgcacca tcgtcggcta 2460
   cagcctcggg aattagatcc ccgaatttcc ccgatcgttc aaacatttgg caataaagtt 2520
   tcttaagatt gaatcctgtt gccggtcttg cgatgattat catataattt ctgttgaatt 2580
   acgttaagca tgtaataatt aacatgtaat gcatgacgtt atttatgaga tgggttttta 2640
   tgattagagt cccgcaatta tacatttaat acgcgataga aaacaaaata tagcgcgcaa 2700
   actaggataa attatcgcgc gcggtgtcat 2730
<210> 42
   <211> 1577
   <212> DNA
   <213> Artificial
<220>
   <223> artificial sequence CmpC-synGFPI-nos expression cassette
<400> 42
   tggcagacaa agtggcagac atactgtccc acaaatgaag atggaatctg taaaagaaaa    60
   cgcgtgaaat aatgcgtctg acaaaggtta ggtcggctgc ctttaatcaa taccaaagtg   120
   gtccctacca cgatggaaaa actgtgcagt cggtttggct ttttctgacg aacaaataag   180
   attcgtggcc gacaggtggg ggtccaccat gtgaaggcat cttcagactc caataatgga   240
   gcaatgacgt aagggcttac gaaataagta agggtagttt gggaaatgtc cactcacccg   300
   tcagtctata aatacttagc ccctccctca ttgttaaggg agcaacgatc cgcgaagggc   360
   gaattcgttt aaacctgcag atgagcaagg gcgaggagct gttcaccggc gtggtgccaa   420
   tcctggtgga gctggacggc gacgtgaacg gccacaagtt cagcgtgagc ggcgagggcg   480
   agggcgacgc gacctacggc aagctgaccc tgaagttcat ctgtaccacc ggcaagctcc   540
   cggtcccgtg gccgaccctg gtgaccacct tcacctacgg cgtgcagtgt ttcagccgct   600
   acccggacca catgaagcgc cacgacttct tcaagagcgc catgccggag ggctacgtaa   660
   gtttctgctt ctacctttga tatatatata ataattatca ttaattagta gtaatataat   720
   atttcaaata tttttttcaa aataaaagaa tgtagtatat agcaattgct tttctgtagt   780
   ttataagtgt gtatatttta atttataact tttctaatat atgaccaaaa tttgttgatg   840
   tgcaggtgca ggagcgcacc atcagcttca aggacgacgg caactacaag acccgcgccg   900
   aggtgaagtt cgagggcgac acactagtga accgcatcga gctgaagggc atcgacttca   960
   aggaggacgg caacatcctg ggccacaagc tggagtacaa ctacaacagc cacaatgtgt  1020
   acatcaccgc ggacaagcag aagaacggca tcaaggcgaa cttcaagatc cgccacaata  1080
   tcgaggacgg cagcgtgcag ctggccgacc actaccagca gaacaccccg atcggcgacg  1140
   gtcctgtgct gctgccggac aaccactacc tgagcaccca gagcgccctg agcaaggacc  1200
   cgaacgagaa gcgcgaccac atggtgctgc tggagttcgt gaccgccgcc ggcatcaccc  1260
   acggcatgga cgagctgtac aaggttaact agagctctag atccccgaat ttccccgatc  1320
   gttcaaacat ttggcaataa agtttcttaa gattgaatcc tgttgccggt cttgcgatga  1380
   ttatcatata atttctgttg aattacgtta agcatgtaat aattaacatg taatgcatga 1440
   cgttatttat gagatgggtt tttatgatta gagtcccgca attatacatt taatacgcga 1500
   tagaaaacaa aatatagcgc gcaaactagg ataaattatc gcgcgcggtg tcatctatgt 1560
   tactagatcg ggaattg 1577
<210> 43
   <211> 2725
   <212> DNA
   <213> Artificial
<220>
   <223> artificial sequence CmpC-GIG-nos expression cassette
<400> 43
   tggcagacaa agtggcagac atactgtccc acaaatgaag atggaatctg taaaagaaaa 60
   cgcgtgaaat aatgcgtctg acaaaggtta ggtcggctgc ctttaatcaa taccaaagtg 120
   gtccctacca cgatggaaaa actgtgcagt cggtttggct ttttctgacg aacaaataag 180
   attcgtggcc gacaggtggg ggtccaccat gtgaaggcat cttcagactc caataatgga 240
   gcaatgacgt aagggcttac gaaataagta agggtagttt gggaaatgtc cactcacccg 300
   tcagtctata aatacttagc ccctccctca ttgttaaggg agcaacgatc cgcgaagggc 360
   gaattcctgc agcccggggg atcccctcga ggtcgaccat ggtccgtcct gtagaaaccc 420
   caacccgtga aatcaaaaaa ctcgacggcc tgtgggcatt cagtctggat cgcgaaaact 480
   gtggaattga tcagcgttgg tgggaaagcg cgttacaaga aagccgggca attgctgtgc 540
   caggcagttt taacgatcag ttcgccgatg cagatattcg taattatgcg ggcaacgtct 600
   ggtatcagcg cgaagtcttt ataccgaaag gttgggcagg ccagcgtatc gtgctgcgtt 660
   tcgatgcggt cactcattac ggcaaagtgt gggtcaataa tcaggaagtg atggagcatc 720
   agggcggcta tacgccattt gaagccgatg tcacgccgta tgttattgcc gggaaaagtg 780
   tacgtaagtt tctgcttcta cctttgatat atatataata attatcatta attagtagta 840
   atataatatt tcaaatattt ttttcaaaat aaaagaatgt agtatatagc aattgctttt 900
   ctgtagttta taagtgtgta tattttaatt tataactttt ctaatatatg accaaaattt 960
   gttgatgtgc aggtatcacc gtttgtgtga acaacgaact gaactggcag actatcccgc 1020
   cgggaatggt gattaccgac gaaaacggca agaaaaagca gtcttacttc catgatttct 1080
   ttaactatgc cggaatccat cgcagcgtaa tgctctacac cacgccgaac acctgggtgg 1140
   acgatatcac cgtggtgacg catgtcgcgc aagactgtaa ccacgcgtct gttgactggc 1200
   aggtggtggc caatggtgat gtcagcgttg aactgcgtga tgcggatcaa caggtggttg 1260
   caactggaca aggcactagc gggactttgc aagtggtgaa tccgcacctc tggcaaccgg 1320
   gtgaaggtta tctctatgaa ctgtgcgtca cagccaaaag ccagacagag tgtgatatct 1380
   acccgcttcg cgtcggcatc cggtcagtgg cagtgaaggg cgaacagttc ctgattaacc 1440
   acaaaccgtt ctactttact ggctttggtc gtcatgaaga tgcggacttg cgtggcaaag 1500
   gattcgataa cgtgctgatg gtgcacgacc acgcattaat ggactggatt ggggccaact 1560
   cctaccgtac ctcgcattac ccttacgctg aagagatgct cgactgggca gatgaacatg 1620
   gcatcgtggt gattgatgaa actgctgctg tcggctttaa cctctcttta ggcattggtt 1680
   tcgaagcggg caacaagccg aaagaactgt acagcgaaga ggcagtcaac ggggaaactc 1740
   agcaagcgca cttacaggcg attaaagagc tgatagcgcg tgacaaaaac cacccaagcg 1800
   tggtgatgtg gagtattgcc aacgaaccgg atacccgtcc gcaaggtgca cgggaatatt 1860
   tcgcgccact ggcggaagca acgcgtaaac tcgacccgac gcgtccgatc acctgcgtca 1920
   atgtaatgtt ctgcgacgct cacaccgata ccatcagcga tctctttgat gtgctgtgcc 1980
   tgaaccgtta ttacggatgg tatgtccaaa gcggcgattt ggaaacggca gagaaggtac 2040
   tggaaaaaga acttctggcc tggcaggaga aactgcatca gccgattatc atcaccgaat 2100
   acggcgtgga tacgttagcc gggctgcact caatgtacac cgacatgtgg agtgaagagt 2160
   atcagtgtgc atggctggat atgtatcacc gcgtctttga tcgcgtcagc gccgtcgtcg 2220
   gtgaacaggt atggaatttc gccgattttg cgacctcgca aggcatattg cgcgttggcg 2280
   gtaacaagaa agggatcttc actcgcgacc gcaaaccgaa gtcggcggct tttctgctgc 2340
   aaaaacgctg gactggcatg aacttcggtg aaaaaccgca gcagggaggc aaacaatgaa 2400
   tcaacaactc tcctggcgca ccatcgtcgg ctacagcctc gggaattaga tccccgaatt 2460
   tccccgatcg ttcaaacatt tggcaataaa gtttcttaag attgaatcct gttgccggtc 2520
   ttgcgatgat tatcatataa tttctgttga attacgttaa gcatgtaata attaacatgt 2580
   aatgcatgac gttatttatg agatgggttt ttatgattag agtcccgcaa ttatacattt 2640
   aatacgcgat agaaaacaaa atatagcgcg caaactagga taaattatcg cgcgcggtgt 2700
   catctatgtt actagatcgg gaatt 2725
<210> 44
   <211> 9172
   <212> DNA
   <213> Artificial
<220>
   <223> artificial sequence vector pNOV2117
<400> 44
   aagcttggcg cgccggtacc agcttgcatg cctgcagtgc agcgtgaccc ggtcgtgccc 60
   ctctctagag ataatgagca ttgcatgtct aagttataaa aaattaccac atattttttt 120
   tgtcacactt gtttgaagtg cagtttatct atctttatac atatatttaa actttactct 180
   acgaataata taatctatag tactacaata atatcagtgt tttagagaat catataaatg 240
   aacagttaga catggtctaa aggacaattg agtattttga caacaggact ctacagtttt 300
   atctttttag tgtgcatgtg ttctcctttt tttttgcaaa tagcttcacc tatataatac 360
   ttcatccatt ttattagtac atccatttag ggtttagggt taatggtttt tatagactaa 420
   tttttttagt acatctattt tattctattt tagcctctaa attaagaaaa ctaaaactct 480
   attttagttt ttttatttaa taatttagat ataaaataga ataaaataaa gtgactaaaa 540
   attaaacaaa taccctttaa gaaattaaaa aaactaagga aacatttttc ttgtttcgag 600
   tagataatgc cagcctgtta aacgccgtcg acgagtctaa cggacaccaa ccagcgaacc 660
   agcagcgtcg cgtcgggcca agcgaagcag acggcacggc atctctgtcg ctgcctctgg 720
   acccctctcg agagttccgc tccaccgttg gacttgctcc gctgtcggca tccagaaatt 780
   gcgtggcgga gcggcagacg tgagccggca cggcaggcgg cctcctcctc ctctcacggc 840
   accggcagct acgggggatt cctttcccac cgctccttcg ctttcccttc ctcgcccgcc 900
   gtaataaata gacaccccct ccacaccctc tttccccaac ctcgtgttgt tcggagcgca 960
   cacacacaca accagatctc ccccaaatcc acccgtcggc acctccgctt caaggtacgc 1020
   cgctcgtcct cccccccccc ccctctctac cttctctaga tcggcgttcc ggtccatggt 1080
   tagggcccgg tagttctact tctgttcatg tttgtgttag atccgtgttt gtgttagatc 1140
   cgtgctgcta gcgttcgtac acggatgcga cctgtacgtc agacacgttc tgattgctaa 1200
   cttgccagtg tttctctttg gggaatcctg ggatggctct agccgttccg cagacgggat 1260
   cgatttcatg attttttttg tttcgttgca tagggtttgg tttgcccttt tcctttattt 1320
   caatatatgc cgtgcacttg tttgtcgggt catcttttca tgcttttttt tgtcttggtt 1380
   gtgatgatgt ggtctggttg ggcggtcgtt ctagatcgga gtagaattct gtttcaaact 1440
   acctggtgga tttattaatt ttggatctgt atgtgtgtgc catacatatt catagttacg 1500
   aattgaagat gatggatgga aatatcgatc taggataggt atacatgttg atgcgggttt 1560
   tactgatgca tatacagaga tgctttttgt tcgcttggtt gtgatgatgt ggtgtggttg 1620
   ggcggtcgtt cattcgttct agatcggagt agaatactgt ttcaaactac ctggtgtatt 1680
   tattaatttt ggaactgtat gtgtgtgtca tacatcttca tagttacgag tttaagatgg 1740
   atggaaatat cgatctagga taggtataca tgttgatgtg ggttttactg atgcatatac 1800
   atgatggcat atgcagcatc tattcatatg ctctaacctt gagtacctat ctattataat 1860
   aaacaagtat gttttataat tattttgatc ttgatatact tggatgatgg catatgcagc 1920
   agctatatgt ggattttttt agccctgcct tcatacgcta tttatttgct tggtactgtt 1980
   tcttttgtcg atgctcaccc tgttgtttgg tgttacttct gcagggatcc ccgatcatgc 2040
   aaaaactcat taactcagtg caaaactatg cctggggcag caaaacggcg ttgactgaac 2100
   tttatggtat ggaaaatccg tccagccagc cgatggccga gctgtggatg ggcgcacatc 2160
   cgaaaagcag ttcacgagtg cagaatgccg ccggagatat cgtttcactg cgtgatgtga 2220
   ttgagagtga taaatcgact ctgctcggag aggccgttgc caaacgcttt ggcgaactgc 2280
   ctttcctgtt caaagtatta tgcgcagcac agccactctc cattcaggtt catccaaaca 2340
   aacacaattc tgaaatcggt tttgccaaag aaaatgccgc aggtatcccg atggatgccg 2400
   ccgagcgtaa ctataaagat cctaaccaca agccggagct ggtttttgcg ctgacgcctt 2460
   tccttgcgat gaacgcgttt cgtgaatttt ccgagattgt ctccctactc cagccggtcg 2520
   caggtgcaca tccggcgatt gctcactttt tacaacagcc tgatgccgaa cgtttaagcg 2580
   aactgttcgc cagcctgttg aatatgcagg gtgaagaaaa atcccgcgcg ctggcgattt 2640
   taaaatcggc cctcgatagc cagcagggtg aaccgtggca aacgattcgt ttaatttctg 2700
   aattttaccc ggaagacagc ggtctgttct ccccgctatt gctgaatgtg gtgaaattga 2760
   accctggcga agcgatgttc ctgttcgctg aaacaccgca cgcttacctg caaggcgtgg 2820
   cgctggaagt gatggcaaac tccgataacg tgctgcgtgc gggtctgacg cctaaataca 2880
   ttgatattcc ggaactggtt gccaatgtga aattcgaagc caaaccggct aaccagttgt 2940
   tgacccagcc ggtgaaacaa ggtgcagaac tggacttccc gattccagtg gatgattttg 3000
   ccttctcgct gcatgacctt agtgataaag aaaccaccat tagccagcag agtgccgcca 3060
   ttttgttctg cgtcgaaggc gatgcaacgt tgtggaaagg ttctcagcag ttacagctta 3120
   aaccgggtga atcagcgttt attgccgcca acgaatcacc ggtgactgtc aaaggccacg 3180
   gccgtttagc gcgtgtttac aacaagctgt aagagcttac tgaaaaaatt aacatctctt 3240
   gctaagctgg gagctcgatc cgtcgacctg cagatcgttc aaacatttgg caataaagtt 3300
   tcttaagatt gaatcctgtt gccggtcttg cgatgattat catataattt ctgttgaatt 3360
   acgttaagca tgtaataatt aacatgtaat gcatgacgtt atttatgaga tgggttttta 3420
   tgattagagt cccgcaatta tacatttaat acgcgataga aaacaaaata tagcgcgcaa 3480
   actaggataa attatcgcgc gcggtgtcat ctatgttact agatctgcta gccctgcagg 3540
   aaatttaccg gtgcccgggc ggccagcatg gccgtatccg caatgtgtta ttaagttgtc 3600
   taagcgtcaa tttgtttaca ccacaatata tcctgccacc agccagccaa cagctccccg 3660
   accggcagct cggcacaaaa tcaccactcg atacaggcag cccatcagaa ttaattctca 3720
   tgtttgacag cttatcatcg actgcacggt gcaccaatgc ttctggcgtc aggcagccat 3780
   cggaagctgt ggtatggctg tgcaggtcgt aaatcactgc ataattcgtg tcgctcaagg 3840
   cgcactcccg ttctggataa tgttttttgc gccgacatca taacggttct ggcaaatatt 3900
   ctgaaatgag ctgttgacaa ttaatcatcc ggctcgtata atgtgtggaa ttgtgagcgg 3960
   ataacaattt cacacaggaa acagaccatg agggaagcgt tgatcgccga agtatcgact 4020
   caactatcag aggtagttgg cgtcatcgag cgccatctcg aaccgacgtt gctggccgta 4080
   catttgtacg gctccgcagt ggatggcggc ctgaagccac acagtgatat tgatttgctg 4140
   gttacggtga ccgtaaggct tgatgaaaca acgcggcgag ctttgatcaa cgaccttttg 4200
   gaaacttcgg cttcccctgg agagagcgag attctccgcg ctgtagaagt caccattgtt 4260
   gtgcacgacg acatcattcc gtggcgttat ccagctaagc gcgaactgca atttggagaa 4320
   tggcagcgca atgacattct tgcaggtatc ttcgagccag ccacgatcga cattgatctg 4380
   gctatcttgc tgacaaaagc aagagaacat agcgttgcct tggtaggtcc agcggcggag 4440
   gaactctttg atccggttcc tgaacaggat ctatttgagg cgctaaatga aaccttaacg 4500
   ctatggaact cgccgcccga ctgggctggc gatgagcgaa atgtagtgct tacgttgtcc 4560
   cgcatttggt acagcgcagt aaccggcaaa atcgcgccga aggatgtcgc tgccgactgg 4620
   gcaatggagc gcctgccggc ccagtatcag cccgtcatac ttgaagctag gcaggcttat 4680
   cttggacaag aagatcgctt ggcctcgcgc gcagatcagt tggaagaatt tgttcactac 4740
   gtgaaaggcg agatcaccaa agtagtcggc aaataaagct ctagtggatc tccgtacccc 4800
   cgggggatct ggctcgcggc ggacgcacga cgccggggcg agaccatagg cgatctccta 4860
   aatcaatagt agctgtaacc tcgaagcgtt tcacttgtaa caacgattga gaatttttgt 4920
   cataaaattg aaatacttgg ttcgcatttt tgtcatccgc ggtcagccgc aattctgacg 4980
   aactgcccat ttagctggag atgattgtac atccttcacg tgaaaatttc tcaagcgctg 5040
   tgaacaaggg ttcagatttt agattgaaag gtgagccgtt gaaacacgtt cttcttgtcg 5100
   atgacgacgt cgctatgcgg catcttatta ttgaatacct tacgatccac gccttcaaag 5160
   tgaccgcggt agccgacagc acccagttca caagagtact ctcttccgcg acggtcgatg 5220
   tcgtggttgt tgatctaaat ttaggtcgtg aagatgggct cgagatcgtt cgtaatctgg 5280
   cggcaaagtc tgatattcca atcataatta tcagtggcga ccgccttgag gagacggata 5340
   aagttgttgc actcgagcta ggagcaagtg attttatcgc taagccgttc agtatcagag 5400
   agtttctagc acgcattcgg gttgccttgc gcgtgcgccc caacgttgtc cgctccaaag 5460
   accgacggtc tttttgtttt actgactgga cacttaatct caggcaacgt cgcttgatgt 5520
   ccgaagctgg cggtgaggtg aaacttacgg caggtgagtt caatcttctc ctcgcgtttt 5580
   tagagaaacc ccgcgacgtt ctatcgcgcg agcaacttct cattgccagt cgagtacgcg 5640
   acgaggaggt ttatgacagg agtatagatg ttctcatttt gaggctgcgc cgcaaacttg 5700
   aggcagatcc gtcaagccct caactgataa aaacagcaag aggtgccggt tatttctttg 5760
   acgcggacgt gcaggtttcg cacgggggga cgatggcagc ctgagccaat tcccagatcc 5820
   ccgaggaatc ggcgtgagcg gtcgcaaacc atccggcccg gtacaaatcg gcgcggcgct 5880
   gggtgatgac ctggtggaga agttgaaggc cgcgcaggcc gcccagcggc aacgcatcga 5940
   ggcagaagca cgccccggtg aatcgtggca agcggccgct gatcgaatcc gcaaagaatc 6000
   ccggcaaccg ccggcagccg gtgcgccgtc gattaggaag ccgcccaagg gcgacgagca 6060
   accagatttt ttcgttccga tgctctatga cgtgggcacc cgcgatagtc gcagcatcat 6120
   ggacgtggcc gttttccgtc tgtcgaagcg tgaccgacga gctggcgagg tgatccgcta 6180
   cgagcttcca gacgggcacg tagaggtttc cgcagggccg gccggcatgg ccagtgtgtg 6240
   ggattacgac ctggtactga tggcggtttc ccatctaacc gaatccatga accgataccg 6300
   ggaagggaag ggagacaagc ccggccgcgt gttccgtcca cacgttgcgg acgtactcaa 6360
   gttctgccgg cgagccgatg gcggaaagca gaaagacgac ctggtagaaa cctgcattcg 6420
   gttaaacacc acgcacgttg ccatgcagcg tacgaagaag gccaagaacg gccgcctggt 6480
   gacggtatcc gagggtgaag ccttgattag ccgctacaag atcgtaaaga gcgaaaccgg 6540
   gcggccggag tacatcgaga tcgagctagc tgattggatg taccgcgaga tcacagaagg 6600
   caagaacccg gacgtgctga cggttcaccc cgattacttt ttgatcgatc ccggcatcgg 6660
   ccgttttctc taccgcctgg cacgccgcgc cgcaggcaag gcagaagcca gatggttgtt 6720
   caagacgatc tacgaacgca gtggcagcgc cggagagttc aagaagttct gtttcaccgt 6780
   gcgcaagctg atcgggtcaa atgacctgcc ggagtacgat ttgaaggagg aggcggggca 6840
   ggctggcccg atcctagtca tgcgctaccg caacctgatc gagggcgaag catccgccgg 6900
   ttcctaatgt acggagcaga tgctagggca aattgcccta gcaggggaaa aaggtcgaaa 6960
   aggtctcttt cctgtggata gcacgtacat tgggaaccca aagccgtaca ttgggaaccg 7020
   gaacccgtac attgggaacc caaagccgta cattgggaac cggtcacaca tgtaagtgac 7080
   tgatataaaa gagaaaaaag gcgatttttc cgcctaaaac tctttaaaac ttattaaaac 7140
   tcttaaaacc cgcctggcct gtgcataact gtctggccag cgcacagccg aagagctgca 7200
   aaaagcgcct acccttcggt cgctgcgctc cctacgcccc gccgcttcgc gtcggcctat 7260
   cgcggccgct ggccgctcaa aaatggctgg cctacggcca ggcaatctac cagggcgcgg 7320
   acaagccgcg ccgtcgccac tcgaccgccg gcgctgaggt ctgcctcgtg aagaaggtgt 7380
   tgctgactca taccaggcct gaatcgcccc atcatccagc cagaaagtga gggagccacg 7440
   gttgatgaga gctttgttgt aggtggacca gttggtgatt ttgaactttt gctttgccac 7500
   ggaacggtct gcgttgtcgg gaagatgcgt gatctgatcc ttcaactcag caaaagttcg 7560
   atttattcaa caaagccgcc gtcccgtcaa gtcagcgtaa tgctctgcca gtgttacaac 7620
   caattaacca attctgatta gaaaaactca tcgagcatca aatgaaactg caatttattc 7680
   atatcaggat tatcaatacc atatttttga aaaagccgtt tctgtaatga aggagaaaac 7740
   tcaccgaggc agttccatag gatggcaaga tcctggtatc ggtctgcgat tccgactcgt 7800
   ccaacatcaa tacaacctat taatttcccc tcgtcaaaaa taaggttatc aagtgagaaa 7860
   tcaccatgag tgacgactga atccggtgag aatggcaaaa gctctgcatt aatgaatcgg 7920
   ccaacgcgcg gggagaggcg gtttgcgtat tgggcgctct tccgcttcct cgctcactga 7980
   ctcgctgcgc tcggtcgttc ggctgcggcg agcggtatca gctcactcaa aggcggtaat 8040
   acggttatcc acagaatcag gggataacgc aggaaagaac atgtgagcaa aaggccagca 8100
   aaaggccagg aaccgtaaaa aggccgcgtt gctggcgttt ttccataggc tccgcccccc 8160
   tgacgagcat cacaaaaatc gacgctcaag tcagaggtgg cgaaacccga caggactata 8220
   aagataccag gcgtttcccc ctggaagctc cctcgtgcgc tctcctgttc cgaccctgcc 8280
   gcttaccgga tacctgtccg cctttctccc ttcgggaagc gtggcgcttt ctcatagctc 8340
   acgctgtagg tatctcagtt cggtgtaggt cgttcgctcc aagctgggct gtgtgcacga 8400
   accccccgtt cagcccgacc gctgcgcctt atccggtaac tatcgtcttg agtccaaccc 8460
   ggtaagacac gacttatcgc cactggcagc agccactggt aacaggatta gcagagcgag 8520
   gtatgtaggc ggtgctacag agttcttgaa gtggtggcct aactacggct acactagaag 8580
   aacagtattt ggtatctgcg ctctgctgaa gccagttacc ttcggaaaaa gagttggtag 8640
   ctcttgatcc ggcaaacaaa ccaccgctgg tagcggtggt ttttttgttt gcaagcagca 8700
   gattacgcgc agaaaaaaag gatctcaaga agatcctttg atcttttcta cggggtctga 8760
   cgctcagtgg aacgaaaact cacgttaagg gattttggtc atgagattat caaaaaggat 8820
   cttcacctag atccttttga tccggaatta attcctgtgg ttggcatgca catacaaatg 8880
   gacgaacgga taaacctttt cacgcccttt taaatatccg attattctaa taaacgctct 8940
   tttctcttag gtttacccgc caatatatcc tgtcaaacac tgatagttta aactgaaggc 9000
   gggaaacgac aatctgatca tgagcggaga attaagggag tcacgttatg acccccgccg 9060
   atgacgcggg acaagccgtt ttacgtttgg aactgacaga accgcaacgc tgcaggaatt 9120
   ggccgcagcg gccatttaaa tcaattgggc gcgccgaatt cgagctcggt ac 9172
<210> 45
   <211> 8849
   <212> DNA
   <213> Artificial
<220>
   <223> artificial sequence vector pNOV4200
<400> 45
   ggtacccccg ggggatcctc tagagtcgac catggtgatc actgcaggca tgcaagcttc      60
   gtacgttaat taattcgaat ccggagcggc cgcacgcgtg ggcccgttta aacctcgaga     120
   gatctgctag ccctgcagga aatttaccgg tgcccgtacc ggatttggag ccaagtctca     180
   taaacgccat tgtggaagaa agtcttgagt tggtggtaat gtaacagagt agtaagaaca     240
   gagaagagag agagtgtgag atacatgaat tgtcgggcaa caaaaatcct gaacatctta     300
   ttttagcaaa gagaaagagt tccgagtctg tagcagaaga gtgaggagaa atttaagctc     360
   ttggacttgt gaattgttcc gcctcttgaa tacttcttca atcctcatat attcttcttc     420
   tatgttacct gaaaaccggc atttaatctc gcgggtttat tccggttcaa catttttttt     480
   gttttgagtt attatctggg cttaataacg caggcctgaa ataaattcaa ggcccaactg     540
   tttttttttt taagaagttg ctgttaaaaa aaaaaaaagg gaattaacaa caacaacaaa     600
   aaaagataaa gaaaataata acaattactt taattgtaga ctaaaaaaac atagatttta     660
   tcatgaaaaa aagagaaaag aaataaaaac ttggatcaaa aaaaaacata cagatcttct     720
   aattattaac ttttcttaaa aattaggtcc tttttcccaa caattaggtt tagagttttg     780
   gaattaaacc aaaaagattg ttctaaaaaa tactcaaatt tggtagataa gtttccttat     840
   tttaattagt caatggtaga tacttttttt tcttttcttt attagagtag attagaatct     900
   tttatgccaa gtattgataa attaaatcaa gaagataaac tatcataatc aacatgaaat     960
   taaaagaaaa atctcatata tagtattagt attctctata tatattatga ttgcttattc    1020
   ttaatgggtt gggttaacca agacatagtc ttaatggaaa gaatcttttt tgaacttttt    1080
   ccttattgat taaattcttc tatagaaaag aaagaaatta tttgaggaaa agtatataca    1140
   aaaagaaaaa tagaaaaatg tcagtgaagc agatgtaatg gatgacctaa tccaaccacc    1200
   accataggat gtttctactt gagtcggtct tttaaaaacg cacggtggaa aatatgacac 1260
   gtatcatatg attccttcct ttagtttcgt gataataatc ctcaactgat atcttccttt 1320
   ttttgttttg gctaaagata ttttattctc attaatagaa aagacggttt tgggcttttg 1380
   gtttgcgata taaagaagac cttcgtgtgg aagataataa ttcatccttt cgtctttttc 1440
   tgactcttca atctctccca aagcctaaag cgatctctgc aaatctctcg cgactctctc 1500
   tttcaaggta tattttctga ttctttttgt ttttgattcg tatctgatct ccaatttttg 1560
   ttatgtggat tattgaatct tttgtataaa ttgcttttga caatattgtt cgtttcgtca 1620
   atccagcttc taaattttgt cctgattact aagatatcga ttcgtagtgt ttacatctgt 1680
   gtaatttctt gcttgattgt gaaattagga ttttcaagga cgatctattc aatttttgtg 1740
   ttttctttgt tcgattctct ctgttttagg tttcttatgt ttagatccgt ttctctttgg 1800
   tgttgttttg atttctctta cggcttttga tttggtatat gttcgctgat tggtttctac 1860
   ttgttctatt gttttatttc aggtggatct cgactctagg ggggcaataa gatatgaaaa 1920
   agcctgaact caccgcgacg tctgtcgaga agtttctgat cgaaaagttc gacagcgtct 1980
   ccgacctgat gcagctctcg gagggcgaag aatctcgtgc tttcagcttc gatgtaggag 2040
   ggcgtggata tgtcctgcgg gtaaatagct gcgccgatgg tttctacaaa gatcgttatg 2100
   tttatcggca ctttgcatcg gccgcgctcc cgattccgga agtgcttgac attggggcat 2160
   tcagcgagag cctgacctat tgcatctccc gccgtgcaca gggtgtcacg ttgcaagacc 2220
   tgcctgaaac cgaactgccc gctgttctgc agccggtcgc ggaggccatg gatgcgatcg 2280
   ctgcggccga tcttagccag acgagcgggt tcggcccatt cggaccgcaa ggaatcggtc 2340
   aatacactac atggcgtgat ttcatatgcg cgattgctga tccccatgtg tatcactggc 2400
   aaactgtgat ggacgacacc gtcagtgcgt ccgtcgcgca ggctctcgat gagctgatgc 2460
   tttgggccga ggactgcccc gaagtccggc acctcgtgca cgcggatttc ggctccaaca 2520
   atgtcctgac ggacaatggc cgcataacag cggtcattga ctggagcgag gcgatgttcg 2580
   gggattccca atacgaggtc gccaacatct tcttctggag gccgtggttg gcttgtatgg 2640
   agcagcagac gcgctacttc gagcggaggc atccggagct tgcaggatcg ccgcggctcc 2700
   gggcgtatat gctccgcatt ggtcttgacc aactctatca gagcttggtt gacggcaatt 2760
   tcgatgatgc agcttgggcg cagggtcgat gcgacgcaat cgtccgatcc ggagccggga 2820
   ctgtcgggcg tacacaaatc gcccgcagaa gcgcggccgt ctggaccgat ggctgtgtag 2880
   aagtactcgc cgatagtgga aaccgacgcc ccagcactcg tccgagggca aaggaataga 2940
   gtagatgccg accgggatcc ccgaatttcc ccgatcgttc aaacatttgg caataaagtt 3000
   tcttaagatt gaatcctgtt gccggtcttg cgatgattat catataattt ctgttgaatt 3060
   acgttaagca tgtaataatt aacatgtaat gcatgacgtt atttatgaga tgggttttta 3120
   tgattagagt cccgcaatta tacatttaat acgcgataga aaacaaaata tagcgcgcaa 3180
   actaggataa attatcgcgc gcggtgtcat ctatgttact agatcgggaa ttgggtacgg 3240
   gcggccagca tggccgtatc cgcaatgtgt tattaagttg tctaagcgtc aatttgttta 3300
   caccacaata tatcctgcca ccagccagcc aacagctccc cgaccggcag ctcggcacaa 3360
   aatcaccact cgatacaggc agcccatcag aattaattct catgtttgac agcttatcat 3420
   cgactgcacg gtgcaccaat gcttctggcg tcaggcagcc atcggaagct gtggtatggc 3480
   tgtgcaggtc gtaaatcact gcataattcg tgtcgctcaa ggcgcactcc cgttctggat 3540
   aatgtttttt gcgccgacat cataacggtt ctggcaaata ttctgaaatg agctgttgac 3600
   aattaatcat ccggctcgta taatgtgtgg aattgtgagc ggataacaat ttcacacagg 3660
   aaacagacca tgagggaagc gttgatcgcc gaagtatcga ctcaactatc agaggtagtt 3720
   ggcgtcatcg agcgccatct cgaaccgacg ttgctggccg tacatttgta cggctccgca 3780
   gtggatggcg gcctgaagcc acacagtgat attgatttgc tggttacggt gaccgtaagg 3840
   cttgatgaaa caacgcggcg agctttgatc aacgaccttt tggaaacttc ggcttcccct 3900
   ggagagagcg agattctccg cgctgtagaa gtcaccattg ttgtgcacga cgacatcatt 3960
   ccgtggcgtt atccagctaa gcgcgaactg caatttggag aatggcagcg caatgacatt 4020
   cttgcaggta tcttcgagcc agccacgatc gacattgatc tggctatctt gctgacaaaa 4080
   gcaagagaac atagcgttgc cttggtaggt ccagcggcgg aggaactctt tgatccggtt 4140
   cctgaacagg atctatttga ggcgctaaat gaaaccttaa cgctatggaa ctcgccgccc 4200
   gactgggctg gcgatgagcg aaatgtagtg cttacgttgt cccgcatttg gtacagcgca 4260
   gtaaccggca aaatcgcgcc gaaggatgtc gctgccgact gggcaatgga gcgcctgccg 4320
   gcccagtatc agcccgtcat acttgaagct aggcaggctt atcttggaca agaagatcgc 4380
   ttggcctcgc gcgcagatca gttggaagaa tttgttcact acgtgaaagg cgagatcacc 4440
   aaagtagtcg gcaaataaag ctctagtgga tctccgtacc cccgggggat ctggctcgcg 4500
   gcggacgcac gacgccgggg cgagaccata ggcgatctcc taaatcaata gtagctgtaa 4560
   cctcgaagcg tttcacttgt aacaacgatt gagaattttt gtcataaaat tgaaatactt 4620
   ggttcgcatt tttgtcatcc gcggtcagcc gcaattctga cgaactgccc atttagctgg 4680
   agatgattgt acatccttca cgtgaaaatt tctcaagcgc tgtgaacaag ggttcagatt 4740
   ttagattgaa aggtgagccg ttgaaacacg ttcttcttgt cgatgacgac gtcgctatgc 4800
   ggcatcttat tattgaatac cttacgatcc acgccttcaa agtgaccgcg gtagccgaca 4860
   gcacccagtt cacaagagta ctctcttccg cgacggtcga tgtcgtggtt gttgatctag 4920
   atttaggtcg tgaagatggg ctcgagatcg ttcgtaatct ggcggcaaag tctgatattc 4980
   caatcataat tatcagtggc gaccgccttg aggagacgga taaagttgtt gcactcgagc 5040
   taggagcaag tgattttatc gctaagccgt tcagtatcag agagtttcta gcacgcattc 5100
   gggttgcctt gcgcgtgcgc cccaacgttg tccgctccaa agaccgacgg tctttttgtt 5160
   ttactgactg gacacttaat ctcaggcaac gtcgcttgat gtccgaagct ggcggtgagg 5220
   tgaaacttac ggcaggtgag ttcaatcttc tcctcgcgtt tttagagaaa ccccgcgacg 5280
   ttctatcgcg cgagcaactt ctcattgcca gtcgagtacg cgacgaggag gtttatgaca 5340
   ggagtataga tgttctcatt ttgaggctgc gccgcaaact tgaggcagat ccgtcaagcc 5400
   ctcaactgat aaaaacagca agaggtgccg gttatttctt tgacgcggac gtgcaggttt 5460
   cgcacggggg gacgatggca gcctgagcca attcccagat ccccgaggaa tcggcgtgag 5520
   cggtcgcaaa ccatccggcc cggtacaaat cggcgcggcg ctgggtgatg acctggtgga 5580
   gaagttgaag gccgcgcagg ccgcccagcg gcaacgcatc gaggcagaag cacgccccgg 5640
   tgaatcgtgg caagcggccg ctgatcgaat ccgcaaagaa tcccggcaac cgccggcagc 5700
   cggtgcgccg tcgattagga agccgcccaa gggcgacgag caaccagatt ttttcgttcc 5760
   gatgctctat gacgtgggca cccgcgatag tcgcagcatc atggacgtgg ccgttttccg 5820
   tctgtcgaag cgtgaccgac gagctggcga ggtgatccgc tacgagcttc cagacgggca 5880
   cgtagaggtt tccgcagggc cggccggcat ggccagtgtg tgggattacg acctggtact 5940
   gatggcggtt tcccatctaa ccgaatccat gaaccgatac cgggaaggga agggagacaa 6000
   gcccggccgc gtgttccgtc cacacgttgc ggacgtactc aagttctgcc ggcgagccga 6060
   tggcggaaag cagaaagacg acctggtaga aacctgcatt cggttaaaca ccacgcacgt 6120
   tgccatgcag cgtacgaaga aggccaagaa cggccgcctg gtgacggtat ccgagggtga 6180
   agccttgatt agccgctaca agatcgtaaa gagcgaaacc gggcggccgg agtacatcga 6240
   gatcgagcta gctgattgga tgtaccgcga gatcacagaa ggcaagaacc cggacgtgct 6300
   gacggttcac cccgattact ttttgatcga tcccggcatc ggccgttttc tctaccgcct 6360
   ggcacgccgc gccgcaggca aggcagaagc cagatggttg ttcaagacga tctacgaacg 6420
   cagtggcagc gccggagagt tcaagaagtt ctgtttcacc gtgcgcaagc tgatcgggtc 6480
   aaatgacctg ccggagtacg atttgaagga ggaggcgggg caggctggcc cgatcctagt 6540
   catgcgctac cgcaacctga tcgagggcga agcatccgcc ggttcctaat gtacggagca 6600
   gatgctaggg caaattgccc tagcagggga aaaaggtcga aaaggtctct ttcctgtgga 6660
   tagcacgtac attgggaacc caaagccgta cattgggaac cggaacccgt acattgggaa 6720
   cccaaagccg tacattggga accggtcaca catgtaagtg actgatataa aagagaaaaa 6780
   aggcgatttt tccgcctaaa actctttaaa acttattaaa actcttaaaa cccgcctggc 6840
   ctgtgcataa ctgtctggcc agcgcacagc cgaagagctg caaaaagcgc ctacccttcg 6900
   gtcgctgcgc tccctacgcc ccgccgcttc gcgtcggcct atcgcggccg ctggccgctc 6960
   aaaaatggct ggcctacggc caggcaatct accagggcgc ggacaagccg cgccgtcgcc 7020
   actcgaccgc cggcgctgag gtctgcctcg tgaagaaggt gttgctgact cataccaggc 7080
   ctgaatcgcc ccatcatcca gccagaaagt gagggagcca cggttgatga gagctttgtt 7140
   gtaggtggac cagttggtga ttttgaactt ttgctttgcc acggaacggt ctgcgttgtc 7200
   gggaagatgc gtgatctgat ccttcaactc agcaaaagtt cgatttattc aacaaagccg 7260
   ccgtcccgtc aagtcagcgt aatgctctgc cagtgttaca accaattaac caattctgat 7320
   tagaaaaact catcgagcat caaatgaaac tgcaatttat tcatatcagg attatcaata 7380
   ccatattttt gaaaaagccg tttctgtaat gaaggagaaa actcaccgag gcagttccat 7440
   aggatggcaa gatcctggta tcggtctgcg attccgactc gtccaacatc aatacaacct 7500
   attaatttcc cctcgtcaaa aataaggtta tcaagtgaga aatcaccatg agtgacgact 7560
   gaatccggtg agaatggcaa aagctctgca ttaatgaatc ggccaacgcg cggggagagg 7620
   cggtttgcgt attgggcgct cttccgcttc ctcgctcact gactcgctgc gctcggtcgt 7680
   tcggctgcgg cgagcggtat cagctcactc aaaggcggta atacggttat ccacagaatc 7740
   aggggataac gcaggaaaga acatgtgagc aaaaggccag caaaaggcca ggaaccgtaa 7800
   aaaggccgcg ttgctggcgt ttttccatag gctccgcccc cctgacgagc atcacaaaaa 7860
   tcgacgctca agtcagaggt ggcgaaaccc gacaggacta taaagatacc aggcgtttcc 7920
   ccctggaagc tccctcgtgc gctctcctgt tccgaccctg ccgcttaccg gatacctgtc 7980
   cgcctttctc ccttcgggaa gcgtggcgct ttctcatagc tcacgctgta ggtatctcag 8040
   ttcggtgtag gtcgttcgct ccaagctggg ctgtgtgcac gaaccccccg ttcagcccga 8100
   ccgctgcgcc ttatccggta actatcgtct tgagtccaac ccggtaagac acgacttatc 8160
   gccactggca gcagccactg gtaacaggat tagcagagcg aggtatgtag gcggtgctac 8220
   agagttcttg aagtggtggc ctaactacgg ctacactaga agaacagtat ttggtatctg 8280
   cgctctgctg aagccagtta ccttcggaaa aagagttggt agctcttgat ccggcaaaca 8340
   aaccaccgct ggtagcggtg gtttttttgt ttgcaagcag cagattacgc gcagaaaaaa 8400
   aggatctcaa gaagatcctt tgatcttttc tacggggtct gacgctcagt ggaacgaaaa 8460
   ctcacgttaa gggattttgg tcatgagatt atcaaaaagg atcttcacct agatcctttt 8520
   gatccggaat taattcctgt ggttggcatg cacatacaaa tggacgaacg gataaacctt 8580
   ttcacgccct tttaaatatc cgattattct aataaacgct cttttctctt aggtttaccc 8640
   gccaatatat cctgtcaaac actgatagtt taaactgaag gcgggaaacg acaatctgat 8700
   catgagcgga gaattaaggg agtcacgtta tgacccccgc cgatgacgcg ggacaagccg 8760
   ttttacgttt ggaactgaca gaaccgcaac gctgcaggaa ttggccgcag cggccattta 8820
   aatcaattgg gcgcgccgaa ttcgagctc 8849
<210> 46
   <211> 2949
   <212> DNA
   <213> Artificial
<220>
   <223> artificial sequence ZmUbi-GFP-35S term expression cassette
<400> 46
   gcatgcctgc agtgcagcgt gacccggtcg tgcccctctc tagagataat gagcattgca 60
   tgtctaagtt ataaaaaatt accacatatt ttttttgtca cacttgtttg aagtgcagtt 120
   tatctatctt tatacatata tttaaacttt actctacgaa taatataatc tatagtacta 180
   caataatatc agtgttttag agaatcatat aaatgaacag ttagacatgg tctaaaggac 240
   aattgagtat tttgacaaca ggactctaca gttttatctt tttagtgtgc atgtgttctc 300
   cttttttttt gcaaatagct tcacctatat aatacttcat ccattttatt agtacatcca 360
   tttagggttt agggttaatg gtttttatag actaattttt ttagtacatc tattttattc 420
   tattttagcc tctaaattaa gaaaactaaa actctatttt agttttttta tttaataatt 480
   tagatataaa atagaataaa ataaagtgac taaaaattaa acaaataccc tttaagaaat 540
   taaaaaaact aaggaaacat ttttcttgtt tcgagtagat aatgccagcc tgttaaacgc 600
   cgtcgacgag tctaacggac accaaccagc gaaccagcag cgtcgcgtcg ggccaagcga 660
   agcagacggc acggcatctc tgtcgctgcc tctggacccc tctcgagagt tccgctccac 720
   cgttggactt gctccgctgt cggcatccag aaattgcgtg gcggagcggc agacgtgagc 780
   cggcacggca ggcggcctcc tcctcctctc acggcacggc agctacgggg gattcctttc 840
   ccaccgctcc ttcgctttcc cttcctcgcc cgccgtaata aatagacacc ccctccacac 900
   cctctttccc caacctcgtg ttgttcggag cgcacacaca cacaaccaga tctcccccaa 960
   atccacccgt cggcacctcc gcttcaaggt acgccgctcg tcctcccccc ccccccctct 1020
   ctaccttctc tagatcggcg ttccggtcca tggttagggc ccggtagttc tacttctgtt 1080
   catgtttgtg ttagatccgt gtttgtgtta gatccgtgct gctagcgttc gtacacggat 1140
   gcgacctgta cgtcagacac gttctgattg ctaacttgcc agtgtttctc tttggggaat 1200
   cctgggatgg ctctagccgt tccgcagacg ggatcgattt catgattttt tttgtttcgt 1260
   tgcatagggt ttggtttgcc cttttccttt atttcaatat atgccgtgca cttgtttgtc 1320
   gggtcatctt ttcatgcttt tttttgtctt ggttgtgatg atgtggtctg gttgggcggt 1380
   cgttctagat cggagtagaa ttctgtttca aactacctgg tggatttatt aattttggat 1440
   ctgtatgtgt gtgccataca tattcatagt tacgaattga agatgatgga tggaaatatc 1500
   gatctaggat aggtatacat gttgatgcgg gttttactga tgcatataca gagatgcttt 1560
   ttgttcgctt ggttgtgatg atgtggtgtg gttgggcggt cgttcattcg ttctagatcg 1620
   gagtagaata ctgtttcaaa ctacctggtg tatttattaa ttttggaact gtatgtgtgt 1680
   gtcatacatc ttcatagtta cgagtttaag atggatggaa atatcgatct aggataggta 1740
   tacatgttga tgtgggtttt actgatgcat atacatgatg gcatatgcag catctattca 1800
   tatgctctaa ccttgagtac ctatctatta taataaacaa gtatgtttta taattatttt 1860
   gatcttgata tacttggatg atggcatatg cagcagctat atgtggattt ttttagccct 1920
   gccttcatac gctatttatt tgcttggtac tgtttctttt gtcgatgctc accctgttgt 1980
   ttggtgttac ttctgcaggt cgactctaga ggatccacca tgctgcagat gagcaagggc 2040
   gaggagctgt tcaccggcgt ggtgccaatc ctggtggagc tggacggcga cgtgaacggc 2100
   cacaagttca gcgtgagcgg cgagggcgag ggcgacgcga cctacggcaa gctgaccctg 2160
   aagttcatct gtaccaccgg caagctcccg gtcccgtggc cgaccctggt gaccaccttc 2220
   acctacggcg tgcagtgttt cagccgctac ccggaccaca tgaagcgcca cgacttcttc 2280
   aagagcgcca tgccggaggg ctacgtgcag gagcgcacca tcagcttcaa ggacgacggc 2340
   aactacaaga cccgcgccga ggtgaagttc gagggcgaca cactagtgaa ccgcatcgag 2400
   ctgaagggca tcgacttcaa ggaggacggc aacatcctgg gccacaagct ggagtacaac 2460
   tacaacagcc acaacgtgta catcaccgcg gacaagcaga agaacggcat caaggcgaac 2520
   ttcaagatcc gccacaacat cgaggacggc agcgtgcagc tggccgacca ctaccagcag 2580
   aacaccccga tcggcgacgg tcctgtgctg ctgccggaca accactacct gagcacccag 2640
   agcgccctga gcaaggaccc gaacgagaag cgcgaccaca tggtgctgct ggagttcgtg 2700
   accgccgccg gcatcaccca cggcatggac gagctgtaca aggttaacta gagctcaaga 2760
   tctgttctgc acaaagtgga gtagtcagtc atcgatcagg aaccagacac cagactttta 2820
   ttcatacagt gaagtgaagt gaagtgcagt gcagtgagtt gctggttttt gtacaactta 2880
   gtatgtattt gtatttgtaa aatacttcta tcaataaaat ttctaattcc taaaaccaaa 2940
   atccagtgg 2949
<210> 47
   <211> 4341
   <212> DNA
   <213> Artificial
<220>
   <223> artificial sequence ZmUBi-GIG-nos expression cassette
<400> 47
   cctgcagtgc agcgtgaccc ggtcgtgccc ctctctagag ataatgagca ttgcatgtct 60
   aagttataaa aaattaccac atattttttt tgtcacactt gtttgaagtg cagtttatct 120
   atctttatac atatatttaa actttactct acgaataata taatctatag tactacaata 180
   atatcagtgt tttagagaat catataaatg aacagttaga catggtctaa aggacaattg 240
   agtattttga caacaggact ctacagtttt atctttttag tgtgcatgtg ttctcctttt 300
   tttttgcaaa tagcttcacc tatataatac ttcatccatt ttattagtac atccatttag 360
   ggtttagggt taatggtttt tatagactaa tttttttagt acatctattt tattctattt 420
   tagcctctaa attaagaaaa ctaaaactct attttagttt ttttatttaa taatttagat 480
   ataaaataga ataaaataaa gtgactaaaa attaaacaaa taccctttaa gaaattaaaa 540
   aaactaagga aacatttttc ttgtttcgag tagataatgc cagcctgtta aacgccgtcg 600
   acgagtctaa cggacaccaa ccagcgaacc agcagcgtcg cgtcgggcca agcgaagcag 660
   acggcacggc atctctgtcg ctgcctctgg acccctctcg agagttccgc tccaccgttg 720
   gacttgctcc gctgtcggca tccagaaatt gcgtggcgga gcggcagacg tgagccggca 780
   cggcaggcgg cctcctcctc ctctcacggc accggcagct acgggggatt cctttcccac 840
   cgctccttcg ctttcccttc ctcgcccgcc gtaataaata gacaccccct ccacaccctc 900
   tttccccaac ctcgtgttgt tcggagcgca cacacacaca accagatctc ccccaaatcc 960
   acccgtcggc acctccgctt caaggtacgc cgctcgtcct cccccccccc ccctctctac 1020
   cttctctaga tcggcgttcc ggtccatggt tagggcccgg tagttctact tctgttcatg 1080
   tttgtgttag atccgtgttt gtgttagatc cgtgctgcta gcgttcgtac acggatgcga 1140
   cctgtacgtc agacacgttc tgattgctaa cttgccagtg tttctctttg gggaatcctg 1200
   ggatggctct agccgttccg cagacgggat cgatttcatg attttttttg tttcgttgca 1260
   tagggtttgg tttgcccttt tcctttattt caatatatgc cgtgcacttg tttgtcgggt 1320
   catcttttca tgcttttttt tgtcttggtt gtgatgatgt ggtctggttg ggcggtcgtt 1380
   ctagatcgga gtagaattct gtttcaaact acctggtgga tttattaatt ttggatctgt 1440
   atgtgtgtgc catacatatt catagttacg aattgaagat gatggatgga aatatcgatc 1500
   taggataggt atacatgttg atgcgggttt tactgatgca tatacagaga tgctttttgt 1560
   tcgcttggtt gtgatgatgt ggtgtggttg ggcggtcgtt cattcgttct agatcggagt 1620
   agaatactgt ttcaaactac ctggtgtatt tattaatttt ggaactgtat gtgtgtgtca 1680
   tacatcttca tagttacgag tttaagatgg atggaaatat cgatctagga taggtataca 1740
   tgttgatgtg ggttttactg atgcatatac atgatggcat atgcagcatc tattcatatg 1800
   ctctaacctt gagtacctat ctattataat aaacaagtat gttttataat tattttgatc 1860
   ttgatatact tggatgatgg catatgcagc agctatatgt ggattttttt agccctgcct 1920
   tcatacgcta tttatttgct tggtactgtt tcttttgtcg atgctcaccc tgttgtttgg 1980
   tgttacttct gcagggatcc cctcgaggtc gaccatggtc cgtcctgtag aaaccccaac 2040
   ccgtgaaatc aaaaaactcg acggcctgtg ggcattcagt ctggatcgcg aaaactgtgg 2100
   aattgatcag cgttggtggg aaagcgcgtt acaagaaagc cgggcaattg ctgtgccagg 2160
   cagttttaac gatcagttcg ccgatgcaga tattcgtaat tatgcgggca acgtctggta 2220
   tcagcgcgaa gtctttatac cgaaaggttg ggcaggccag cgtatcgtgc tgcgtttcga 2280
   tgcggtcact cattacggca aagtgtgggt caataatcag gaagtgatgg agcatcaggg 2340
   cggctatacg ccatttgaag ccgatgtcac gccgtatgtt attgccggga aaagtgtacg 2400
   taagtttctg cttctacctt tgatatatat ataataatta tcattaatta gtagtaatat 2460
   aatatttcaa atattttttt caaaataaaa gaatgtagta tatagcaatt gcttttctgt 2520
   agtttataag tgtgtatatt ttaatttata acttttctaa tatatgacca aaatttgttg 2580
   atgtgcaggt atcaccgttt gtgtgaacaa cgaactgaac tggcagacta tcccgccggg 2640
   aatggtgatt accgacgaaa acggcaagaa aaagcagtct tacttccatg atttctttaa 2700
   ctatgccgga atccatcgca gcgtaatgct ctacaccacg ccgaacacct gggtggacga 2760
   tatcaccgtg gtgacgcatg tcgcgcaaga ctgtaaccac gcgtctgttg actggcaggt 2820
   ggtggccaat ggtgatgtca gcgttgaact gcgtgatgcg gatcaacagg tggttgcaac 2880
   tggacaaggc actagcggga ctttgcaagt ggtgaatccg cacctctggc aaccgggtga 2940
   aggttatctc tatgaactgt gcgtcacagc caaaagccag acagagtgtg atatctaccc 3000
   gcttcgcgtc ggcatccggt cagtggcagt gaagggcgaa cagttcctga ttaaccacaa 3060
   accgttctac tttactggct ttggtcgtca tgaagatgcg gacttgcgtg gcaaaggatt 3120
   cgataacgtg ctgatggtgc acgaccacgc attaatggac tggattgggg ccaactccta 3180
   ccgtacctcg cattaccctt acgctgaaga gatgctcgac tgggcagatg aacatggcat 3240
   cgtggtgatt gatgaaactg ctgctgtcgg ctttaacctc tctttaggca ttggtttcga 3300
   agcgggcaac aagccgaaag aactgtacag cgaagaggca gtcaacgggg aaactcagca 3360
   agcgcactta caggcgatta aagagctgat agcgcgtgac aaaaaccacc caagcgtggt 3420
   gatgtggagt attgccaacg aaccggatac ccgtccgcaa ggtgcacggg aatatttcgc 3480
   gccactggcg gaagcaacgc gtaaactcga cccgacgcgt ccgatcacct gcgtcaatgt 3540
   aatgttctgc gacgctcaca ccgataccat cagcgatctc tttgatgtgc tgtgcctgaa 3600
   ccgttattac ggatggtatg tccaaagcgg cgatttggaa acggcagaga aggtactgga 3660
   aaaagaactt ctggcctggc aggagaaact gcatcagccg attatcatca ccgaatacgg 3720
   cgtggatacg ttagccgggc tgcactcaat gtacaccgac atgtggagtg aagagtatca 3780
   gtgtgcatgg ctggatatgt atcaccgcgt ctttgatcgc gtcagcgccg tcgtcggtga 3840
   acaggtatgg aatttcgccg attttgcgac ctcgcaaggc atattgcgcg ttggcggtaa 3900
   caagaaaggg atcttcactc gcgaccgcaa accgaagtcg gcggcttttc tgctgcaaaa 3960
   acgctggact ggcatgaact tcggtgaaaa accgcagcag ggaggcaaac aatgaatcaa 4020
   caactctcct ggcgcaccat cgtcggctac agcctcggga attagatccc cgaatttccc 4080
   cgatcgttca aacatttggc aataaagttt cttaagattg aatcctgttg ccggtcttgc 4140
   gatgattatc atataatttc tgttgaatta cgttaagcat gtaataatta acatgtaatg 4200
   catgacgtta tttatgagat gggtttttat gattagagtc ccgcaattat acatttaata 4260
   cgcgatagaa aacaaaatat agcgcgcaaa ctaggataaa ttatcgcgcg cggtgtcatc 4320
   tatgttacta gatcgggaat t 4341
<210> 48
   <211> 2943
   <212> DNA
   <213> Artificial
<220>
   <223> artificial sequence Ubq3(At)-synGFPI-nos expression cassette
<400> 48
   accggatttg gagccaagtc tcataaacgc cattgtggaa gaaagtcttg agttggtggt     60
   aatgtaacag agtagtaaga acagagaaga gagagagtgt gagatacatg aattgtcggg    120
   caacaaaaat cctgaacatc ttattttagc aaagagaaag agttccgagt ctgtagcaga    180
   agagtgagga gaaatttaag ctcttggact tgtgaattgt tccgcctctt gaatacttct    240
   tcaatcctca tatattcttc ttctatgtta cctgaaaacc ggcatttaat ctcgcgggtt    300
   tattccggtt caacattttt tttgttttga gttattatct gggcttaata acgcaggcct    360
   gaaataaatt caaggcccaa ctgttttttt ttttaagaag ttgctgttaa aaaaaaaaaa    420
   agggaattaa caacaacaac aaaaaaagat aaagaaaata ataacaatta ctttaattgt    480
   agactaaaaa aacatagatt ttatcatgaa aaaaagagaa aagaaataaa aacttggatc    540
   aaaaaaaaaa catacagatc ttctaattat taacttttct taaaaattag gtcctttttc    600
   ccaacaatta ggtttagagt tttggaatta aaccaaaaag attgttctaa aaaatactca    660
   aatttggtag ataagtttcc ttattttaat tagtcaatgg tagatacttt tttttctttt    720
   ctttattaga gtagattaga atcttttatg ccaagtattg ataaattaaa tcaagaagat    780
   aaactatcat aatcaacatg aaattaaaag aaaaatctca tatatagtat tagtattctc    840
   tatatatatt atgattgctt attcttaatg ggttgggtta accaagacat agtcttaatg    900
   gaaagaatct tttttgaact ttttccttat tgattaaatt cttctataga aaagaaagaa    960
   attatttgag gaaaagtata tacaaaaaga aaaatagaaa aatgtcagtg aagcagatgt   1020
   aatggatgac ctaatccaac caccaccata ggatgtttct acttgagtcg gtcttttaaa   1080
   aacgcacggt ggaaaatatg acacgtatca tatgattcct tcctttagtt tcgtgataat   1140
   aatcctcaac tgatatcttc ctttttttgt tttggctaaa gatattttat tctcattaat   1200
   agaaaagacg gttttgggct tttggtttgc gatataaaga agaccttcgt gtggaagata   1260
   ataattcatc ctttcgtctt tttctgactc ttcaatctct cccaaagcct aaagcgatct   1320
   ctgcaaatct ctcgcgactc tctctttcaa ggtatatttt ctgattcttt ttgtttttga   1380
   ttcgtatctg atctccaatt tttgttatgt ggattattga atcttttgta taaattgctt   1440
   ttgacaatat tgttcgtttc gtcaatccag cttctaaatt ttgtcctgat tactaagata   1500
   tcgattcgta gtgtttacat ctgtgtaatt tcttgcttga ttgtgaaatt aggattttca   1560
   aggacgatct attcaatttt tgtgttttct ttgttcgatt ctctctgttt taggtttctt   1620
   atgtttagat ccgtttctct ttggtgttgt tttgatttct cttacggctt ttgatttggt   1680
   atatgttcgc tgattggttt ctacttgttc tattgtttta tttcaggtgg atccaccatg 1740
   ctgcagatga gcaagggcga ggagctgttc accggcgtgg tgccaatcct ggtggagctg 1800
   gacggcgacg tgaacggcca caagttcagc gtgagcggcg agggcgaggg cgacgcgacc 1860
   tacggcaagc tgaccctgaa gttcatctgt accaccggca agctcccggt cccgtggccg 1920
   accctggtga ccaccttcac ctacggcgtg cagtgtttca gccgctaccc ggaccacatg 1980
   aagcgccacg acttcttcaa gagcgccatg ccggagggct acgtaagttt ctgcttctac 2040
   ctttgatata tatataataa ttatcattaa ttagtagtaa tataatattt caaatatttt 2100
   tttcaaaata aaagaatgta gtatatagca attgcttttc tgtagtttat aagtgtgtat 2160
   attttaattt ataacttttc taatatatga ccaaaatttg ttgatgtgca ggtgcaggag 2220
   cgcaccatca gcttcaagga cgacggcaac tacaagaccc gcgccgaggt gaagttcgag 2280
   ggcgacacac tagtgaaccg catcgagctg aagggcatcg acttcaagga ggacggcaac 2340
   atcctgggcc acaagctgga gtacaactac aacagccaca atgtgtacat caccgcggac 2400
   aagcagaaga acggcatcaa ggcgaacttc aagatccgcc acaatatcga ggacggcagc 2460
   gtgcagctgg ccgaccacta ccagcagaac accccgatcg gcgacggtcc tgtgctgctg 2520
   ccggacaacc actacctgag cacccagagc gccctgagca aggacccgaa cgagaagcgc 2580
   gaccacatgg tgctgctgga gttcgtgacc gccgccggca tcacccacgg catggacgag 2640
   ctgtacaagg ttaactagag ctctagatcc ccgaatttcc ccgatcgttc aaacatttgg 2700
   caataaagtt tcttaagatt gaatcctgtt gccggtcttg cgatgattat catataattt 2760
   ctgttgaatt acgttaagca tgtaataatt aacatgtaat gcatgacgtt atttatgaga 2820
   tgggttttta tgattagagt cccgcaatta tacatttaat acgcgataga aaacaaaata 2880
   tagcgcgcaa actaggataa attatcgcgc gcggtgtcat ctatgttact agatcgggaa 2940
   ttg 2943
<210> 49
   <211> 4072
   <212> DNA
   <213> Artificial
<220>
   <223> artificial sequence Ubq3(At)-GIG-nos expression cassette
<400> 49
   cggatttgga gccaagtctc ataaacgcca ttgtggaaga aagtcttgag ttggtggtaa 60
   tgtaacagag tagtaagaac agagaagaga gagagtgtga gatacatgaa ttgtcgggca 120
   acaaaaatcc tgaacatctt attttagcaa agagaaagag ttccgagtct gtagcagaag 180
   agtgaggaga aatttaagct cttggacttg tgaattgttc cgcctcttga atacttcttc 240
   aatcctcata tattcttctt ctatgttacc tgaaaaccgg catttaatct cgcgggttta 300
   ttccggttca acattttttt tgttttgagt tattatctgg gcttaataac gcaggcctga 360
   aataaattca aggcccaact gttttttttt ttaagaagtt gctgttaaaa aaaaaaaaag 420
   ggaattaaca acaacaacaa aaaaagataa agaaaataat aacaattact ttaattgtag 480
   actaaaaaaa catagatttt atcatgaaaa aaagagaaaa gaaataaaaa cttggatcaa 540
   aaaaaaacat acagatcttc taattattaa cttttcttaa aaattaggtc ctttttccca 600
   acaattaggt ttagagtttt ggaattaaac caaaaagatt gttctaaaaa atactcaaat 660
   ttggtagata agtttcctta ttttaattag tcaatggtag atactttttt ttcttttctt 720
   tattagagta gattagaatc ttttatgcca agtattgata aattaaatca agaagataaa 780
   ctatcataat caacatgaaa ttaaaagaaa aatctcatat atagtattag tattctctat 840
   atatattatg attgcttatt cttaatgggt tgggttaacc aagacatagt cttaatggaa 900
   agaatctttt ttgaactttt tccttattga ttaaattctt ctatagaaaa gaaagaaatt 960
   atttgaggaa aagtatatac aaaaagaaaa atagaaaaat gtcagtgaag cagatgtaat 1020
   ggatgaccta atccaaccac caccatagga tgtttctact tgagtcggtc ttttaaaaac 1080
   gcacggtgga aaatatgaca cgtatcatat gattccttcc tttagtttcg tgataataat 1140
   cctcaactga tatcttcctt tttttgtttt ggctaaagat attttattct cattaataga 1200
   aaagacggtt ttgggctttt ggtttgcgat ataaagaaga ccttcgtgtg gaagataata 1260
   attcatcctt tcgtcttttt ctgactcttc aatctctccc aaagcctaaa gcgatctctg 1320
   caaatctctc gcgactctct ctttcaaggt atattttctg attctttttg tttttgattc 1380
   gtatctgatc tccaattttt gttatgtgga ttattgaatc ttttgtataa attgcttttg 1440
   acaatattgt tcgtttcgtc aatccagctt ctaaattttg tcctgattac taagatatcg 1500
   attcgtagtg tttacatctg tgtaatttct tgcttgattg tgaaattagg attttcaagg 1560
   acgatctatt caatttttgt gttttctttg ttcgattctc tctgttttag gtttcttatg 1620
   tttagatccg tttctctttg gtgttgtttt gatttctctt acggcttttg atttggtata 1680
   tgttcgctga ttggtttcta cttgttctat tgttttattt caggtggatc ccctcgaggt 1740
   cgaccatggt ccgtcctgta gaaaccccaa cccgtgaaat caaaaaactc gacggcctgt 1800
   gggcattcag tctggatcgc gaaaactgtg gaattgatca gcgttggtgg gaaagcgcgt 1860
   tacaagaaag ccgggcaatt gctgtgccag gcagttttaa cgatcagttc gccgatgcag 1920
   atattcgtaa ttatgcgggc aacgtctggt atcagcgcga agtctttata ccgaaaggtt 1980
   gggcaggcca gcgtatcgtg ctgcgtttcg atgcggtcac tcattacggc aaagtgtggg 2040
   tcaataatca ggaagtgatg gagcatcagg gcggctatac gccatttgaa gccgatgtca 2100
   cgccgtatgt tattgccggg aaaagtgtac gtaagtttct gcttctacct ttgatatata 2160
   tataataatt atcattaatt agtagtaata taatatttca aatatttttt tcaaaataaa 2220
   agaatgtagt atatagcaat tgcttttctg tagtttataa gtgtgtatat tttaatttat 2280
   aacttttcta atatatgacc aaaatttgtt gatgtgcagg tatcaccgtt tgtgtgaaca 2340
   acgaactgaa ctggcagact atcccgccgg gaatggtgat taccgacgaa aacggcaaga 2400
   aaaagcagtc ttacttccat gatttcttta actatgccgg aatccatcgc agcgtaatgc 2460
   tctacaccac gccgaacacc tgggtggacg atatcaccgt ggtgacgcat gtcgcgcaag 2520
   actgtaacca cgcgtctgtt gactggcagg tggtggccaa tggtgatgtc agcgttgaac 2580
   tgcgtgatgc ggatcaacag gtggttgcaa ctggacaagg cactagcggg actttgcaag 2640
   tggtgaatcc gcacctctgg caaccgggtg aaggttatct ctatgaactg tgcgtcacag 2700
   ccaaaagcca gacagagtgt gatatctacc cgcttcgcgt cggcatccgg tcagtggcag 2760
   tgaagggcga acagttcctg attaaccaca aaccgttcta ctttactggc tttggtcgtc 2820
   atgaagatgc ggacttgcgt ggcaaaggat tcgataacgt gctgatggtg cacgaccacg 2880
   cattaatgga ctggattggg gccaactcct accgtacctc gcattaccct tacgctgaag 2940
   agatgctcga ctgggcagat gaacatggca tcgtggtgat tgatgaaact gctgctgtcg 3000
   gctttaacct ctctttaggc attggtttcg aagcgggcaa caagccgaaa gaactgtaca 3060
   gcgaagaggc agtcaacggg gaaactcagc aagcgcactt acaggcgatt aaagagctga 3120
   tagcgcgtga caaaaaccac ccaagcgtgg tgatgtggag tattgccaac gaaccggata   3180
   cccgtccgca aggtgcacgg gaatatttcg cgccactggc ggaagcaacg cgtaaactcg   3240
   acccgacgcg tccgatcacc tgcgtcaatg taatgttctg cgacgctcac accgatacca   3300
   tcagcgatct ctttgatgtg ctgtgcctga accgttatta cggatggtat gtccaaagcg   3360
   gcgatttgga aacggcagag aaggtactgg aaaaagaact tctggcctgg caggagaaac   3420
   tgcatcagcc gattatcatc accgaatacg gcgtggatac gttagccggg ctgcactcaa   3480
   tgtacaccga catgtggagt gaagagtatc agtgtgcatg gctggatatg tatcaccgcg   3540
   tctttgatcg cgtcagcgcc gtcgtcggtg aacaggtatg gaatttcgcc gattttgcga   3600
   cctcgcaagg catattgcgc gttggcggta acaagaaagg gatcttcact cgcgaccgca   3660
   aaccgaagtc ggcggctttt ctgctgcaaa aacgctggac tggcatgaac ttcggtgaaa   3720
   aaccgcagca gggaggcaaa caatgaatca acaactctcc tggcgcacca tcgtcggcta   3780
   cagcctcggg aattagatcc ccgaatttcc ccgatcgttc aaacatttgg caataaagtt   3840
   tcttaagatt gaatcctgtt gccggtcttg cgatgattat catataattt ctgttgaatt   3900
   acgttaagca tgtaataatt aacatgtaat gcatgacgtt atttatgaga tgggttttta   3960
   tgattagagt cccgcaatta tacatttaat acgcgataga aaacaaaata tagcgcgcaa   4020
   actaggataa attatcgcgc gcggtgtcat ctatgttact agatcgggaa tt           4072